(19) Europäisches Patentamt
European Patent Office
Office européen des brevets

(11) EP 3 592 851 B1

(12) EUROPEAN PATENT SPECIFICATION

(45) Date of publication and mention of the grant of the patent:
**27.11.2024 Bulletin 2024/48**

(21) Application number: **18711249.5**

(22) Date of filing: **05.03.2018**

(51) International Patent Classification (IPC):
***C12N 15/10*** (2006.01) ***C12Q 1/6811*** (2018.01)

(52) Cooperative Patent Classification (CPC):
**C12Q 1/6811; C07K 16/00; G16B 20/00; G16B 20/20; G16B 20/30; G16B 20/50; G16B 30/00; G16B 30/10; G16B 35/00; G16B 35/20; G16C 20/60**

(86) International application number:
**PCT/EP2018/055278**

(87) International publication number:
**WO 2018/162376 (13.09.2018 Gazette 2018/37)**

(54) **METHOD FOR DISCOVERY OF ALTERNATIVE ANTIGEN SPECIFIC ANTIBODY VARIANTS**

VERFAHREN ZUR ENTDECKUNG ALTERNATIVER ANTIGENSPEZIFISCHER ANTIKÖRPERVARIANTEN

PROCÉDÉ PERMETTANT DE DÉCOUVRIR DES VARIANTES ALTERNATIFS D'ANTICORPS SPÉCIFIQUES À UN ANTIGÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.03.2017 EP 17159617**

(43) Date of publication of application:
**15.01.2020 Bulletin 2020/03**

(73) Proprietor: **F. Hoffmann-La Roche AG**
**4070 Basel (CH)**

(72) Inventors:
- **GEORGES, Guy**
  **82377 Penzberg (DE)**
- **KLOSTERMANN, Stefan**
  **82377 Penzberg (DE)**
- **TISSOT, Alain**
  **82377 Penzberg (DE)**
- **ROS, Francesca**
  **82377 Penzberg (DE)**
- **BUJOTZEK, Alexander**
  **82377 Penzberg (DE)**
- **WRZODEK, Clemens**
  **82377 Penzberg (DE)**
- **SCHULTZ, Frederic**
  **82377 Penzberg (DE)**

(74) Representative: **Skolaut, Alexander et al**
**Roche Diagnostics GmbH**
**Patent Department (LPP.....6164)**
**Nonnenwald 2**
**82372 Penzberg (DE)**

(56) References cited:
- **X. WU ET AL: "Focused Evolution of HIV-1 Neutralizing Antibodies Revealed by Structures and Deep Sequencing", SCIENCE, vol. 333, no. 6049, 16 September 2011 (2011-09-16), pages 1593 - 1602, XP055330892, ISSN: 0036-8075, DOI: 10.1126/science.1207532**
- **J. ZHU ET AL: "De novo identification of VRC01 class HIV-1-neutralizing antibodies by next-generation sequencing of B-cell transcripts", PROCEEDINGS NATIONAL ACADEMY OF SCIENCES PNAS, vol. 110, no. 43, 22 October 2013 (2013-10-22), US, pages E4088 - E4097, XP055400388, ISSN: 0027-8424, DOI: 10.1073/pnas.1306262110**
- **PETER C FRIDY ET AL: "A robust pipeline for rapid production of versatile nanobody repertoires", NATURE METHODS, vol. 11, no. 12, 2 November 2014 (2014-11-02), pages 1253 - 1260, XP055247223, ISSN: 1548-7091, DOI: 10.1038/nmeth.3170**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PETER C FRIDY ET AL: "A robust pipeline for rapid production of versatile nanobody repertoires: Supplementary Information", NATURE METHODS, vol. 11, no. 12, 2 November 2014 (2014-11-02), pages 1253 - 1260, XP055399861, ISSN: 1548-7091, DOI: 10.1038/nmeth.3170**
- **J GLANVILLE ET AL: "Deep sequencing in library selection projects: what insight does it bring?", CURRENT OPINION IN STRUCTURAL BIOLOGY, vol. 33, 1 August 2015 (2015-08-01), GB, pages 146 - 160, XP055400390, ISSN: 0959-440X, DOI: 10.1016/j.sbi.2015.09.001**
- **OLGA OBREZANOVA ET AL: "Aggregation risk prediction for antibodies and its application to biotherapeutic development", MABS, vol. 7, no. 2, 4 March 2015 (2015-03-04), US, pages 352 - 363, XP055268078, ISSN: 1942-0862, DOI: 10.1080/19420862.2015.1007828**
- **ALEXANDER JARASCH ET AL: "Developability Assessment During the Selection of Novel Therapeutic Antibodies", JOURNAL OF PHARMACEUTICAL SCIENCES, vol. 104, no. 6, 1 June 2015 (2015-06-01), pages 1885 - 1898, XP055217440, ISSN: 0022-3549, DOI: 10.1002/jps.24430**


Remarks:

The complete document including Reference Table(s) and the Sequence Listing(s) can be downloaded from the EPO website

## Description

The current invention is in the field of antibody technology. More precisely herein is reported a method combining the versatility of B-cell cloning (BCC) with the power of next generation sequencing (NGS) to identify variant binders of a reference binder present within the B-cell population obtained from one or more immunized animals.

## Background of the Invention

Today antibodies are generally generated either by phage display or by immunizing laboratory animals and isolating the antibody producing B-cells therefrom. In the latter case the number of B-cells to be processed is reduced based on the properties of the B-cell or the respective secreted antibody. Thereafter the sequence information is obtained. Thus, candidate selection is done mostly based on the binding and functional properties of the antibodies but "blinded" with respect to the amino acid sequence, and thereby with respect e.g. to developability aspects, such as unpaired Cys-residues, unusual glycosylation sites, degradation hotspots (Asp, Asn, Met etc.).

In WO 2015/070191 a systems and methods for detection of genomic variants are reported. WO 2015/155035 reports methods for identifying and mapping the epitopes targeted by an antibody response. In WO 2015/164757 methods of viral neutralizing antibody epitope mapping are reported. WO 2016/023962 reports consensus-based allele detection. In WO 2016/118883 the detection of rare sequence variants, methods and compositions therefore are reported.

The huge number of B-cell obtained during current immunization processes prevents the characterization of all isolated antibodies produced thereby in detail. A selection and reduction of clone numbers has to be made, reducing the characterized immune response diversity.

Wu et al. disclosed the focused evolution of HIV-1 neutralizing antibodies revealed by structures and deep sequencing (Science 333 (2011) 1593-1602).

Zhu et al. disclosed the de novo identification of VRCO1 class HIV-1-neutralizing antibodies by next-generation sequencing of B-cell transcripts (Proc. Natl. Acad. Sci. USA 110 (2013) E4088-E4097).

Wu et al. and Zhu et al. each disclose a method for the identification of "new" cognate antibody variants for a given reference binder sequences from the same or different species as a reference binder.

Fridy et al. disclosed a robust pipeline for rapid production of versatile nanobody repertoires (Nat. Meth. 11 (2014) 1253-1260 + SI). Like Wu et al. and Zhu et al. do the methods as done by Fridy et al. not disclose any result or method or approach using sequence repertoire for searching antibody variants.

Glanville et al. disclosed what insight deep sequencing in library selection projects brings (Curr. Opin. Struct. Biol. 33 (2015) 146-160).

Obrezanova, et al. disclosed an aggregation risk prediction for antibodies and its application to biotherapeutic development (Mabs 7 (2015) 352-363).

Jarasch, et al., disclosed a developability assessment during the selection of novel therapeutic antibodies (J. Pharm. Sci. 104 (2015) 1885-1898).

Thus, there is a need to provide methods for identifying based on sequence information contained in the immune response diversity additional or variant antibodies binding to the same antigen but having different properties.

## Summary of the Invention

However, the present inventors have found that the challenge presented by the aforementioned loss of characterized immune response diversity can be overcome by exploiting the quantitative nature of next-generation sequencing.

It has been found by the current inventors that by combining the versatility of B-cell cloning (BCC) with the power of next generation sequencing (NGS) it is possible to identify variant binders of a reference binder present within the B-cell population obtained from one or more immunized animals with respect to the same antigen.

The method as reported herein merges the efficiency of B-cell cloning with the power of next generation sequencing, providing a highly streamlined approach for the identification of antibody variants of a reference antibody without the need to do an extensive (immuno- or cellular-) assay based screening.

It has been found that with the methods as reported herein it is possible to identify for antibody variable domains and also complete VH/VL pairs that have at least one developability hot-spot, i.e. that have at least one amino acid residue prone to post-translational modification, a variant that does not comprise said developability hot-spot, i.e. that has said at least one amino acid residue prone to post-translational modification changed to a different amino acid residue not prone to the same post-translational modification.

One other result of the herein reported method is the ability to provide a unique profile of the antibody response of one individual animal or of a group of animals immunized with the same antigen. This profile can be a source of valuable information.

One aspect as reported herein is a method for selecting a variant of a reference antibody variable domain encoding nucleic acid, wherein either the variant antibody variable domain amino acid sequence encoded by said variant of the reference antibody variable domain encoding nucleic acid, has improved developability compared to the reference antibody variable domain amino acid sequence encoded by said reference encoding nucleic acid, or wherein in the variant antibody variable domain amino acid sequence encoded by said variant of the reference antibody variable domain encoding nucleic acid, at least one amino acid residue that is post-translationally modified has been changed compared to the reference antibody variable domain amino acid sequence encoded by said reference encoding nucleic acid, wherein the variant and the reference antibody variable domain when paired with the respective other domain form an antibody binding site that bind to the same antigen,
the method comprising the following steps:

(i) providing a multitude of DNA-containing samples each including one or more antibody variable domain encoding nucleic acids;

(ii) performing PCR amplification of the antibody variable domain encoding nucleic acids of the multitude of (i) using consensus sequence-specific primers to obtain amplification products;

(iii) sequencing a plurality of the amplification products obtained in step (ii) in order to determine the relative proportion of each nucleotide at each position (in a sequencing read);

(iv) performing a sequence alignment between the sequencing (read) results of (iii) and the reference antibody variable domain encoding nucleic acid;

(v) performing a sequence-identity or homology-based ranking of the antibody variable domain encoding nucleic acids in said sequence alignment of (iv) with the reference antibody variable domain encoding nucleic acid being the template sequence; and

(vi) selecting the variant antibody variable domain encoding nucleic acid from one of the top 10 sequences of the sequence ranking of step (v);

whereby the variant selected in step (vi) is selected so that the developability has improved and/or at least one amino acid residue that is post-translationally modified has been changed.

One aspect according to the invention is a method for selecting a variant of a reference antibody variable domain, wherein the reference antibody variable domain has at least one of i) an unpaired Cys-residue in the variable domain or the HVR, ii) a glycosylation site, or iii) a post-translationally modified Asp, Asn or Met amino acid residue, the method comprising the following steps:

- aligning antibody variable domain encoding nucleic acids produced by sequencing nucleic acids from a multitude of B-cell clones each producing an antibody specifically binding to the same target as the reference antibody with the sequence of the reference antibody being the template sequence; and

- selecting a sequence that has the highest similarity to the reference sequence wherein at least one of the at least one amino acid residue of i) an unpaired Cys-residue in the variable domain or the HVR, ii) a glycosylation site, or iii) a post-translationally modified Asp, Asn or Met amino acid residue of the reference antibody variable domain is replaced by/changed to an amino acid residue that is not posttranslationally modified,

wherein the B-cells are obtained from the same immunization campaign as the reference antibody, wherein the B-cells have been enriched for antigen-specific antibody expressing B-cells.

One aspect as reported herein is a method for selecting a variant of a reference antibody variable domain, wherein the reference antibody variable domain has at least one of i) an unpaired Cys-residue in the variable domain or the HVR, ii) a glycosylation site, or iii) a post-translationally modified Asp, Asn or Met amino acid residue, the method comprising the following steps:

- receiving sequencing data produced by sequencing nucleic acids from a multitude of B-cell clones each producing an antibody specifically binding to the same target as the reference antibody;

- aligning the sequencing data with the sequence of the reference antibody being the template sequence;

- selecting a sequence that has the highest similarity to the reference sequence wherein at least one of the at least one amino acid residue of i) an unpaired Cys-residue in the variable domain or the HVR, ii) a glycosylation site, or iii) a post-translationally modified Asp, Asn or Met amino acid residue of the reference antibody variable domain is replaced by/changed to an amino acid residue that is not posttranslationally modified,

wherein the B-cells are obtained from the same immunization campaign as the reference antibody, wherein the B-cells have been enriched for antigen-specific antibody expressing B-cells.

One aspect according to the invention is a method for identifying a variant antibody of a reference antibody specifically binding to the same target/antigen comprising the following steps:

i) generating one or more sets of VH or/and VL sequences of a multitude of amino acid sequences or nucleic acid sequences of variant antibodies of the reference antibody specifically binding to the same target as the reference antibody, which have been determined by next generation sequencing, wherein the sequences are aligned with the amino acid sequence or nucleic acid sequence of a reference antibody as template, whereby the reference antibody specifically binds to a target/antigen, based on

α) identical length of VH or/and VL, or/and

β) identical length of all β-sheet framework regions, or/and

γ) identical length of all HVRs/CDRs, or/and

δ) identical HVR3/CDR3 sequence, mutations in frameworks and/or other HVRs/CDRs with up to 3 amino acid exchanges allowed, or/and

ε) homologous HVR/CDR3 sequence with up to 2 amino acid exchanges allowed and identical HVR/CDR1 and 2, or/and

ζ) mutations in frameworks and HVR/CDRs are allowed;

ii) ranking the aligned sequences in the one or more sets of ii) by

α) the number of, and/or

β) the position(s) of, and/or

γ) the change of the physico-chemical properties resulting from the, and/or

δ) the difference of VH/VL orientation resulting from the amino acid difference(s) to the reference antibody sequence;

iv) identifying one of the best 10 aligned and ranked antibodies as a variant antibody of a reference antibody, whereby the variant antibody does have at least one of i) an unpaired Cys-residue in the variable domain or the HVR, ii) a glycosylation site, or iii) a post-translationally modified Asp, Asn or Met amino acid residue less as the reference antibody,

wherein the multitude of amino acid sequences or nucleic acid sequences of variant antibodies specifically binding to the same target as the reference antibody are obtained from B-cells from the same immunization campaign as the reference antibody, wherein the B-cells have been enriched for antigen-specific antibody expressing B-cells.

One aspect as reported herein is a method for identifying a variant antibody of a reference antibody specifically binding to the same target/antigen comprising the following steps:

i) providing

α) the amino acid sequence or nucleic acid sequence of a reference antibody, whereby the reference antibody specifically binds to a target/antigen,

β) at least one biological property of said reference antibody and optionally one or more assays to determine the at least one biological property,

γ) a multitude of amino acid sequences or nucleic acid sequences of variant antibodies specifically binding to the same target as the reference antibody, which have been determined by next generation sequencing,

ii) generating one or more sets of (related) VH or/and VL sequences, wherein the sequences are aligned based on

α) identical length of VH or/and VL, or/and

β) identical length of all β-sheet framework regions, or/and

γ) identical length of all HVRs/CDRs, or/and

δ) identical HVR3/CDR3 sequence, mutations in frameworks and/or other HVRs/CDRs with up to 3 amino acid exchanges allowed, or/and

ε) homologous HVR/CDR3 sequence with up to 2 amino acid exchanges allowed and identical HVR/CDR1 and 2, or/and

ζ) mutations in frameworks and HVR/CDRs are allowed;

iii) ranking the aligned sequences in the one or more sets of ii) by

α) the number of, and/or

β) the position(s) of, and/or

γ) the change of the physico-chemical properties resulting from the, and/or

δ) the difference of VH/VL orientation resulting from the amino acid difference(s) to the reference antibody sequence;

iv) identifying one of the best 10 aligned and ranked antibodies as a variant antibody of a reference antibody, whereby the variant antibody does have at least one amino acid residue less that is post-translationally modified as the reference antibody,

wherein the multitude of amino acid sequences or nucleic acid sequences of variant antibodies specifically binding to the same target as the reference antibody are obtained from B-cells from the same immunization campaign as the reference antibody, wherein the B-cells have been enriched for antigen-specific antibody expressing B-cells.

In one embodiment of this aspect step i) or step ii), respectively, further comprises annotating the sequence with the same numbering scheme, which is the Wolfguy numbering scheme.

In one embodiment of this aspect differences in the sequence are annotated in the form: reference antibody amino acid residue-position-variant antibody amino acid residue and are grouped into a mutation tuple.

In one embodiment of this aspect the change of the physico-chemical properties is determined by the change in charge, hydrophobicity and/or size.

In one embodiment of this aspect the change of the physico-chemical properties is determined using a mutation risk score.

In one embodiment of this aspect the mutation risk score is determined based on the following Table, wherein residues that are not explicitly given in this Table are weighted with the value one:

| Wolfguy Index | Weight | Wolfguy Index | Weight |
|---|---|---|---|
| 101 | 0.2 | 151 | 2 |
| 102 | 1.1 | 152 | 2.6 |
| 103 | 0 | 153 | 1.2 |
| 104 | 0.5 | 154 | 2.3 |
| 105 | 0.2 | 155 | 1.9 |
| 106 | 0.8 | 156 | 3.7 |

(continued)

| Wolfguy Index | Weight | Wolfguy Index | Weight |
|---|---|---|---|
| 107 | 0.5 | 157 | 4 |
| 108 | 0.8 | 158 | 4 |
| 109 | 0.4 | 193 | 4 |
| 110 | 0.2 | 194 | 4 |
| 111 | 0 | 195 | 4 |
| 112 | 0.4 | 196 | 3.3 |
| 113 | 0 | 197 | 3.9 |
| 114 | 0.1 | 198 | 2.6 |
| 115 | 0.6 | 199 | 3.4 |
| 116 | 0.1 | 251 | 3.8 |
| 117 | 0.1 | 252 | 1.9 |
| 118 | 0.2 | 253 | 4 |
| 119 | 0.2 | 254 | 3.8 |
| 120 | 1.2 | 255 | 3.6 |
| 121 | 0 | 256 | 4 |
| 122 | 4 | 287 | 4 |
| 123 | 0 | 288 | 4 |
| 124 | 2 | 289 | 3.9 |
| 125 | 0.5 | 290 | 3.4 |
| 201 | 4 | 291 | 3.7 |
| 202 | 2.6 | 292 | 2.1 |
| 203 | 2 | 293 | 3.6 |
| 204 | 1.7 | 294 | 2.3 |
| 205 | 1 | 295 | 2.3 |
| 206 | 0 | 296 | 1 |
| 207 | 0 | 297 | 1.2 |
| 208 | 0.7 | 298 | 2.4 |
| 209 | 1.6 | 299 | 0.5 |
| 210 | 2 | 351 | 4 |
| 211 | 1.3 | 352 | 3.5 |
| 212 | 3.1 | 353 | 4 |
| 213 | 0.9 | 354 | 3.5 |
| 214 | 3.1 | 355 | 3.5 |
| 301 | 0.1 | 356 | 3 |
| 302 | 1.2 | 357 | 3 |
| 303 | 1.7 | 358 | 3 |
| 304 | 0.3 | 359 | 3 |
| 305 | 1.8 | 360 | 3 |
| 306 | 0 | 361 | 3 |
| 307 | 2.4 | 362 | 3 |
| 308 | 0 | 363 | 3 |
| 309 | 1.2 | 364 | 3 |
| 334 | 1 | 365 | 3 |
| 335 | 1 | 366 | 3 |
| 336 | 1 | 367 | 3 |
| 337 | 1 | 382 | 3 |
| 310 | 0.8 | 383 | 3 |
| 311 | 0.9 | 384 | 3 |
| 312 | 0.8 | 385 | 3 |
| 313 | 2.5 | 386 | 3 |

(continued)

| Wolfguy Index | Weight | Wolfguy Index | Weight |
|---|---|---|---|
| 314 | 0.1 | 387 | 3 |
| 315 | 1.5 | 388 | 3 |
| 316 | 0 | 389 | 3 |
| 317 | 1.5 | 390 | 3 |
| 318 | 0.8 | 391 | 3 |
| 319 | 0.1 | 392 | 3 |
| 320 | 1.4 | 393 | 3 |
| 321 | 0.2 | 394 | 3 |
| 322 | 0.3 | 395 | 3.5 |
| 323 | 0.2 | 396 | 3 |
| 324 | 1.8 | 397 | 3.5 |
| 325 | 0.6 | 398 | 1.5 |
| 326 | 2.2 | 399 | 3 |
| 333 | 1 | | |
| 327 | 0.6 | | |
| 328 | 3 | | |
| 329 | 2.5 | | |
| 330 | 4 | | |
| 331 | 2.9 | | |
| 332 | 2.8 | | |
| 401 | 3.5 | | |
| 402 | 2 | | |
| 403 | 0.5 | | |
| 404 | 1 | | |
| 405 | 0.3 | | |
| 406 | 0.1 | | |
| 407 | 1 | | |
| 408 | 0.1 | | |
| 409 | 1 | | |
| 410 | 0.1 | | |
| 411 | 0.1 | | |

with positions 101 to 125 corresponding to heavy chain variable domain framework 1, positions 151 to 199 corresponding to CDR-H1, positions 201 to 214 corresponding to heavy chain variable domain framework 2, positions 251 to 299 corresponding to CDR-H2, positions 301 to 332 corresponding to heavy chain variable domain framework 3, positions 351 to 399 corresponding to CDR-H3, positions 401 to 411 corresponding to heavy chain variable domain framework 4.

The multitude of amino acid sequences or nucleic acid sequences of variant antibodies specifically binding to the same target as the reference antibody are obtained from B-cells from the same immunization campaign as the reference antibody, wherein the B-cells have been enriched for antigen-specific antibody expressing B-cells.

One or more of the following is removed in the variant: i) unpaired Cys-residues in the variable domain or the HVR, ii) glycosylation sites, and iii) degradation hot-spots (Asp, Asn or Met).

One aspect as reported herein is a method for selecting a variant of a reference antibody variable domain, wherein the reference antibody variable domain has at least one amino acid residue that is post-translational modified, the method comprising the steps of the method according to any one of the preceding aspects.

One aspect as reported herein is a method for producing an antibody comprising the following steps:

- cultivating a cell comprising the nucleic acid obtained with a method according to any one of the previous aspects and all other nucleic acids required for the expression of a functional antibody,

- recovering the antibody from the cell or the cultivation medium.

Disclosed herein is a cell comprising the nucleic acid obtained with the method according to any one of the previous aspects.

One aspect as reported herein is a method for identifying a variant antibody of a reference antibody (that has comparable biological properties as the reference antibody) specifically binding to the same target/antigen comprising the following steps:

i) providing

α) the amino acid sequence or nucleic acid sequence of a reference antibody, whereby the reference antibody specifically binds to a target/antigen,

β) at least one biological property of said reference antibody and optionally one or more assays to determine the at least one biological property,

γ) a multitude (at least 10, at least 100, at least 1,000, at least 10,000) of amino acid sequences or nucleic acid sequences of variant antibodies specifically binding to the same target as the reference antibody (optionally obtained in/from the same immunization campaign as the reference antibody), which have been determined by next generation sequencing;

ii) generating one or more sets of related VH or/and VL sequences, wherein the sequences are aligned based on

α) identical length of VH or/and VL, or/and

β) identical length of all β-sheet framework regions, or/and

γ) identical length of all HVRs/CDRs, or/and

δ) identical HVR3/CDR3 sequence, 1 or 2 (1 to 3 amino acid exchanges) mutations in frameworks and/or HVRs/CDRs allowed, or/and

ε) highly homologous HVR/CDR3 sequence (1 or 2 amino acid exchanges allowed) and identical HVR/CDR1 and 2 (with mutations in frameworks allowed), or/and

ζ) mutations in frameworks and HVR/CDRs are allowed;

iii) ranking the aligned sequences in the one or more sets of ii) by

α) the number of, and/or

β) the position(s) of, and/or

γ) the change of the physico-chemical properties resulting from the, and/or

δ) the difference of VH/VL orientation (angle) resulting from the amino acid difference(s) to the reference antibody sequence;

iv) identifying one of the best 10 (or best 5, or best 3, or the best) aligned and ranked antibodies as a variant antibody of a reference antibody,

wherein the multitude of amino acid sequences or nucleic acid sequences of variant antibodies specifically binding to the same target as the reference antibody are obtained from B-cells from the same immunization campaign as the reference antibody, wherein the B-cells have been enriched for antigen-specific antibody expressing B-cells.

In one embodiment step ii) further comprises annotating the sequence with the same numbering scheme and annotating differences. In one embodiment the numbering scheme is the Kabat EU numbering or Wolfguy numbering scheme. In one preferred embodiment the numbering scheme is the Wolfguy numbering scheme. In one embodiment the differences are annotated in the form: reference antibody amino acid residue-position-variant antibody amino acid residue. In one embodiment the mutations of a variant antibody with respect to the reference antibody are grouped in a mutation tuple.

In one embodiment step ii) further comprises removing sequences that are identical in sequence to the reference antibody and/or one of the variant antibodies, or that are identical or similar with regard to the number, location and/or type of amino acid difference with respect to the reference antibody.

In one embodiment the change of the physico-chemical properties is determined by the change in (overall) charge, hydrophobicity and/or size. In one embodiment the change of the physico-chemical properties is determined using a mutation risk score. In one embodiment the risk score is determined based on the following Table, wherein residues that are not explicitly given in this Table are weighted with the value one:

| | Wolfguy Index | Weight | | Wolfguy Index | Weight |
|---|---|---|---|---|---|
| | 101 | 0.2 | | 151 | 2 |
| | 102 | 1.1 | | 152 | 2.6 |
| | 103 | 0 | | 153 | 1.2 |
| | 104 | 0.5 | | 154 | 2.3 |
| | 105 | 0.2 | | 155 | 1.9 |
| | 106 | 0.8 | | 156 | 3.7 |
| | 107 | 0.5 | | 157 | 4 |
| | 108 | 0.8 | | 158 | 4 |
| | 109 | 0.4 | | 193 | 4 |
| | 110 | 0.2 | | 194 | 4 |
| | 111 | 0 | | 195 | 4 |
| | 112 | 0.4 | CDR-H1 | 196 | 3.3 |
| | 113 | 0 | | 197 | 3.9 |
| | 114 | 0.1 | | 198 | 2.6 |
| | 115 | 0.6 | | 199 | 3.4 |
| | 116 | 0.1 | | 251 | 3.8 |
| | 117 | 0.1 | | 252 | 1.9 |
| | 118 | 0.2 | | 253 | 4 |
| | 119 | 0.2 | | 254 | 3.8 |
| | 120 | 1.2 | | 255 | 3.6 |
| Framework 1 | 121 | 0 | | 256 | 4 |
| | 122 | 4 | | 287 | 4 |
| | 123 | 0 | CDR-H2 | 288 | 4 |
| | 124 | 2 | | 289 | 3.9 |
| | 125 | 0.5 | | 290 | 3.4 |
| F | 201 | 4 | | 291 | 3.7 |

| | | |
|---|---|---|
| | 202 | 2.6 |
| | 203 | 2 |
| | 204 | 1.7 |
| | 205 | 1 |
| | 206 | 0 |
| | 207 | 0 |
| | 208 | 0.7 |
| | 209 | 1.6 |
| | 210 | 2 |
| | 211 | 1.3 |
| | 212 | 3.1 |
| | 213 | 0.9 |
| | 214 | 3.1 |
| | 301 | 0.1 |
| | 302 | 1.2 |
| | 303 | 1.7 |
| | 304 | 0.3 |
| | 305 | 1.8 |
| | 306 | 0 |
| | 307 | 2.4 |
| | 308 | 0 |
| | 309 | 1.2 |
| | 334 | 1 |
| | 335 | 1 |
| | 336 | 1 |
| | 337 | 1 |
| | 310 | 0.8 |
| | 311 | 0.9 |
| | 312 | 0.8 |
| | 313 | 2.5 |
| | 314 | 0.1 |
| | 315 | 1.5 |
| | 316 | 0 |
| | 317 | 1.5 |
| | 318 | 0.8 |
| | 319 | 0.1 |
| | 320 | 1.4 |
| | 321 | 0.2 |
| | 322 | 0.3 |
| | 323 | 0.2 |
| | 324 | 1.8 |
| | 325 | 0.6 |
| | 326 | 2.2 |
| | 333 | 1 |
| | 327 | 0.6 |
| | 328 | 3 |
| Framework 3 | 329 | 2.5 |
| | 330 | 4 |
| | 331 | 2.9 |
| | 292 | 2.1 |
| | 293 | 3.6 |
| | 294 | 2.3 |
| | 295 | 2.3 |
| | 296 | 1 |
| | 297 | 1.2 |
| | 298 | 2.4 |
| | 299 | 0.5 |
| | 351 | 4 |
| | 352 | 3.5 |
| | 353 | 4 |
| | 354 | 3.5 |
| | 355 | 3.5 |
| | 356 | 3 |
| | 357 | 3 |
| | 358 | 3 |
| | 359 | 3 |
| | 360 | 3 |
| | 361 | 3 |
| | 362 | 3 |
| | 363 | 3 |
| | 364 | 3 |
| | 365 | 3 |
| | 366 | 3 |
| | 367 | 3 |
| | 382 | 3 |
| | 383 | 3 |
| | 384 | 3 |
| | 385 | 3 |
| | 386 | 3 |
| | 387 | 3 |
| | 388 | 3 |
| | 389 | 3 |
| | 390 | 3 |
| | 391 | 3 |
| | 392 | 3 |
| | 393 | 3 |
| | 394 | 3 |
| | 395 | 3.5 |
| | 396 | 3 |
| CDR H-3 | 397 | 3.5 |
| | 398 | 1.5 |
| | 399 | 3 |

| | | |
|---|---|---|
| | 332 | 2.8 |
| Framework 4 | 401 | 3.5 |
| | 402 | 2 |
| | 403 | 0.5 |
| | 404 | 1 |
| | 405 | 0.3 |
| | 406 | 0.1 |
| | 407 | 1 |
| | 408 | 0.1 |
| | 409 | 1 |
| | 410 | 0.1 |
| | 411 | 0.1 |

with WolfGuy Index positions 101 to 125 corresponding to heavy chain variable domain framework 1, positions 151 to 199 corresponding to CDR-H1, positions 201 to 214 corresponding to heavy chain variable domain framework 2, positions 251 to 299 corresponding to CDR-H2, positions 301 to 332 corresponding to heavy chain variable domain framework 3, positions 351 to 399 corresponding to CDR-H3, positions 401 to 411 corresponding to heavy chain variable domain framework 4.

In one embodiment the mutation risk score takes negative values, whereby larger negative values indicate a larger risk of loss of function.

The multitude (at least 10, at least 100, at least 1,000, at least 10,000) of amino acid sequences or nucleic acid sequences of variant antibodies specifically binding to the same target as the reference antibody are obtained from B-cells from the same immunization campaign as the reference antibody, wherein the B-cells have been enriched for antigen-specific antibody expressing B-cells. In one embodiment the enrichment is by antigen-specific sorting or/and cell panning or/and non-antigen specific antibody producing B-cells depletion.

One aspect as reported herein is a method for identifying a variant antibody of a reference antibody (that has comparable biological properties as the reference antibody) specifically binding to the same target/antigen comprising the following steps:

i) determining/generating/measuring

α) the amino acid sequence or nucleic acid sequence of a reference antibody, whereby the reference antibody specifically binds to a target/antigen,

β) at least one biological property of the reference antibody,

γ) by next generation sequencing for a multitude (at least 10, at least 100, at least 1,000, at least 10,000) of variant antibodies the amino acid sequence or nucleic acid sequence, whereby the variant antibodies specifically bind to the same target/antigen as the reference antibody and optionally are obtained in/from the same immunization campaign as the reference antibody;

ii) aligning the VH or/and VL sequences based on

α) identical length of VH or/and VL, or/and

β) identical length of all β-sheet framework regions, or/and

γ) identical length of all HVRs/CDRs, or/and

δ) identical HVR3/CDR3 sequence, mutations in frameworks and/or HVRs/CDRs 1 or 2 (1 to 3 amino acid exchanges) allowed, or/and

ε) highly homologous HVR3/CDR3 sequence (1 or 2 amino acid exchanges allowed) and identical HVR/CDR 1 and 2 (with mutations in frameworks allowed), or/and

ζ) mutations in frameworks and HVRs/CDRs are allowed to generated one or more sets of related VH/VL sequences;

iii) ranking the aligned sequences in the one or more sets of ii) by

α) the number of, and/or

β) the position(s) of, and/or

γ) the change of the physico-chemical properties resulting from the, and/or

δ) the difference of VH/VL orientation (angle) resulting from the amino acid difference(s) to the reference antibody sequence;

iv) identifying one of the 10 (or 5 or 3 or the) best aligned and ranked antibodies as a variant antibody of a reference antibody,

wherein the multitude of amino acid sequences or nucleic acid sequences of variant antibodies specifically binding to the same target as the reference antibody are obtained from B-cells from the same immunization campaign as the reference antibody, wherein the B-cells have been enriched for antigen-specific antibody expressing B-cells.

In one embodiment step ii) further comprises annotating the sequence with the same numbering scheme and annotate differences. In one embodiment the numbering scheme is the Kabat EU numbering or the Wolfguy numbering scheme. In one preferred embodiment the numbering scheme is the Wolfguy numbering scheme. In one embodiment the differences are annotated in the form: reference antibody amino acid residue-position-variant antibody amino acid residue. In one embodiment the mutations of a variant antibody with respect to the reference antibody are grouped in a mutation tuple.

In one embodiment step ii) further comprises removing sequences that are identical in sequence to the reference antibody and/or one of the variant antibodies, or that are identical or similar with regard to the number, location and type of amino acid difference with respect to the reference antibody.

In one embodiment the change of the physico-chemical properties is determined by the change in charge, hydrophobicity and/or size. In one embodiment the change of the physico-chemical properties is determined using a mutation risk score. In one embodiment the risk score is determined based on the following Table, wherein residues that are not explicitly given in this Table are weighted with the value one:

| | Wolfguy Index | Weight | | Wolfguy Index | Weight |
|---|---|---|---|---|---|
| Framework 1 | 101 | 0.2 | CDR-H1 | 151 | 2 |
| | 102 | 1.1 | | 152 | 2.6 |
| | 103 | 0 | | 153 | 1.2 |
| | 104 | 0.5 | | 154 | 2.3 |
| | 105 | 0.2 | | 155 | 1.9 |
| | 106 | 0.8 | | 156 | 3.7 |
| | 107 | 0.5 | | 157 | 4 |
| | 108 | 0.8 | | 158 | 4 |
| | 109 | 0.4 | | 193 | 4 |
| | 110 | 0.2 | | 194 | 4 |
| | 111 | 0 | | 195 | 4 |
| | 112 | 0.4 | | 196 | 3.3 |
| | 113 | 0 | | 197 | 3.9 |
| | 114 | 0.1 | | 198 | 2.6 |
| | 115 | 0.6 | | 199 | 3.4 |

| | | | | | |
|---|---|---|---|---|---|
| | 116 | 0.1 | | 251 | 3.8 |
| | 117 | 0.1 | | 252 | 1.9 |
| | 118 | 0.2 | | 253 | 4 |
| | 119 | 0.2 | | 254 | 3.8 |
| | 120 | 1.2 | | 255 | 3.6 |
| | 121 | 0 | | 256 | 4 |
| | 122 | 4 | | 287 | 4 |
| | 123 | 0 | | 288 | 4 |
| | 124 | 2 | | 289 | 3.9 |
| | 125 | 0.5 | | 290 | 3.4 |
| Framework 2 | 201 | 4 | CDR-H2 | 291 | 3.7 |
| | 202 | 2.6 | | 292 | 2.1 |
| | 203 | 2 | | 293 | 3.6 |
| | 204 | 1.7 | | 294 | 2.3 |
| | 205 | 1 | | 295 | 2.3 |
| | 206 | 0 | | 296 | 1 |
| | 207 | 0 | | 297 | 1.2 |
| | 208 | 0.7 | | 298 | 2.4 |
| | 209 | 1.6 | | 299 | 0.5 |
| | 210 | 2 | | 351 | 4 |
| | 211 | 1.3 | | 352 | 3.5 |
| | 212 | 3.1 | | 353 | 4 |
| | 213 | 0.9 | | 354 | 3.5 |
| | 214 | 3.1 | | 355 | 3.5 |
| Framework 3 | 301 | 0.1 | CDR H-3 | 356 | 3 |
| | 302 | 1.2 | | 357 | 3 |
| | 303 | 1.7 | | 358 | 3 |
| | 304 | 0.3 | | 359 | 3 |
| | 305 | 1.8 | | 360 | 3 |
| | 306 | 0 | | 361 | 3 |
| | 307 | 2.4 | | 362 | 3 |
| | 308 | 0 | | 363 | 3 |
| | 309 | 1.2 | | 364 | 3 |
| | 334 | 1 | | 365 | 3 |
| | 335 | 1 | | 366 | 3 |
| | 336 | 1 | | 367 | 3 |
| | 337 | 1 | | 382 | 3 |
| | 310 | 0.8 | | 383 | 3 |
| | 311 | 0.9 | | 384 | 3 |
| | 312 | 0.8 | | 385 | 3 |
| | 313 | 2.5 | | 386 | 3 |
| | 314 | 0.1 | | 387 | 3 |
| | 315 | 1.5 | | 388 | 3 |
| | 316 | 0 | | 389 | 3 |
| | 317 | 1.5 | | 390 | 3 |
| | 318 | 0.8 | | 391 | 3 |
| | 319 | 0.1 | | 392 | 3 |
| | 320 | 1.4 | | 393 | 3 |
| | 321 | 0.2 | | 394 | 3 |

| | | | | |
|---|---|---|---|---|
| | 322 | 0.3 | 395 | 3.5 |
| | 323 | 0.2 | 396 | 3 |
| | 324 | 1.8 | 397 | 3.5 |
| | 325 | 0.6 | 398 | 1.5 |
| | 326 | 2.2 | 399 | 3 |
| | 333 | 1 | | |
| | 327 | 0.6 | | |
| | 328 | 3 | | |
| | 329 | 2.5 | | |
| | 330 | 4 | | |
| | 331 | 2.9 | | |
| | 332 | 2.8 | | |
| Framework 4 | 401 | 3.5 | | |
| | 402 | 2 | | |
| | 403 | 0.5 | | |
| | 404 | 1 | | |
| | 405 | 0.3 | | |
| | 406 | 0.1 | | |
| | 407 | 1 | | |
| | 408 | 0.1 | | |
| | 409 | 1 | | |
| | 410 | 0.1 | | |
| | 411 | 0.1 | | |

with Wolf-Guy Index positions 101 to 125 corresponding to heavy chain variable domain framework 1, positions 151 to 199 corresponding to CDR-H1, positions 201 to 214 corresponding to heavy chain variable domain framework 2, positions 251 to 299 corresponding to CDR-H2, positions 301 to 332 corresponding to heavy chain variable domain framework 3, positions 351 to 399 corresponding to CDR-H3, positions 401 to 411 corresponding to heavy chain variable domain framework 4 (positions 501 to 523 corresponding to light chain variable domain framework 1, positions 551 to 599 corresponding to CDR-L1, positions 601 to 615 corresponding to light chain variable domain framework 2, positions 651 to 699 corresponding to CDR-L2, positions 701 to 734 corresponding to light chain variable domain framework 3, positions 751 to 799 corresponding to CDR-L3, positions 801 to 810 corresponding to light chain variable domain framework 4).

In one embodiment the mutation risk score takes negative values, whereby larger negative values indicate a larger risk of loss of function.

The multitude (at least 10, at least 100, at least 1,000, at least 10,000) of amino acid sequences or nucleic acid sequences of variant antibodies specifically binding to the same target as the reference antibody are obtained from B-cells from the same immunization campaign as the reference antibody, wherein the B-cells have been enriched for antigen-specific antibody expressing B-cells. In one embodiment the enrichment is by antigen-specific sorting or/and cell panning or/and non-antigen specific antibody producing B-cells depletion.

One aspect as reported herein is a method for identifying a variant antibody of a reference antibody (that has comparable biological properties as the reference antibody) specifically binding to the same target/antigen comprising the following steps:

a) providing a multitude of B-cells producing/expressing (a multitude of) different antibodies binding to the same target/antigen as the reference antibody (obtained from the same animal species as the reference antibody);

b) isolating (and amplifying) from the multitude of B-cells the antibody encoding nucleic acids;

c) sequencing the antibody encoding nucleic acids by means of a next generation sequencing method;

d) generating a sequence alignment of the antibody encoding nucleic acid sequences by

i) aligning the VH or/and VL sequences based on

α) identical length of VH or/and VL, or/and

β) identical length of all β-sheet framework regions, or/and

γ) identical length of all HVRs/CDRs, or/and

δ) identical HVR3/CDR3 sequence, mutations in frameworks and/or HVRs/CDRs 1 or 2 (1 to 3 amino acid exchanges) allowed, or/and

ε) highly homologous HVR3/CDR3 sequence (1 or 2 amino acid exchanges allowed) and identical HVR/CDR 1 and 2 (with mutations in frameworks allowed), or/and

ζ) mutations in frameworks and HVRs/CDRs are allowed to generated one or more sets of related VH/VL sequences;

ii) ranking the aligned sequences in the one or more sets of ii) by

α) the number of, and/or

β) the position(s) of, and/or

γ) the change of the physico-chemical properties resulting from the, and/or

δ) the difference of VH/VL orientation (angle) resulting from the amino acid difference(s) to the reference antibody sequence;

e) identifying one or more variant antibodies of the reference antibody;

f) determining one or more biological properties of the variant antibodies; and

g) selecting a variant antibody with comparable or improved one or more biological properties and thereby identifying a variant antibody of a reference antibody,

wherein the multitude of amino acid sequences or nucleic acid sequences of variant antibodies specifically binding to the same target as the reference antibody are obtained from B-cells from the same immunization campaign as the reference antibody, wherein the B-cells have been enriched for antigen-specific antibody expressing B-cells.

One aspect as reported herein is a method for identifying a variant antibody of a reference antibody (that has comparable biological properties as the reference antibody) specifically binding to the same target/antigen comprising the following steps:

(i) providing one or more DNA-containing samples that comprise a multitude of antibody encoding nucleic acids;

(ii) performing PCR amplification of regions of the antibody encoding nucleic acids in each of the samples of (i) using consensus sequence-specific primers, wherein the consensus sequence-specific primers bind to consensus sequences that are common to a plurality of genes within the multitude of antibody encoding nucleic acids, thereby generating a pool of amplification products;

(iii) sequencing said amplification products in order to determine the relative proportion of each nucleotide at each position (in a sequencing read);

(iv) performing a sequence alignment (based on sequence identity) between the sequencing (read) results of (iii) and at least one reference sequence, which reference sequence corresponds to an antibody having desirable properties; and

(v) identifying one or more variant antibodies of the reference antibody,

wherein the multitude of nucleic acid sequences of variant antibodies specifically binding to the same target as the reference antibody are obtained from B-cells from the same immunization campaign as the reference antibody, wherein the B-cells have been enriched for antigen-specific antibody expressing B-cells.

One aspect as reported herein is a method for identifying a variant antibody of a reference antibody (that has comparable biological properties as the reference antibody) specifically binding to the same target/antigen comprising the following steps:

a) amplifying one or more regions of interest from a biological sample comprising nucleic acids encoding antibody variable domains, wherein a plurality of amplicons for each region of interest are generated;

b) attaching an adapter and a random component to each amplicon generated in (a) and amplifying each of said extended amplicon;

c) sequencing the amplicons comprising the random component generated in (b), wherein redundant reads are generated and wherein the redundant reads are grouped by the random component, and identifying a consensus sequence;

d) comparing the consensus sequence to a reference sequence, wherein a consensus sequence that differs from the reference sequence comprises a mutation/variation; and

e) identifying one or more variant antibodies based on the results of step d).

In one embodiment steps a) to c) are

a) hybridizing a primer pool comprising one or more primer pairs specific to one or more regions of interest from a biological sample comprising nucleic acids encoding antibody variable domains, extending from an upstream primer of the primer pair to a downstream primer of the primer pair, and ligating the extension product to the downstream primer of the primer pair, wherein products comprising the regions of interest flanked by sequences required for amplification are generated;

b) attaching an adapter comprising a random component and attaching an adapter comprising an index sequence to the products from (a) and amplifying each of said extended sequences;

c) sequencing the products comprising the random component generated in (b), wherein redundant reads are generated and wherein the redundant reads are grouped by the random component and identifying a consensus sequence.

One aspect as reported herein is a method for selecting a variant of a reference antibody variable domain encoding nucleic acid, wherein the reference antibody variable domain amino acid sequence encoded by said encoding nucleic acid has at least one developability hot-spot (i.e. comprises at least one amino acid residue that is post-translationally modified resulting in a change of the biological properties (reduction of the binding affinity to its target/antigen) of the reference antibody comprising said reference antibody variable domain in one of its binding sites), the method comprising the following steps:

(i) providing a multitude of DNA-containing samples (genomic material of antibody secreting B-cell) each including one or more antibody variable domain encoding nucleic acids;

(ii) performing PCR amplification of the antibody variable domain encoding nucleic acids of (i) using consensus sequence-specific primers to obtain amplification products (wherein said consensus sequence-specific primers bind to consensus sequences that are common to a plurality of genes within the nucleic acids, thereby generating a pool of amplification products);

(iii) sequencing a plurality of the amplification products obtained in step (ii) in order to determine the relative proportion of each nucleotide at each position (in a sequencing read);

(iv) performing a sequence alignment (based on sequence identity) between the sequencing (read) results of (iii) and the reference antibody variable domain encoding nucleic acid;

(v) performing a sequence-identity/homology-based ranking of the antibody variable domain encoding nucleic acids in the sequence alignment with the reference antibody variable domain encoding nucleic acid being the perfect/template/reference sequence; and

(vi) selecting the variant antibody variable domain encoding nucleic acid based on the sequence ranking of step (v),

whereby the variant antibody variable domain selected in step (vi) is selected so that the developability hot-spot is removed (i.e. so that it comprises at least one amino acid residue less that is post-translationally modified compared to the reference antibody resulting in a reduced change of the biological properties (reduced reduction of binding affinity to its target/antigen) of the reference antibody comprising said reference antibody variable domain in one of its binding sites),

wherein the multitude of nucleic acid sequences of variant antibodies specifically binding to the same target as the reference antibody are obtained from B-cells from the same immunization campaign as the reference antibody, wherein the B-cells have been enriched for antigen-specific antibody expressing B-cells,

wherein the developability hot-spot (the amino acid reside that is post-translationally modified) is selected from the group consisting of one or more unpaired Cys-residues in the variable domain or in one of the HVRs, one or more glycosylation sites, an amino acid residue in an N- or O-glycosylation site, or/and one or more degradation hot-spots (Asp, Asn or Met).

One aspect as reported herein is a method for selecting a variant of a reference antibody variable domain, wherein the reference antibody variable domain has at least one developability hot-spot, the method comprising the following steps:

- receiving sequencing data produced by sequencing nucleic acids from a multitude of B-cell clones each producing an antibody specifically binding to the same target as the reference antibody;

- aligning the sequencing data with the sequence of the reference antibody variable domain being the perfect/template/reference sequence;

- selecting a sequence that has the highest structural/functional identity/similarity to the reference antibody variable domain sequence but not having the developability hot-spot,

  wherein the multitude of amino acid sequences or nucleic acid sequences of variant antibodies specifically binding to the same target as the reference antibody are obtained from B-cells from the same immunization campaign as the reference antibody, wherein the B-cells have been enriched for antigen-specific antibody expressing B-cells,

  wherein the developability hot-spot (the amino acid reside that is post-translationally modified) is selected from the group consisting of one or more unpaired Cys-residues in the variable domain or in one of the HVRs, one or more glycosylation sites, an amino acid residue in an N- or O-glycosylation site, or/and one or more degradation hot-spots (Asp, Asn or Met).

Reported herein are a methods for selecting a variant of a reference antibody variable domain, wherein the reference antibody variable domain has at least one developability hot-spot, the method comprising the steps of one of the method as reported herein.

One aspect as reported herein is a method for producing an antibody comprising the following steps:

- cultivating a cell comprising the nucleic acid encoding a variant antibody of a reference antibody, wherein the nucleic acid has been obtained with one of the methods as reported herein and all other nucleic acids required for the expression of a functional antibody,

- recovering the antibody from the cell or the cultivation medium.

One aspect as reported herein is a cell comprising the nucleic acid obtained with one of the methods as reported herein.

It is expressly stated that each aspect may also be an embodiment of a different aspect.

The methods as reported herein can be used for the identification of alternative antigen specific antibody variants of a reference antibody without developability issues.

The method as reported herein can even be worked with polyclonal sera as by the use of NGS these are "monoclonalized".

## Detailed Description of the Invention

It will be readily understood that the embodiments, as generally described herein, are exemplary. The following more detailed description of various embodiments is not intended to limit the scope of the present disclosure, but is merely representative of various embodiments.

## DEFINITIONS

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991).

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically, the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype, and the Kabat EU index numbering system (see pages 661-723) is used for the constant heavy chain domains (CH1, Hinge, CH2 and CH3, which is herein further clarified by referring to "numbering according to Kabat EU index" in this case).

It must be noted that as used herein and in the appended claims, the singular forms "a", "an", and "the" include plural reference unless the context clearly dictates otherwise. Thus, for example, reference to "a cell" includes a plurality of such cells and equivalents thereof known to those skilled in the art, and so forth. As well, the terms "a" (or "an"), "one or more" and "at least one" can be used interchangeably herein. It is also to be noted that the terms "comprising", "including", and "having" can be used interchangeably.

To a person skilled in the art procedures and methods are well known to convert an amino acid sequence, e.g. of a polypeptide, into a corresponding nucleic acid sequence encoding this amino acid sequence. Therefore, a nucleic acid is characterized by its nucleic acid sequence consisting of individual nucleotides and likewise by the amino acid sequence of a polypeptide encoded thereby.

The use of recombinant DNA technology enables the generation derivatives of a nucleic acid. Such derivatives can, for example, be modified in individual or several nucleotide positions by substitution, alteration, exchange, deletion or insertion. The modification or derivatization can, for example, be carried out by means of site directed mutagenesis. Such modifications can easily be carried out by a person skilled in the art (see e.g. Sambrook, J., et al., Molecular Cloning: A laboratory manual (1999) Cold Spring Harbor Laboratory Press, New York, USA; Hames, B.D., and Higgins, S.G., Nucleic acid hybridization - a practical approach (1985) IRL Press, Oxford, England).

Useful methods and techniques for carrying out the current invention are described in e.g. Ausubel, F.M. (ed.), Current Protocols in Molecular Biology, Volumes I to III (1997); Glover, N.D., and Hames, B.D., ed., DNA Cloning: A Practical Approach, Volumes I and II (1985), Oxford University Press; Freshney, R.I. (ed.), Animal Cell Culture - a practical approach, IRL Press Limited (1986); Watson, J.D., et al., Recombinant DNA, Second Edition, CHSL Press (1992); Winnacker, E.L., From Genes to Clones; N.Y., VCH Publishers (1987); Celis, J., ed., Cell Biology, Second Edition, Academic Press (1998); Freshney, R.I., Culture of Animal Cells: A Manual of Basic Technique, second edition, Alan R. Liss, Inc., N.Y. (1987).

The term "about" denotes a range of +/- 20 % of the thereafter following numerical value. In one embodiment the term about denotes a range of +/- 10 % of the thereafter following numerical value. In one embodiment the term about denotes a range of +/- 5 % of the thereafter following numerical value.

The term "glycan" denotes a polysaccharide, or oligosaccharide. Glycan is also used herein to refer to the carbohydrate portion of a glycoconjugate, such as a glycoprotein, glycolipid, glycopeptide, glycoproteome, peptidoglycan, lipopolysaccharide or a proteoglycan. Glycans usually consist solely of β-glycosidic linkages between monosaccharides. Glycans can be homo- or heteropolymers of monosaccharide residues, and can be linear or branched.

The term "antibody" herein is used in the broadest sense and encompasses various antibody structures, including but not limited to monoclonal antibodies, polyclonal antibodies, and multispecific antibodies (e.g., bispecific antibodies) so long as they exhibit the desired antigen-binding activity.

The term "next generation sequencing" as used herein denotes a method comprising massive parallel sequencing providing a sequence output much higher than that of traditional sequencing (e.g. traditional Sanger sequencing). This term is also often defined as "deep sequencing" or "second-generation sequencing" (Metzker, M.L., Nat. Rev. Genet. 11 (2010) 31-46; Mardis, E.R., Annu. Rev. Genom. Hum. Genet. 9 (2008) 387-402). The use of a next generation sequencing method allows obtaining of sequencing data in a very short time. Different technologies are commercially available for performing next generation sequencing, such as, for example, pyrosequencing (454 Life Sciences, Roche Diagnostics Corp., Basel, Switzerland), sequencing by synthesis (HiSeq™ and MiSeq™, Illumina, Inc., San Diego, CA), sequencing by ligation (SOLiD™, Life Technologies Corp. Logan, UT), Polonator sequencing, ion semiconductor sequencing (Ion PGM™, and Ion Proton™, Life Technologies Corp., Logan, UT), Ion Torrent sequencing, nanopore sequencing, single-molecule real-time sequencing (SMRT™, Pacific Biosciences, Menlo Park, CA), HeliScope Single Molecule sequencing, tunneling currents sequencing, sequencing by hybridization, mass spectrometry sequencing, microfluidic Sanger sequencing, RNA polymerase (RNAP) sequencing and others.

The term "sequencing platform" denotes a system for sequencing nucleic acids, including genomic DNA (gDNA), complementary DNA (cDNA) and RNA. The system may include one or more machines or apparatuses (e.g., amplification machines, sequencing machines, detection devices, etc.), data storage and analytical devices (e.g., hard drives, remote storage systems, processors, etc.), reagents (e.g., primers, probes, linkers, tags, NTPs, etc.) and particular methods for their use. For example, sequencing by synthesis and pyrosequencing and different platforms.

The term "read" denotes a single instance of determining the identity of a nucleotide at a particular position or the sequence of nucleotides in a particular polynucleotide. If a nucleotide or polynucleotide sequence is determined X times in a sequencing assay, there are "X reads" or a "read depth of X" or "read coverage of X" for that nucleotide or polynucleotide.

The term "hypervariable region" or "HVR", as used herein, refers to each of the regions of an antibody variable domain which are hypervariable in sequence ("complementarity determining regions" or "CDRs") and/or form structurally defined loops ("hypervariable loops"), and/or contain the antigen-contacting residues ("antigen contacts"). Generally, antibodies comprise six HVRs; three in the VH (H1, H2, H3), and three in the VL (L1, L2, L3).

HVRs herein include

(a) hypervariable loops occurring at amino acid residues 26-32 (L1), 50-52 (L2), 91-96 (L3), 26-32 (H1), 53-55 (H2), and 96-101 (H3) (Chothia, C. and Lesk, A.M., J. Mol. Biol. 196 (1987) 901-917);

(b) CDRs occurring at amino acid residues 24-34 (L1), 50-56 (L2), 89-97 (L3), 31-35b (H1), 50-65 (H2), and 95-102 (H3) (Kabat, E.A. et al., Sequences of Proteins of Immunological Interest, 5th ed. Public Health Service, National Institutes of Health, Bethesda, MD (1991), NIH Publication 91-3242.);

(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and

(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., supra.

An "isolated" antibody is one, which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman, S. et al., J. Chromatogr. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called $\alpha$, $\delta$, $\varepsilon$, $\gamma$, and $\mu$, respectively.

The term "N-linked oligosaccharide" denotes oligosaccharides that are linked to the peptide backbone at an asparagine amino acid residue, by way of an asparagine-N-acetyl glucosamine linkage. N-linked oligosaccharides are also called "N-glycans." All N-linked oligo saccharides have a common pentasaccharide core of Man3GlcNAc2. They differ in the presence of, and in the number of branches (also called antennae) of peripheral sugars such as N-acetyl glucosamine, galactose, N-acetyl galactosamine, fucose and sialic acid. Optionally, this structure may also contain a core fucose molecule and/or a xylose molecule. N-linked oligosaccharides are attached to a nitrogen of asparagine or arginine side-chains. N-glycosylation motifs, i.e. N-glycosylation sites, comprise an Asn-X-Ser/Thr consensus sequence, where X is any amino acid except proline. Thus, an amino acid residue in an N-glycosylation site can be any amino acid residue in the Asn-X-Ser/Thr consensus sequence, where X is any amino acid except proline. In one embodiment is the amino acid residue in an N-glycosylation site Asn, Ser or Thr.

The term "O-linked oligosaccharide" denotes oligosaccharides that are linked to the peptide backbone at a threonine or serine amino acid residue. In one embodiment is the amino acid residue in an O-glycosylation site Ser or Thr.

The term "glycosylation state" denotes a specific or desired glycosylation pattern of an antibody. A "glycoform" is an antibody comprising a particular glycosylation state. Such glycosylation patterns include, for example, attaching one or more sugars at position N-297 of the Fc-region of an antibody (numbering according to Kabat), wherein said sugars are produced naturally, recombinantly, synthetically, or semi-synthetically. The glycosylation pattern can be determined by many methods known in the art. For example, methods of analyzing carbohydrates on proteins have been reported in US 2006/0057638 and US 2006/0127950.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt, T.J. et al. Kuby Immunology, 6th ed., W.H. Freeman and Co., N.Y. (2007), page 91) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano, S. et al., J. Immunol. 150 (1993) 880-887; Clackson, T. et al., Nature 352 (1991) 624-628).

Alignment of a variant amino acid sequence or variant nucleic acid sequence with respect to a reference amino acid sequence or reference nucleic acid sequence can be done based on the "percent (%) sequence identity". The "percent (%) sequence identity" is defined as the percentage of residues in a variant sequence that are identical with the residues in the reference sequence, after aligning the sequences and introducing gaps, if necessary, to achieve the maximum percent sequence identity. Alignment can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for sequence comparisons, the % sequence identity of a given (variant) sequence A to, with, or against a given (reference) sequence B (which can alternatively be phrased as a given (variant) sequence A that has or comprises a certain % sequence identity to, with, or against a given (reference) sequence B) is calculated as follows:

100 times the fraction X/Y

where X is the number of residues scored as identical matches by the sequence alignment program ALIGN-2 in that program's alignment of A and B, and where Y is the total number of residues in B. It will be appreciated that where the length of sequence A is not equal to the length of sequence B, the % sequence identity of A to B will not equal the % sequence identity of B to A.

The term "glycostructure" as used within this application denotes a single, defined N- or O-linked oligosaccharide at a specified amino acid residue. Thus, the term "antibody with a G1 glycostructure" denotes an antibody comprising at the asparagine amino acid residue at about amino acid position 297 according to the Kabat numbering scheme or in the FAB region a biantennary oligosaccharide comprising only one terminal galactose residue at the non-reducing ends of the oligosaccharide. The term "oligosaccharide" as used within this application denotes a polymeric saccharide comprising two or more covalently linked monosaccharide units.

The term "developability hot-spot" denotes an amino acid residue within the amino acid sequence of a polypeptide, such as e.g. an antibody variable domain, that is post-translationally modified, i.e. that is prone to post-translational modification.

This post-translational modification results in a change, e.g. a reduction or loss, of at least one biological property, such as e.g. antigen binding in case of an antibody variable domain.

The term "post-translational modification" denotes a covalent modification of amino acid residues within a polypeptide following biosynthesis. Post-translational modifications can occur on the amino acid side chains by modifying an existing functional group or introducing a new one. Common post-translational modifications for Ala is N-acetylation; for Arg is deimination, methylation; for Asn is deamidation, N-linked glycosylation; for Asp is isomerization; for Cys is disulfide-bond formation, oxidation, palmitoylation, N-acetylation, S-nitrosylation; for Gln is cyclization; for Glu is cyclization, gamma-carboxylation; for Gly is N-myristoylation, N-acetylation; for His is phosphorylation; for Lys is acetylation, ubiquitination, SUMOylation, methylation, hydroxylation; for Met is N-acetylation, oxidation; for Pro is hydroxylation; for Ser is phosphorylation, O-linked glycosylation, N-acetylation; for Thr is phosphorylation, O-linked glycosylation, N-acetylation; for Trp is oxidation, formation of Kynurenine; for Tyr is sulfation, phosphorylation; for Val is N-acetylation.

The term "binding site of an antibody" denotes the pair of a light chain variable domain and a heavy chain variable domain.

## Antibody Glycosylation

Human antibodies are mainly glycosylated at the asparagine residue at about position 297 (Asn297) of the heavy chain CH2 domain or in the FAB region with a more or less fucosylated biantennary complex oligosaccharide (antibody amino acid residue numbering according to Kabat, supra). The biantennary glycostructure can be terminated by up to two consecutive galactose (Gal) residues in each arm. The arms are denoted (1,6) and (1,3) according to the glycoside bond to the central mannose residue. The glycostructure denoted as G0 comprises no galactose residue. The glycostructure denoted as G1 contains one or more galactose residues in one arm. The glycostructure denoted as G2 contains one or more galactose residues in each arm (Raju, T.S., Bioprocess Int. 1 (2003) 44-53). Human constant heavy chain regions are reported in detail by Kabat, supra, and by Brueggemann, M., et al., J. Exp. Med. 166 (1987) 1351-1361; Love, T.W., et al., Methods Enzymol. 178 (1989) 515-527. CHO type glycosylation of antibody Fc-regions is e.g. described by Routier, F.H., Glycoconjugate J. 14 (1997) 201-207.

An antibody in general comprises two so called full length light chain polypeptides (light chain) and two so called full length heavy chain polypeptides (heavy chain). Each of the full length heavy and light chain polypeptides contains a variable domain (variable region) (generally the amino terminal portion of the full length polypeptide chain) comprising binding regions, which interact with an antigen. Each of the full length heavy and light chain polypeptides comprises a constant region (generally the carboxyl terminal portion). The constant region of the full length heavy chain mediates the binding of the antibody i) to cells bearing a Fc gamma receptor (FcγR), such as phagocytic cells, or ii) to cells bearing the neonatal Fc receptor (FcRn) also known as Brambell receptor. It also mediates the binding to some factors including factors of the classical complement system such as component (C1q). The variable domain of a full length antibody's light or heavy chain in turn comprises different segments, i.e. four framework regions (FR) and three hypervariable regions (CDR). A "full length antibody heavy chain" is a polypeptide consisting in N-terminal to C-terminal direction of an antibody heavy chain variable domain (VH), an antibody constant domain 1 (CH1), an antibody hinge region, an antibody constant domain 2 (CH2), an antibody constant domain 3 (CH3), and optionally an antibody constant domain 4 (CH4) in case of an antibody of the subclass IgE. A "full length antibody light chain" is a polypeptide consisting in N-terminal to C-terminal direction of an antibody light chain variable domain (VL), and an antibody light chain constant domain (CL). The full length antibody chains a linked together via inter-polypeptide disulfide bonds between the CL-domain and the CH1 domain and between the hinge regions of the full length antibody heavy chains.

Is has been reported in recent years that the glycosylation pattern of antibodies, i.e. the saccharide composition and multitude of attached glycostructures, has a strong influence on the biological properties (see e.g. Jefferis, R., Biotechnol. Prog. 21 (2005) 11-16). Antibodies produced by mammalian cells contain 2-3 % by mass oligosaccharides (Taniguchi, T., et al., Biochem. 24 (1985) 5551-5557). This is equivalent e.g. in an antibody of class G (IgG) to 2.3 oligosaccharide residues in an IgG of mouse origin (Mizuochi, T., et al., Arch. Biochem. Biophys. 257 (1987) 387-394) and to 2.8 oligosaccharide residues in an IgG of human origin (Parekh, R.B., et al., Nature 316 (1985) 452-457), whereof generally two are located in the Fc-region at Asn297 and the remaining in the variable region (Saba, J.A., et al., Anal. Biochem. 305 (2002) 16-31).

For the notation of the different N- or O-linked oligosaccharides the individual sugar residues are listed from the non-reducing end to the reducing end of the oligosaccharide molecule. The longest sugar chain is chosen as basic chain for the notation. The reducing end of an N- or O-linked oligosaccharide is the monosaccharide residue, which is directly bound to the amino acid of the amino acid backbone of the antibody, whereas the end of an N- or O-linked oligosaccharide, which is located at the opposite terminus as the reducing end of the basic chain, is termed non-reducing end.

All oligosaccharides are described with the name or abbreviation for the non-reducing saccharide (i.e., Gal), followed by the configuration of the glycosidic bond ($\alpha$ or $\beta$), the ring bond (1 or 2), the ring position of the reducing saccharide involved in the bond (2, 3, 4, 6 or 8), and then the name or abbreviation of the reducing saccharide (i.e., GlcNAc). Each saccharide is preferably a pyranose. For a review of standard glycobiology nomenclatures see, Essentials of Glycobiology Varki et al. eds., 1999, CSHL Press.

The term "defined glycostructure" denotes within this application a glycostructure in which the monosaccharide residue at the non-reducing ends of the glycostructure is of a specific kind. The term "defined glycostructure" denotes within this application a glycostructure in which the monosaccharide residue at the non-reducing end of glycostructures are defined and of a specific kind.

Post-translational modification prone amino acid residues

Asn and Asp residues share a common degradation pathway that precedes via the formation of a cyclic succinimide intermediate. Succinimide formation results from an intramolecular rearrangement after deamidation of Asn or dehydration of Asp by nucleophilic attack of the backbone nitrogen of the succeeding amino acid on the Asn/Asp side chain $\gamma$-carbonyl group. The metastable cyclic imide can hydrolyze at either one of its two carbonyl groups to form aspartyl or iso-aspartyl linkages in different ratios, depending on hydrolysis conditions and conformational restraints. In addition, alternative degradation mechanisms were proposed such as nucleophilic attack by the backbone carbonyl oxygen to form a cyclic isoimide or direct water-assisted hydrolysis of Asn to Asp. Several analytical methods, mostly charge-sensitive methods such as ion exchange chromatography or isoelectric focusing, are known to a person skilled in the art to detect either of the degradation products, i.e. succinimide, Asp or isoAsp. Most suitable for the quantification and the localization of degradation sites in proteins is the analysis via liquid chromatography tandem mass spectrometry (LC-MS/MS).

Wolfguy Numbering Scheme

The Wolfguy numbering defines CDR regions as the set union the Kabat and Chothia definition. Furthermore, the numbering scheme annotates CDR loop tips based on CDR lenght ( and partly based on sequnce) so that the index of a CDR position indicated if a CDR residue is part of the ascending or the descending loop. A comparison with established numbering schemes is shown in the following Table.

**Table:** Numbering of CDR-L3 and CDR-H3 using Chothia/Kabat (Ch-Kb), Honegger and Wolfguy numbering schemes. The latter has increasing numbers from the N-terminal basis to the CDR peak and decreasing ones starting from the C-terminal CDR end. Kabat schemes fix the two last CDR residues and introduce letters to accommodate for the CDR lenght. In contrast to Kabat nomenclature, the Noegger numbering does not use letters and is common for VH and VL.

| | | | | |
|---|---|---|---|---|
| 326 | 88 | 102 | 84 | 730 |
| 327 | 89 | 103 | 85 | 731 |
| 328 | 90 | 104 | 86 | 732 |
| 329 | 91 | 105 | 87 | 733 |
| 330 | 92 | C | 88 | 734 |
| 331 | 93 | 107 | 89 | 751 |
| 332 | 94 | 108 | 90 | 752 |
| 351 | 95 | 109 | 91 | 753 |
| 352 | 96 | 110 | 92 | 754 |
| 353 | 97 | 111 | 93 | 755 |
| 354 | 98 | 112 | 94 | 756 |
| 355 | 99 | 113 | 95 | 757 |
| 356 | 100 | 114 | 95a | 758 |

(continued)

| | | | | |
|---|---|---|---|---|
| 357 | 100a | 115 | 95b | 759 |
| 358 | 100b | 116 | 95c | 760 |
| 359 | 100c | 117 | 95d | 761 |
| 360 | 100d | 118 | 95e | 762 |
| 361 | 100e | 119 | 95f | 763 |
| 362 | 100f | 120 | | 764 |
| 363 | 100g | 121 | | 765 |
| 364 | 100h | 122 | | 766 |
| 384 | 100i | 123 | | 784 |
| 385 | 100i | 124 | | 785 |
| 386 | 100k | 125 | | 786 |
| 387 | 100l | 126 | | 787 |
| 388 | | 127 | | 788 |
| 389 | | 128 | | 789 |
| 390 | | 129 | | 790 |
| 391 | | 130 | | 791 |
| 392 | | 131 | | 792 |
| 393 | | 132 | | 793 |
| 394 | | 133 | | 794 |
| 395 | | 134 | | 795 |
| 396 | | 135 | | 796 |
| 397 | | 136 | | 797 |
| 398 | 101 | 137 | 96 | 798 |
| 399 | 102 | 138 | 97 | 799 |
| 401 | 103 | FW | 98 | 801 |
| 402 | 104 | 140 | 99 | 802 |
| 403 | 105 | 141 | 100 | 803 |
| 404 | 106 | 142 | 101 | 804 |
| **Wolfguy VH** | **Ch-Kb** | **Honegger** | **Ch-Kb** | **Wolfguy VL** |

Wolfguy os designed such that structurally equivalent residues (i.e residues that are very similar in terms of conserved spatial localization in the Fv structure) are numbered with equivalent indices as far as possible. This is illustrated in Figure 1. An example for a Wolfguy-numbered full-length VH and VL sequence can be found in the following Table.

**Table:** VH (left) and VL (right) sequence of the crystal structure with PDB ID 3PP4 (21), numbered with Wolfguy, Kabat and Chothia. In Wolfguy, CDR-H1-H3, CDR-L2 and CDR-L3 are numbered depending only on length, while CDR-L1 is numbered depending on loop length and canonical cluster membership. The latter is determined by calculating sequence similarities to different consensus sequences. Here, we only give a single example of CDR-L1 numbering, as it is of no importance for generating our VH-VL orientation sequence fingerprint.

| PDB ID 3PP4 VH | | | | | | |
|---|---|---|---|---|---|---|
| | Wolfguy | | Kabat | | Chothia | |
| Framework 1 | 101 | Q | 1 | Q | 1 | Q |
| | 102 | V | 2 | V | 2 | V |
| | 103 | Q | 3 | Q | 3 | Q |
| | 104 | L | 4 | L | 4 | L |
| | 105 | V | 5 | V | 5 | V |
| | 106 | Q | 6 | Q | 6 | Q |
| | 107 | S | 7 | S | 7 | S |
| | 108 | G | 8 | G | 8 | G |
| | 109 | A | 9 | A | 9 | A |
| | 110 | E | 10 | E | 10 | E |
| | 111 | V | 11 | V | 11 | V |
| | 112 | K | 12 | K | 12 | K |
| | 113 | K | 13 | K | 13 | K |
| | 114 | P | 14 | P | 14 | P |
| | 115 | G | 15 | G | 15 | G |
| | 116 | S | 16 | S | 16 | S |
| | 117 | S | 17 | S | 17 | S |
| | 118 | V | 18 | V | 18 | V |
| | 119 | K | 19 | K | 19 | K |
| | 120 | V | 20 | V | 20 | V |
| | 121 | S | 21 | S | 21 | S |
| | 122 | C | 22 | C | 22 | C |
| | 123 | K | 23 | K | 23 | K |
| | 124 | A | 24 | A | 24 | A |
| | 125 | S | 25 | S | 25 | S |
| CDR-H1 | 151 | G | 26 | G | 26 | G |
| | 152 | Y | 27 | Y | 27 | Y |
| | 153 | A | 28 | A | 28 | A |
| | 154 | F | 29 | F | 29 | F |
| | 155 | S | 30 | S | 30 | S |
| | 156 | Y | 31 | Y | 31 | Y |
| | 157 | . | 32 | S | 31a | . |
| | 158 | . | 33 | W | 31b | . |
| | 193 | . | 34 | I | 31c | . |
| | 194 | . | 35 | N | 31d | . |
| | 195 | . | 35a | . | 31e | . |
| | 196 | S | 35b | . | 32 | S |
| | 197 | W | 35c | . | 33 | W |
| | 198 | I | 35d | . | 34 | I |

| PDB ID 3PP4 VL | | | | | | |
|---|---|---|---|---|---|---|
| | Wolfguy | | Kabat | | Chothia | |
| Framework 1 | 501 | D | 1 | D | 1 | D |
| | 502 | I | 2 | I | 2 | I |
| | 503 | V | 3 | V | 3 | V |
| | 504 | M | 4 | M | 4 | M |
| | 505 | T | 5 | T | 5 | T |
| | 506 | Q | 6 | Q | 6 | Q |
| | 507 | T | 7 | T | 7 | T |
| | 508 | P | 8 | P | 8 | P |
| | 509 | L | 9 | L | 9 | L |
| | 510 | S | 10 | S | 10 | S |
| | 511 | L | 11 | L | 11 | L |
| | 512 | P | 12 | P | 12 | P |
| | 513 | V | 13 | V | 13 | V |
| | 514 | T | 14 | T | 14 | T |
| | 515 | P | 15 | P | 15 | P |
| | 516 | G | 16 | G | 16 | G |
| | 517 | E | 17 | E | 17 | E |
| | 518 | P | 18 | P | 18 | P |
| | 519 | A | 19 | A | 19 | A |
| | 520 | S | 20 | S | 20 | S |
| | 521 | I | 21 | I | 21 | I |
| | 522 | S | 22 | S | 22 | S |
| | 523 | C | 23 | C | 23 | C |
| CDR-L1 | 551 | R | 24 | R | 24 | R |
| | 552 | S | 25 | S | 25 | S |
| | 553 | S | 26 | S | 26 | S |
| | 556 | K | 27 | K | 27 | K |
| | 561 | S | 27a | S | 28 | S |
| | 562 | L | 27b | L | 29 | L |
| | 563 | L | 27c | L | 30 | L |
| | 581 | H | 27d | H | 30a | H |
| | 582 | S | 27e | S | 30b | S |
| | 583 | N | 28 | N | 30c | N |
| | 594 | G | 29 | G | 30d | G |
| | 595 | I | 30 | I | 30e | I |
| | 596 | T | 31 | T | 31 | T |
| | 597 | Y | 32 | Y | 32 | Y |
| | 598 | L | 33 | L | 33 | L |
| | 599 | Y | 34 | Y | 34 | Y |

| | | | | | | |
|---|---|---|---|---|---|---|
| | 199 | N | 35e | . | 35 | N |
| Framework 2 | 201 | W | 36 | W | 36 | W |
| | 202 | V | 37 | V | 37 | V |
| | 203 | R | 38 | R | 38 | R |
| | 204 | Q | 39 | Q | 39 | Q |
| | 205 | A | 40 | A | 40 | A |
| | 206 | P | 41 | P | 41 | P |
| | 207 | G | 42 | G | 42 | G |
| | 208 | Q | 43 | Q | 43 | Q |
| | 209 | G | 44 | G | 44 | G |
| | 210 | L | 45 | L | 45 | L |
| | 211 | E | 46 | E | 46 | E |
| | 212 | W | 47 | W | 47 | W |
| | 213 | M | 48 | M | 48 | M |
| | 214 | G | 49 | G | 49 | G |
| CDR-H2 | 251 | R | 50 | R | 50 | R |
| | 252 | I | 51 | I | 51 | I |
| | 253 | F | 52 | F | 52 | F |
| | 254 | P | 52a | P | 52a | P |
| | 255 | G | 52b | . | 52b | . |
| | 256 | . | 52c | . | 52c | . |
| | 286 | . | 52d | . | 52d | . |
| | 287 | . | 53 | G | 53 | G |
| | 288 | D | 54 | D | 54 | D |
| | 289 | G | 55 | G | 55 | G |
| | 290 | D | 56 | D | 56 | D |
| | 291 | T | 57 | T | 57 | T |
| | 292 | D | 58 | D | 58 | D |
| | 293 | Y | 59 | Y | 59 | Y |
| | 294 | N | 60 | N | 60 | N |
| | 295 | G | 61 | G | 61 | G |
| | 296 | K | 62 | K | 62 | K |
| | 297 | F | 63 | F | 63 | F |
| | 298 | K | 64 | K | 64 | K |
| | 299 | G | 65 | G | 65 | G |
| Framework 3 | 301 | R | 66 | R | 66 | R |
| | 302 | V | 67 | V | 67 | V |
| | 303 | T | 68 | T | 68 | T |
| | 304 | I | 69 | I | 69 | I |
| | 305 | T | 70 | T | 70 | T |
| | 306 | A | 71 | A | 71 | A |
| | 307 | D | 72 | D | 72 | D |
| | 308 | K | 73 | K | 73 | K |
| | 309 | S | 74 | S | 74 | S |
| | 310 | T | 75 | T | 75 | T |
| | 311 | S | 76 | S | 76 | S |
| | 312 | T | 77 | T | 77 | T |
| | 313 | A | 78 | A | 78 | A |
| | 314 | Y | 79 | Y | 79 | Y |
| | 315 | M | 80 | M | 80 | M |
| | 316 | E | 81 | E | 81 | E |
| | 317 | L | 82 | L | 82 | L |
| | 318 | S | 82a | S | 82a | S |
| | 319 | S | 82b | S | 82b | S |
| | 320 | L | 82c | L | 82c | L |
| | 321 | R | 83 | R | 83 | R |
| | 322 | S | 84 | S | 84 | S |
| | 323 | E | 85 | E | 85 | E |
| | 324 | D | 86 | D | 86 | D |

| | | | | | | |
|---|---|---|---|---|---|---|
| Framework 2 | 601 | W | 35 | W | 35 | W |
| | 602 | Y | 36 | Y | 36 | Y |
| | 603 | L | 37 | L | 37 | L |
| | 604 | Q | 38 | Q | 38 | Q |
| | 605 | K | 39 | K | 39 | K |
| | 606 | P | 40 | P | 40 | P |
| | 607 | G | 41 | G | 41 | G |
| | 608 | Q | 42 | Q | 42 | Q |
| | 609 | S | 43 | S | 43 | S |
| | 610 | P | 44 | P | 44 | P |
| | 611 | Q | 45 | Q | 45 | Q |
| | 612 | L | 46 | L | 46 | L |
| | 613 | L | 47 | L | 47 | L |
| | 614 | I | 48 | I | 48 | I |
| | 615 | Y | 49 | Y | 49 | Y |
| CDR-L2 | 651 | Q | 50 | Q | 50 | Q |
| | 652 | . | * | . | * | . |
| | 653 | . | * | . | * | . |
| | 692 | . | * | . | * | . |
| | 693 | . | * | . | * | . |
| | 694 | M | 51 | M | 51 | M |
| | 695 | S | 52 | S | 52 | S |
| | 696 | N | 53 | N | 53 | N |
| | 697 | L | 54 | L | 54 | L |
| | 698 | V | 55 | V | 55 | V |
| | 699 | S | 56 | S | 56 | S |
| Framework 3 | 701 | G | 57 | G | 57 | G |
| | 702 | V | 58 | V | 58 | V |
| | 703 | P | 59 | P | 59 | P |
| | 704 | D | 60 | D | 60 | D |
| | 705 | R | 61 | R | 61 | R |
| | 706 | F | 62 | F | 62 | F |
| | 707 | S | 63 | S | 63 | S |
| | 708 | G | 64 | G | 64 | G |
| | 709 | S | 65 | S | 65 | S |
| | 710 | G | 66 | G | 66 | G |
| | 711 | S | 67 | S | 67 | S |
| | 712 | G | 68 | G | 68 | G |
| | 713 | . | * | . | * | . |
| | 714 | . | * | . | * | . |
| | 715 | T | 69 | T | 69 | T |
| | 716 | D | 70 | D | 70 | D |
| | 717 | F | 71 | F | 71 | F |
| | 718 | T | 72 | T | 72 | T |
| | 719 | L | 73 | L | 73 | L |
| | 720 | K | 74 | K | 74 | K |
| | 721 | I | 75 | I | 75 | I |
| | 722 | S | 76 | S | 76 | S |
| | 723 | R | 77 | R | 77 | R |
| | 724 | V | 78 | V | 78 | V |
| | 725 | E | 79 | E | 79 | E |
| | 726 | A | 80 | A | 80 | A |
| | 727 | E | 81 | E | 81 | E |
| | 728 | D | 82 | D | 82 | D |
| | 729 | V | 83 | V | 83 | V |
| | 730 | G | 84 | G | 84 | G |
| | 731 | V | 85 | V | 85 | V |
| | 732 | Y | 86 | Y | 86 | Y |
| | 733 | Y | 87 | Y | 87 | Y |

| | | | | | | |
|---|---|---|---|---|---|---|
| | 325 | T | 87 | T | 87 | T |
| | 326 | A | 88 | A | 88 | A |
| | 327 | V | 89 | V | 89 | V |
| | 328 | Y | 90 | Y | 90 | Y |
| | 329 | Y | 91 | Y | 91 | Y |
| | 330 | C | 92 | C | 92 | C |
| | 331 | A | 93 | A | 93 | A |
| | 332 | R | 94 | R | 94 | R |
| CDR-H3 | 351 | N | 95 | N | 95 | N |
| | 352 | V | 96 | V | 96 | V |
| | 353 | F | 97 | F | 97 | F |
| | 354 | D | 98 | D | 98 | D |
| | 355 | G | 99 | G | 99 | G |
| | 356 | . | 100 | Y | 100 | Y |
| | 357 | . | 100a | W | 100a | W |
| | 358 | . | 100b | L | 100b | L |
| | 359 | . | 100c | . | 100c | . |
| | 360 | . | 100d | . | 100d | . |
| | 361 | . | 100e | . | 100e | . |
| | 362 | . | 100f | . | 100f | . |
| | 363 | . | 100g | . | 100g | . |
| | 364 | . | 100h | . | 100h | . |
| | 365 | . | 100i | . | 100i | . |
| | 385 | . | 100j | . | * | . |
| | 386 | . | 100k | . | * | . |
| | 387 | . | 100l | . | * | . |
| | 388 | . | 100m | . | * | . |
| | 389 | . | 100n | . | * | . |
| | 390 | . | 100o | . | * | . |
| | 391 | . | 100p | . | * | . |
| | 392 | . | 100q | . | * | . |
| | 393 | . | 100r | . | * | . |
| | 394 | . | 100s | . | * | . |
| | 395 | Y | 100t | . | * | . |
| | 396 | W | 100u | . | * | . |
| | 397 | L | 100v | . | * | . |
| | 398 | V | 101 | V | 101 | V |
| | 399 | Y | 102 | Y | 102 | Y |
| Framework 4 | 401 | W | 103 | W | 103 | W |
| | 402 | G | 104 | G | 104 | G |
| | 403 | Q | 105 | Q | 105 | Q |
| | 404 | G | 106 | G | 106 | G |
| | 405 | T | 107 | T | 107 | T |
| | 406 | L | 108 | L | 108 | L |
| | 407 | V | 109 | V | 109 | V |
| | 408 | T | 110 | T | 110 | T |
| | 409 | V | 111 | V | 111 | V |
| | 410 | S | 112 | S | 112 | S |
| | 411 | S | 113 | S | 113 | S |

| | | | | | | |
|---|---|---|---|---|---|---|
| | 734 | C | 88 | C | 88 | C |
| CDR-L3 | 751 | A | 89 | A | 89 | A |
| | 752 | Q | 90 | Q | 90 | Q |
| | 753 | N | 91 | N | 91 | N |
| | 754 | L | 92 | L | 92 | L |
| | 755 | E | 93 | E | 93 | E |
| | 756 | . | 94 | L | 94 | L |
| | 757 | . | 95 | P | 95 | P |
| | 758 | . | 95a | . | 95a | . |
| | 793 | . | 95b | . | 95b | . |
| | 794 | . | 95c | . | 95c | . |
| | 795 | . | 95d | . | 95d | . |
| | 796 | L | 95e | . | 95e | . |
| | 797 | P | 95f | . | 95f | . |
| | 798 | Y | 96 | Y | 96 | Y |
| | 799 | T | 97 | T | 97 | T |
| Framework 4 | 801 | F | 98 | F | 98 | F |
| | 802 | G | 99 | G | 99 | G |
| | 803 | G | 100 | G | 100 | G |
| | 804 | G | 101 | G | 101 | G |
| | 805 | T | 102 | T | 102 | T |
| | 806 | K | 103 | K | 103 | K |
| | 807 | V | 104 | V | 104 | V |
| | 808 | E | 105 | E | 105 | E |
| | 809 | I | 106 | I | 106 | I |
| | 810 | K | 107/106 | K | 107 | K |

Next Generation Sequencing (NGS)

(a) sample preparation

In certain aspects, the method as reported herein comprises, in part, amplifying one or more regions of interest from a biological sample comprising nucleic acid. The amplification generates a plurality of amplicons for each region of interest.

Amplification takes place in the presence of one or more primer pairs. A first primer of the primer pair comprises a sequence complementary to an upstream portion of the region of interest and a second primer of the primer pair comprises a sequence complementary to a downstream portion of the region of interest. The primer pairs are designed to anneal to complementary strands of nucleic acid (i.e. one primer of the primer pair anneals to the sense strand and one primer of the primer pair anneals to the antisense strand). The complementary sequence may be altered based on the region of interest to be amplified. The complementary sequences of the primer pair may comprise about 10 to about 100 nucleotides complementary to the region of interest.

One or more primer pairs are contacted with a sample comprising nucleic acid. Nucleic acid may be, for example, RNA or DNA. Modified forms of RNA or DNA may be used. In one exemplary embodiment, the nucleic acid is cDNA.

In general, amplification of the region of interest is carried out using polymerase chain reaction (PCR). A PCR reaction may comprise sample comprising nucleic acid, one or more primer pairs, polymerase, water, buffer, and deoxynucleotide triphosphates (dNTPs) in a single reaction vial. PCR may be performed according to standard methods in the art. By way of non-limiting example, the PCR reaction may comprise denaturation, followed by about 15 to about 30 cycles of denaturation, annealing and extension, followed by a final extension.

(b) sequencing library preparation

In certain aspects, the method as reported herein comprises, in part, attaching an adapter, and/or index sequence to each amplicon or product generated in Section (a).

In one embodiment, the nucleotide sequence comprising an adapter, a random component and/or an index sequence is attached to an amplicon or product via PCR. For example, the amplicon or product may be contacted with a nucleotide sequence comprising an adaptor and an index sequence and a PCR reaction is conducted. Then, this product is contacted with a nucleotide sequence comprising an adaptor and a PCR reaction is conducted. The resulting product is a nucleotide sequence comprising an adaptor, a region of interest, an index sequence and a downstream adaptor. Alternatively, the amplicon or product may be contacted with a nucleotide sequence comprising an adaptor and a random component and a PCR reaction is conducted. Then, this product is contacted with a nucleotide sequence comprising an adaptor and an index sequence and a PCR reaction is conducted. The resulting product is a nucleotide sequence comprising an adaptor, an index sequence, a region of interest, a random component and a downstream adaptor.

The products or amplicons comprising an adapter, a random component and/or an index sequence are then subjected to exponential PCR. In an embodiment, an exponential PCR reaction may comprise the products or amplicons comprising an adapter, a random component and/or an index sequence, primers, polymerase, water, buffer, and deoxynucleotide triphosphates (dNTPs) in a single reaction vial. Exponential PCR may be performed according to standard methods in the art. By way of non-limiting example, the exponential PCR reaction may comprise denaturation, followed by about 15-30 cycles of denaturation, annealing and extension, followed by a final extension.

Upon performing exponential PCR, the products or amplicons comprising an adapter, and/or an index sequence are amplified. The exponential PCR products comprise: an adapter, a region of interest, a downstream adapter and an index sequence.

(c) sequencing

In certain aspects, the method as reported herein comprises, in part, sequencing the exponential PCR product. The sequencing of the exponential PCR product generates redundant reads. The redundant reads are grouped by random component and a consensus sequence is identified such that the redundant reads mitigate sequence errors.

Sequencing may be performed according to standard methods in the art. Sequencing is preferably performed on a massively parallel sequencing platform, many of which are commercially available including, but not limited to Illumina, Roche/454, Ion Torrent, and PacBIO. In an exemplary embodiment, Illumina sequencing is used.

Reads may be separated by the index sequence and trimmed to remove primer sequences. Reads may be grouped by the random component. In certain embodiments, groups of reads with less than three, less than four, or less than five reads may be removed. To eliminate ambiguous sequences, the random components may be sorted by abundance and clustered at an identity of about 85%. Alternatively, the random components may be sorted by abundance and clustered at an identity of about 65% to about 95%. The random components may be clustered from most abundant to least abundant. Given that most sequencing errors are random and that the correct sequence should occur more often than a variant with sequencing errors, the abundance-weighted clustering provides a means to eliminate spurious random components that are most likely due to sequencing errors while retaining the more abundant (and most likely true positive) random components.

This redundant sequencing of each amplicon or product allows the error-correction of each amplicon or product. For example, a consensus sequence is generated for each random component group by scoring and weighing the nucleotide at each base position. Sequences with a consensus sequence that is identical to the most abundant sequence associated with the same random component are kept; this process is called quality filtering. Specifically, at every position, the nucleotides called by each sequence read are compared and a consensus nucleotide is called if there is at least about 90% agreement between the reads. If there is less than about 90% agreement, an "N" is called in the consensus sequence at that position.

(d) comparison to reference sequence

After an error-corrected consensus sequence (ECCS) has been identified, the ECCS may be compared to a reference sequence to determine the presence of one or more differences. A reference sequence may be a sequence of an antibody for which variants with the same biological properties but without negative features, such as developability issues (hot-spots), are searched for.

**THE METHOD AS REPORTED HEREIN**

In the art a back-up/replacement candidate is generally identified either by introducing mutations in the amino acid sequence to address, e.g., developability liabilities of the original candidate or by performing a de-novo screening of deselected antibodies. In the first case it cannot be excluded that by removing the developability liability also the binding and/or therapeutic properties of the antibody can be affected. In the latter case it is questionable if a true replacement candidate can be found.

By using next generation sequencing (NGS) the number of B-cell clones that can be sequenced is dramatically increased compared to previous methods. But only clones showing promising properties in the early screening will be further pursued. For the other clones the sequence data will simply be stored. That is, not all of the sequenced clones will be expressed and fully characterized.

It has now been found that this unused potential of the NGS data obtained in a project can be used to identify one or more replacement candidates for a lead candidate, wherein the replacement candidates do not have e.g. the developability liability/liabilities of the original lead antibody, such as e.g. amino acid residues prone to post-translational modification or isolated cysteine-residues. This identification and selection is achieved by using the NGS sequence information to identify an antibody closely related to the selected candidate based on the amino acid/nucleotide sequence and at the same time maintaining at least one of the biological/binding properties of the lead candidate, i.e. the reference antibody comprising the developability hot-spot.

In one embodiment of all aspects, a high-diversity library is included within the original antigen-specific library, in order to increase library diversity and allow a better sequencing. For example, the genomic PhiX library, sold by Illumina Inc., may be used.

In one embodiment of all aspects, both the amplification and the sequencing steps are performed using the Illumina technology. In this case, during the amplification step specific subsequences, called adaptors and required for the subsequent step of sequencing, are added to the nucleotide fragments according to Illumina's instructions.

In one preferred embodiment of all aspects two PCRs are performed before sequencing.

Sequencing is in one embodiment performed using the commercially available MiSeq kit (Illumina Inc.).

In one embodiment of all aspects sequencing is performed using a paired-end sequencing method, in which both ends of a nucleotide fragment are simultaneously sequenced. This allows for a more rapid sequencing and is particularly useful in the case of long fragments.

In one embodiment of all aspects a multiplexing technique is used so that different samples can be sequenced during the same run, thus allowing a rapid and simultaneous analysis of many combinations of sample/library. To perform multiplexing, typically, specific sequences (barcodes or indexes) are added during an amplification step using specific PCR primers.

For example, the commercially available MiSeq kit provides for both paired-end sequencing and multiplexing.

Analysis of the sequencing data can be performed by a software able to process the data generated by the sequencing. Suitable software tools are commercially available. An example of a suitable software is BWA (Burrows-Wheeler Aligner), see the work of Li H. and Durbin R. (2009) (Li H. and Durbin R. (2009) Fast and accurate short read alignment with Burrows-Wheeler Transform. Bioinformatics, 25:1754-60). Another suitable software is FastQC (see website: www.bioinformatics.babraham.ac.uk/projects/fastqc/), which is a tool for analyzing and controlling high throughput sequence data.

In other cases, in accordance with the methods as reported herein, one or more of the consensus sequence-specific primers may further comprise a tag and/or adaptor.

The current method comprises in one specific embodiment the following general steps:

- assembly of paired (Illumina MiSeq) reads, e.g. by a computer program (optionally including corrections: Molecular Identifier Group-based Error Correction (MiGec) software pipeline that allows UMI-barcode extraction and error correction based on consensus building of the complete VH sequence);

- extraction of antibody variable domains, optionally including correction: sequence replicas correction, considering only n≥3 CDR3 clusters, signal peptide detection and quality assessment;
- structure-guided clustering and ranking of antibody variants; and
- ranking mutations versus a reference sequence for their ability to retain VH/VL pairing, binding and function, optionally the ranking is based on matrices and depends also on the conservation of the mutation(s).

**Structure-guided Clustering and Ranking of Antibody Variants (SCaRAb)**

Antibody variable region variant characterization

The method as reported herein is based on a given reference antibody for which the heavy chain variable region (VH) sequence and the light chain variable region (VL) sequence are known (subsequently denoted as the "reference" or "reference antibody").

In addition, there exists a set of known VH and/or VL sequences that can be considered to be related to the reference antibody's VH and/or VL sequences (subsequently denoted as the "variants" or "variant antibodies"), e.g. derived from B-cells obtained in the same immunization campaign as the reference antibody. The relation between the reference and its variants is/can be based, for example, on i) identical lengths of VH and VL sequence, ii) identical lengths of all β-sheet framework regions, or/and iii) identical lengths of all complementarity-determining regions (CDRs).

The criteria for the alignment/selection of the respective sequences depends on the available data pool. If a limited number of variants is available the criteria should be less stringent, whereas when a high number of variants is available the criteria can be/should be more stringent (in order to identify the best possible variant). For example, the number of mutations in the entire VH and/or VL could be used as criterion with less than 10, less than 9, less than 8, less than 7, less than 6, less than 5, less than 4 allowed. But it has to be noted that with the number of allowed mutations the likelihood that the variant has not the same/comparable binding properties as the reference antibody is increasing. Alternatively, it is possible to use criteria such as less than "x" mutations in HVR3/CDR3, less than "y" mutations in all HVRs/CDRs, less than "z" mutations in all FRs, less than "n" mutations in total, or combinations thereof on amino acid or/and nucleic acid level.

If the criterion for the selection is the removal of developability hot-spots the alignment/selection criteria could be:

- without (free) cysteine in the HVRs/CDRs
- without degradation prone Asp, Asn, Met residues/motifs in the variable domains.

Both the reference sequences as well as their variants are then annotated with an established annotation scheme, such as e.g. the Wolfguy antibody numbering scheme (Bujotzek, A., et al., Prot. Struct. Funct. Bioinform., 83 (2015) 681-695). This is used throughout the method, i.e. it is used for all and any annotation of positions in the method as reported herein. An example for a Wolfguy-annotated VH reference sequence and its variants can be found in Figure 1.

Once the annotated VH and VL sequences have been aligned based on the residue indices, the variants can be described in terms of a compact "mutation tuple", which describes only the sequence differences with regard to the reference (i.e., type and location of substituted amino acids). This principle is illustrated in the following Table using VH variants of antibody 763.

**Table:** Mutation tuple notation for six VH variants of reference antibody 763 (see also Figure 1). The mutation tuple is a concatenated series of strings, each specifying i) the amino acid type found in the reference sequence (1-letter code), ii) the Wolfguy position at which the amino acid is located, and iii) the amino acid type of the sequence variant (1-letter code), if it not matches the one of the reference sequence.

| VH variant name | Number of exchanged amino acids | Mutation tuple |
|---|---|---|
| 1101:11801:20894 | 1 | T322P |
| 1101:1836:14971 | 3 | Y293D,S295N,T322P |
| 1114:12508:8829 | 5 | Q102P,S103P,S125P,Y196S, R306K |

(continued)

| VH variant name | Number of exchanged amino acids | Mutation tuple |
|---|---|---|
| 1114:28111:18680 | 5 | S103L,N197K,T322P,F329V, V331G |
| 2103:7194:16368 | 2 | E211A,R306K |
| 2106:2910:18342 | 1 | R306K |

## Clustering

Because a given set of sequence variants can be large and redundant, it is meaningful to perform a clustering that identifies variants that are identical or similar with regard to number, location and type of the respective amino acid substitutions. For this purpose, e.g., the well-established k-medoids clustering algorithm can be used.

For the current case (antibody variable domains), a suitable lower boundary for k is the number of different mutation tuple lengths in the dataset. For example, if the dataset to cluster consists of the variants (T322P), (R306K), (E211A,R306K), and (Q102P,S103P,S125P,Y196S,R306K), the minimum value for k should be set to 3, with allows for separate clusters for tuple length one, two, and five. If desired, the value of k can be increased further to realize a finer clustering based on where the amino acid substitutions are located.

To perform a k-medoids-based clustering of sequence variants described by mutation tuples, a novel distance metric that incorporates both the antibody-specific location of the amino acid substitution, as well as the approximate physico-chemical properties of the exchange, can be devised. The latter is quantified using the BLOSUM62 amino acid substitution matrix (Henikoff, S. and Henikoff, J.G., Proc. Nat. Acad. Sci., 89 (1992) 10915-10919).

This is done as outlined in the following.

Given the two exemplary mutation tuples (E211A,R306K) and (Y293D, S295N, T322P):

1. Calculate the location-based distance matrix between the two tuples (distance in residue numbers: 293-211=82 etc.):

| | E211A | R306K |
|---|---|---|
| Y293D | 82 | 13 |
| S295N | 84 | 11 |
| T322P | 111 | 16 |

2. Pick the mutation pair with the minimum distance: S295N and R306K (residue number distance of 11). The distance contribution for this pair is $(11 + \text{abs}(\text{BLOSUM62}(S,N) - \text{BLOSUM62}(R,K)))^2$.
Update the distance matrix so that the taken pair is removed:

| | E211A | R306K |
|---|---|---|
| Y293D | 82 | inf |
| S295N | inf | inf |
| T322P | 111 | inf |

Repeat procedure (step 2) until all mutations have been paired.

3. If the number of mutations per tuple does not match, unpaired mutations will remain (in this example, T322P). To account for the unpaired mutation T322P, add to the distance

$$(310 + \text{abs}(\text{BLOSUM62}(T,T) - \text{BLOSUM62}(T,P)))^2$$

The value 310 is the theoretical maximum distance in terms of VH Wolfguy indices (411-101). Repeat procedure (3) until all unpaired mutations are accounted for.

In this example, the distance between the two mutation tuples is:

$$\text{mutation_tuple_dist[ (E211A,R306K), (Y293D,S295N,T322P) ]} =$$
$$\text{sqrt}((11 + \text{abs}(\text{BLOSUM62}(S, N) - \text{BLOSUM62}(R, K)))^2 +$$
$$(82 + \text{abs}(\text{BLOSUM62}(Y, D) - \text{BLOSUM62}(E, A)))^2 +$$
$$(310 + \text{abs}(\text{BLOSUM62}(T, T) - \text{BLOSUM62}(T, P)))^2 )$$

$$= \text{sqrt}(12^2 + 84^2 + 316^2) \approx 327.194$$

## Mutation risk score

The mutation risk score is a means to quantify the risk that an antibody variant, specified by a mutation tuple as defined above, will lose the function that is displayed by the reference antibody (where the function is typically binding affinity towards a given target/antigen). The mutation risk score always takes negative values, and larger negative values indicate a larger risk of loss of function.

The mutation risk score incorporates the BLOSUM62 matrix to rate the severity of the amino acid substitution in terms of the resulting changes in biochemical properties such as charge, hydrophobicity and size. Furthermore, the mutation risk score contains an antibody variable region position-specific weighting factor to account for the location where the amino acid substitution occurs. For example, residues belonging to the CDRs typically involved in antigen binding are weighted higher than peripheral residues that are unlikely to be involved in antigen binding.

For a given mutation tuple consisting of *n* mutation strings of the form $X_i pos_i Y_i$, the mutation risk score is calculated as follows:

$$Mutant\ Risk\ Score = \sum_{i=1}^{n} \begin{cases} \frac{-1}{BLOSUM62(X_i, Y_i)} * weight(pos_i) & \} BLOSUM62(X_i, Y_i) > 0 \\ -2 * weight(pos_i) & \} BLOSUM62(X_i, Y_i) = 0 \\ -2 + BLOSUM62(X_i, Y_i) * weight(pos_i) & \} BLOSUM62(X_i, Y_i) < 0 \end{cases}$$

The following Table specifies the antibody-specific weights for the conserved positions of the VH domain. Residues that are not explicitly given in this Table are weighted with the value one. Residues are numbered with the Wolfguy numbering scheme.

**Table**: Antibody-specific weights for the conserved positions of the VH domain for the framework (left) and CDR regions (right).

| | Wolfguy Index | Weight | | Wolfguy Index | Weight |
|---|---|---|---|---|---|
| Framework 1 | 101 | 0.2 | CDR-H1 | 151 | 2 |
| | 102 | 1.1 | | 152 | 2.6 |
| | 103 | 0 | | 153 | 1.2 |
| | 104 | 0.5 | | 154 | 2.3 |
| | 105 | 0.2 | | 155 | 1.9 |
| | 106 | 0.8 | | 156 | 3.7 |
| | 107 | 0.5 | | 157 | 4 |
| | 108 | 0.8 | | 158 | 4 |
| | 109 | 0.4 | | 193 | 4 |
| | 110 | 0.2 | | 194 | 4 |
| | 111 | 0 | | 195 | 4 |
| | 112 | 0.4 | | 196 | 3.3 |
| | 113 | 0 | | 197 | 3.9 |
| | 114 | 0.1 | | 198 | 2.6 |
| | 115 | 0.6 | | 199 | 3.4 |
| | 116 | 0.1 | CDR-H2 | 251 | 3.8 |
| | 117 | 0.1 | | 252 | 1.9 |
| | 118 | 0.2 | | 253 | 4 |
| | 119 | 0.2 | | 254 | 3.8 |
| | 120 | 1.2 | | 255 | 3.6 |
| | 121 | 0 | | 256 | 4 |
| | 122 | 4 | | 287 | 4 |
| | 123 | 0 | | 288 | 4 |
| | 124 | 2 | | 289 | 3.9 |
| | 125 | 0.5 | | 290 | 3.4 |
| Framework 2 | 201 | 4 | | 291 | 3.7 |
| | 202 | 2.6 | | 292 | 2.1 |
| | 203 | 2 | | 293 | 3.6 |
| | 204 | 1.7 | | 294 | 2.3 |
| | 205 | 1 | | 295 | 2.3 |
| | 206 | 0 | | 296 | 1 |
| | 207 | 0 | | 297 | 1.2 |
| | 208 | 0.7 | | 298 | 2.4 |
| | 209 | 1.6 | | 299 | 0.5 |
| | 210 | 2 | CDR H-3 | 351 | 4 |
| | 211 | 1.3 | | 352 | 3.5 |
| | 212 | 3.1 | | 353 | 4 |
| | 213 | 0.9 | | 354 | 3.5 |
| | 214 | 3.1 | | 355 | 3.5 |
| Framework 3 | 301 | 0.1 | | 356 | 3 |
| | 302 | 1.2 | | 357 | 3 |
| | 303 | 1.7 | | 358 | 3 |
| | 304 | 0.3 | | 359 | 3 |
| | 305 | 1.8 | | 360 | 3 |

| | | | | |
|---|---|---|---|---|
| | 306 | 0 | 361 | 3 |
| | 307 | 2.4 | 362 | 3 |
| | 308 | 0 | 363 | 3 |
| | 309 | 1.2 | 364 | 3 |
| | 334 | 1 | 365 | 3 |
| | 335 | 1 | 366 | 3 |
| | 336 | 1 | 367 | 3 |
| | 337 | 1 | 382 | 3 |
| | 310 | 0.8 | 383 | 3 |
| | 311 | 0.9 | 384 | 3 |
| | 312 | 0.8 | 385 | 3 |
| | 313 | 2.5 | 386 | 3 |
| | 314 | 0.1 | 387 | 3 |
| | 315 | 1.5 | 388 | 3 |
| | 316 | 0 | 389 | 3 |
| | 317 | 1.5 | 390 | 3 |
| | 318 | 0.8 | 391 | 3 |
| | 319 | 0.1 | 392 | 3 |
| | 320 | 1.4 | 393 | 3 |
| | 321 | 0.2 | 394 | 3 |
| | 322 | 0.3 | 395 | 3.5 |
| | 323 | 0.2 | 396 | 3 |
| | 324 | 1.8 | 397 | 3.5 |
| | 325 | 0.6 | 398 | 1.5 |
| | 326 | 2.2 | 399 | 3 |
| | 333 | 1 | | |
| | 327 | 0.6 | | |
| | 328 | 3 | | |
| | 329 | 2.5 | | |
| | 330 | 4 | | |
| | 331 | 2.9 | | |
| | 332 | 2.8 | | |
| | 401 | 3.5 | | |
| | 402 | 2 | | |
| | 403 | 0.5 | | |
| | 404 | 1 | | |
| | 405 | 0.3 | | |
| | 406 | 0.1 | | |
| Framework 4 | 407 | 1 | | |
| | 408 | 0.1 | | |
| | 409 | 1 | | |
| | 410 | 0.1 | | |
| | 411 | 0.1 | | |

## ABangle distance

In addition to quantifying the mutation risk for a given variant, it is meaningful to assess if the variant is likely to preserve the VH-VL orientation of the reference antibody. The aim is to obtain antibody variants that retain the same antigen-binding properties and stability as the reference antibody. For this purpose, the VH-VL orientation has been characterized using the six ABangle orientation measures HL, HC1, LC1, HC2, LC2, and dc (five angular and one linear distance measure) defined by Dunbar et al. (Prot. Eng. Des. Sel. 26 (2013) 611-620). The individual ABangle values for the reference antibody and its variants are predicted using the machine learning-based approach described by Bujotzek et al. (Bujotzek, A., et al., Prot. Struct. Funct. Bioinform., 83 (2015) 681-695). In this approach, the parameters of VH-VL orientation are predicted from a sequence fingerprint of influential residues at the domain interface between VH and VL domain.

The ABangle concept

When making a comparison between any two amino acid based structures, generally distance-based metrics such as the root-mean-square deviation (RMSD) of equivalent atoms are used.

To characterize the orientation between any two three-dimensional objects, it is necessary to define:

- a frame of reference on each object.
- axes to measure orientation parameters about.
- terminology to describe and quantify these parameters.

The ABangle concept is a method which fully characterizes VH-VL orientation in a consistent and absolute sense using five angles (HL, HC1, LC1, HC2 and LC2) and a distance (dc). The pair of variable domains of an antibody, VH and VL, is denoted collectively as an antibody Fv fragment.

In a first step antibody structures are extracted from a data bank (e.g. the protein data bank, PDB). Chothia antibody numbering (Chothia and Lesk, 1987) is applied to each of the antibody chains. Chains that are successfully numbered are paired to form Fv regions. This is done by applying the constraint that the H37 position C$\alpha$ coordinate of the heavy chain (alpha carbon atom of the amino acid residue at heavy chain variable domain position 37) must be within 20 Å of the L87 position C$\alpha$ coordinate of the light chain. A non-redundant set of antibodies is created using CDHIT (Li, W. and Godzik, A. Bioinformatics, 22 (2006) 1658-1659), applying a sequence identity cut-off over the framework of the Fv region of 99 %.

The most structurally conserved residue positions in the heavy and light domains are used to define domain location. These positions are denoted as the VH and VL coresets. These positions are predominantly located on the $\beta$-strands of the framework and form the core of each domain. The coreset positions are given in the following Table:

| light chain | light chain | heavy chain | heavy chain |
|---|---|---|---|
| L44 | L35 | H35 | H17 |
| L19 | L37 | H12 | H72 |
| L69 | L74 | H38 | H92 |
| L14 | L88 | H36 | H84 |
| L75 | L38 | H83 | H91 |
| L82 | L18 | H19 | H90 |
| L15 | L87 | H94 | H20 |
| L21 | L17 | H37 | H21 |
| L47 | L86 | H11 | H85 |
| L20 | L85 | H47 | H25 |
| L48 | L46 | H39 | H24 |
| L49 | L70 | H93 | H86 |
| L22 | L45 | H46 | H89 |
| L81 | L16 | H45 | H88 |
| L79 | L71 | H68 | H87 |
| L80 | L72 | H69 | H22 |
| L23 | L73 | H71 | H23 |
| L36 | | H70 | |

The coreset positions are used to register frames of reference onto the antibody Fv region domains.

The VH domains in the non-redundant dataset are clustered using e.g. CDHIT, applying a sequence identity cut-off of 80 % over framework positions in the domain. One structure is randomly chosen from each of the 30 largest clusters. This set of domains is aligned over the VH coreset positions e.g. using Mammoth-mult (Lupyan, D., et al., Bioinf. 21 (2005) 3255-3263). From this alignment the Cα coordinates corresponding to the eight structurally conserved positions H36, H37, H38, H39, H89, H90, H91 and H92 in the β-sheet interface are extracted. Through the resulting 240 coordinates a plane is fitted. For the VL domain positions L35, L36, L37, L38, L85, L86, L87 and L88 are used to fit the plane.

The procedure described above allows mapping the two reference frame planes onto any Fv structure. Therefore, the measuring of the VH-VL orientation can be made equivalent to measuring the orientation between the two planes. To do this fully and in an absolute sense requires at least six parameters: a distance, a torsion angle and four bend angles. These parameters must be measured about a consistently defined vector that connects the planes. This vector is denoted C in the following. To identify C, the reference frame planes are registered onto each of the structures in the non-redundant set as described above and a mesh placed on each plane. Each structure therefore has equivalent mesh points and, thus, equivalent VH-VL mesh point pairs. The Euclidean distance is measured for each pair of mesh points in each structure. The pair of points with the minimum variance in their separation distance is identified. The vector which joins these points is defined as C.

The coordinate system is fully defined using vectors, which lie in each plane and are centered on the points corresponding to C. H1 is the vector running parallel to the first principal component of the VH plane, while H2 runs parallel to the second principal component. L1 and L2 are similarly defined on the VL domain. The HL angle is a torsion angle between the two domains. The HC1 and LC1 bend angles are equivalent to tilting-like variations of one domain with respect to the other. The HC2 and LC2 bend angles describe twisting-like variations of one domain to the other.

To describe the VH-VL orientation six measures are used, a distance and five angles. These are defined in the coordinate system as follows:

- the length of C, dc,
- the torsion angle, HL, from H1 to L1 measured about C,
- the bend angle, HC1, between H1 and C,
- the bend angle, HC2, between H2 and C,
- the bend angle, LC1 between L1 and C, and
- the bend angle, LC2, between L2 and C.

The term "VH-VL orientation" is used in accordance with its common meaning in the art as it would be understood by a person skilled in the art (see, e.g., Dunbar et al., Prot. Eng. Des. Sel. 26 (2013) 611-620; and Bujotzek, A., et al., Proteins, Struct. Funct. Bioinf., 83 (2015) 681-695). It denotes how the VH and VL domains orientate with respect to one another.

Thus the VH-VL orientation is defined by

- the length of C, dc,
- the torsion angle, HL, from H1 to L1 measured about C,
- the bend angle, HC1, between H1 and C,
- the bend angle, HC2, between H2 and C,
- the bend angle, LC1 between L1 and C, and
- the bend angle, LC2, between L2 and C,

wherein reference frame planes are registered by i) aligning the Cα coordinates corresponding to the eight positions H36, H37, H38, H39, H89, H90, H91 and H92 of VH and fitting a plane through them and ii) aligning the Cα coordinates corresponding to the eight positions L35, L36, L37, L38, L85, L86, L87 and L88 of VL and fitting a plane through them, iii) placing a placed on each plane, whereby each structure has equivalent mesh points and equivalent VH-VL mesh point pairs, and iv) measuring the Euclidean distance for each pair of mesh points in each structure, whereby the vector C joins the pair of points with the minimum variance in their separation distance,

wherein H1 is the vector running parallel to the first principal component of the VH plane, H2 is the vector running parallel to the second principal component of the VH plane, L1 is the vector running parallel to the first principal component of the VL plane, L2 is the vector running parallel to the second principal component of the VL plane, the HL angle is the torsion angle between the two domains, the HC1 and LC1 are the bend angles equivalent to tilting-like variations of one domain with respect to the other, and the HC2 and LC2 bend angles are equivalent to the twisting-like variations of one domain to the other.

The positions are determined according to the Chothia index.

The vector C was chosen to have the most conserved length over the non-redundant set of structures. The distance, dc, is this length. It has a mean value of 16.2 Å and a standard deviation of only 0.3 Å.

The following Table lists the top 10 positions and residues identified by the random forest algorithm as being important in determining each of the angular measures of VH-VL orientation.

**Table:** X represents the variable L36V/L38E/L42H/L43L/L44F/L45T/ L46G/L49G/L95H

| Angle | top 10 important input variables |
|---|---|
| HL | L87F L42G/L43T L44V H61D L89L H43Q H43N/H44K H62K/H89V L55H L53R |
| HC1 | X L56P L41D L89A L97V L94N L34H L34N L96W L100A |
| HC2 | H62S H62K/H89V H43K H50W H46K/H62D H35S H61Q H43Q H33W H58T |
| LC1 | L91W L89A X L97V L94N L50G H43Q L56P H62Sb L55A |
| LC2 | L50Y L42G/L43T L44V L42Q L55H H99Y L93T L94L L53R L85T |

(for more detailed information see Dunbar, J., et al., Protein Eng. Des. Sel., 26 (2013) 611-620 and Bujotzek, A., et al., Prot. Struct. Funct. Bioinf. 83 (2015) 681-695).

Thereby a fast sequence-based predictor that predicts VH-VL-interdomain orientation is provided. The VH-VL-orientation is described in terms of the six absolute ABangle parameters to precisely separate the different degrees of freedom of VH-VL-orientation. The deviation between two sequences/structures is shown by the average root-mean-square deviation (RMSD) of the carbonyl atoms of the amino acid backbone.

In one embodiment of all aspects as reported herein the VH/VL orientation is determined as follows:

- generating from the multitude of variant antibody sequences and for the reference antibody Fv fragments,
- determining the VH-VL-orientation for the reference Fv fragment and for each of the variant antibody Fv fragments of the multitude of variant antibody Fv fragments based on a sequence fingerprint of the antibody Fv fragment,
- identifying/selecting/obtaining/ranking those variant antibody Fv fragments that have the smallest difference in the VH-VL-orientation compared to the reference antibody's VH-VL-orientation.

In one embodiment the method comprising the following step:

- identifying/selecting/obtaining/ranking those variant antibody Fv fragments that have the highest (structural) similarity in the VH-VL-interdomain angle compared to the reference antibody's VH-VL-interdomain angle.

In one embodiment a VH-VL-interface residue is an amino acid residue whose side chain atoms have neighboring atoms of the opposite chain with a distance of less than or equal to 4 Å (in at least 90 % of all superimposed Fv structures).

In one embodiment the set of VH-VL-interface residues comprises residues 210, 296, 610, 612, 733 (numbering according to Wolfguy index).

In one embodiment the set of VH-VL-interface residues comprises residues 199, 202, 204, 210, 212, 251, 292, 294, 295, 329, 351, 352, 354, 395, 396, 397, 398, 399, 401, 403, 597, 599, 602, 604, 609, 610, 612, 615, 651, 698, 733, 751, 753, 796, 797, 798 (numbering according to Wolfguy index).

In one embodiment the set of VH-VL-interface residues comprises residues 197, 199, 208, 209, 211, 251, 289, 290, 292, 295, 296, 327, 355, 599, 602, 604, 607, 608, 609, 610, 611, 612, 615, 651, 696, 698, 699, 731, 733, 751, 753, 755, 796, 797, 798, 799, 803 (numbering according to Wolfguy index).

In one embodiment the set of VH-VL-interface residues comprises residues 197, 199, 202, 204, 208, 209, 210, 211, 212, 251, 292, 294, 295, 296, 327, 329, 351, 352, 354, 355, 395, 396, 397, 398, 399, 401, 403, 597, 599, 602, 604, 607, 608, 609, 610, 611, 612, 615, 651, 696, 698, 699, 731, 733, 751, 753, 755, 796, 796, 797, 798, 799, 801, 803 (numbering according to Wolfguy index).

In one embodiment the set of VH-VL-interface residues comprises residues 199, 202, 204, 210, 212, 251, 292, 294, 295, 329, 351, 352, 354, 395, 396, 397, 398, 399, 401, 403, 597, 599, 602, 604, 609, 610, 612, 615, 651, 698, 733, 751, 753, 796, 797, 798, 801 (numbering according to Wolfguy index).

In one embodiment the set of VH-VL-interface residues comprises residues 197, 199, 202, 204, 208, 209, 210, 211, 212, 251, 292, 294, 295, 296, 327, 329, 351, 352, 354, 355, 395, 396, 397, 398, 399, 401, 403, 597, 599, 602, 604, 607, 608, 609, 610, 611, 612, 615, 651, 696, 698, 699, 731, 733, 751, 753, 755, 796, 797, 798, 799, 801, 803 (numbering according to Wolfguy index).

In one embodiment the identifying/selecting/obtaining/ranking is based on the top 80 % variant antibody Fv fragments regarding VH-VL-orientation.

In one embodiment the identifying/selecting/obtaining/ranking is of the top 20 % variant antibody Fv fragments regarding VH-VL-orientation.

In one embodiment the VH-VL-orientation is determined by calculating the six ABangle VH-VL-orientation parameters.

In one embodiment the VH-VL-orientation is determined by calculating the ABangle VH-VL-orientation parameters using a random forest method.

In one embodiment the VH-VL-orientation is determined by calculating the ABangle VH-VL-orientation parameters using one random forest method for each ABangle.

In one embodiment the VH-VL-orientation is determined by calculating the habitual torsion angle, the four bend angles (two per variable domain), and the length of the pivot axis of VH and VL (HL, HC1, LC1, HC2, LC2, dc) using a random forest model.

In one embodiment the random forest model is trained only with complex antibody structure data.

In one embodiment the highest structural similarity is the lowest average root-mean-square deviation (RMSD). In one embodiment the RMSD is the RMSD determined for all Calpha atoms (or carbonyl atoms) of the amino acid residues of the non-human or parent antibody to the corresponding Calpha atoms of the variant antibody.

In one embodiment a model assembled from template structures aligned on either consensus VH or VL framework, followed by VH-VL reorientation on a consensus Fv framework is used for determining the VH-VL-orientation.

In one embodiment a model aligned on the β-sheet core of the complete Fv (VH and VL simultaneously) is used for determining the VH-VL-orientation.

In one embodiment a model in which the antibody Fv fragment is reoriented on a consensus Fv framework is used for determining the VH-VL-orientation.

In one embodiment a model using template structures aligned onto a common consensus Fv framework and VH-VL orientation not being adjusted in any form is used for determining the VH-VL-orientation.

In one embodiment a model assembled from template structures aligned on either consensus VH or VL framework, followed by VH-VL reorientation on a VH-VL orientation template structure chosen based on similarity is used to determine the VH-VL-orientation.

Once the ABangle values for the reference antibody and its variants have been determined, one can rank the variants according to their similarity with regard to the VH-VL orientation of the reference antibody. In order to compare similarity in ABangle space, we define a set of ABangle parameters as the tuple $\theta$ := (*HL, HC*1, *LC*1, *HC*2, *LC*2, *dc*) := ($\vartheta_1$, $\vartheta_2$, $\vartheta_3$, $\vartheta_4$, $\vartheta_5$, $\vartheta_6$). The Euclidean distance between two sets of ABangle parameters is then

$$dist_{ABangle}(\theta_a, \theta_b) = \sqrt{\sum_{i=1}^{6}(\vartheta_{i_a} - \vartheta_{i_b})^2}.$$

As $dist_{ABangle}$ mingles angular (HL, HC1, LC1, HC2, LC2) with linear (dc) distance measures, they cannot be interpreted as factual distance in angular space, but serve only as an abstract distance measure.

## Filtering

Depending on the application of SCaRAb, the mutation tuples can be screened for certain sequence features (e.g., removal of a potential glycosylation site that is present in the reference antibody) or liabilities (e.g., introduction of a new free cysteine residue that is not present in the reference antibody) and filters can be applied accordingly.

## EXAMPLE A

*Isolation and properties of B-cell cloning binders (BCC binders, Binding ELISA)*

At day 6 after the 3rd immunization 15 ml blood were harvested from the immunized rabbit R176 and 108.6 × 10E6 PBMC were isolated. For the NGS of VHs 4.2 × 10E6 PBMC were resuspended in RLT Buffer, whereas the remaining PBMCs were further processed (macrophage depletion, enrichment on antigen) for isolation of antigen specific B-cell clones by B-cell cloning process (see e.g. Seeber, S., et al, PLoS One, 9 (2014) e86184).

In total, during this B-cell cloning process 504 single B-cells of bleed 1 of animal R176 were deposited and cultivated after macrophage/KLH-binder depletion and 840 single B-cells were deposited and cultivated after enrichment on the antigen (LRP8, Low-density lipoprotein receptor-related protein 8, UniProtKB - Q14114).

After macrophage depletion, the primary screening identified 279 IgG-secreting B-cell clones. Of these clones 4 supernatants bound to the antigen. After antigen enrichment 341 IgG-positive supernatants could be identified. Among them 55 B-cell supernatants bound to the antigen (see following Table).

**Table:** Isolation and properties of binders identified by B-cell cloning

| Cell Treatment | wells total | IgG+ wells | rbIgG [% total wells] | antigen [n] | antigen [% total wells] | antigen [% IgG+ wells] |
|---|---|---|---|---|---|---|
| Macrophage depletion (SA_KLH biot. neg.) | 504 | 279 | 55 | 4 | 0.8 | 1.4 |
| antigen-specific enrichment SA_ KLH biot. neg., SA antigen pos. | 840 | 341 | 41 | 55 | 6.5 | 16.1 |

*Identification in NGS data pool variants of reference antibody (BCC binder variant)*

Totally 4 binders identified by B-cell cloning were chosen for identification in NGS repertoires of VHs variants; all clones were isolated by B-cell cloning after antigen specific enrichment, all exhibited specificity for the antigen ($EC_{50}$ below 20 ng/ml) and two of them revealed cross-reactivity to the murine antigen ($EC_{50}$ below 20 ng/ml) (see following Table).

**Table:** Properties of B-cell clones selected for NGS variants analysis

| clone | binding specificity (ELISA) | $EC_{50}$ hu-antigen [ng/ml] | $EC_{50}$ mu-antigen [ng/ml] |
|---|---|---|---|
| BCC.755 | hu | <20 | >2000 |
| BCC.763 | hu; mu | <20 | <20 |
| BCC.770 | hu | <20 | >2000 |
| BCC.776 | hu; mu | <20 | <20 |

NGS repertoire from PBMCs and from antigen-enriched B-cells was analyzed for identification of VHs variants with ≤ 6 amino acid replacements in the entire VH (FR1 to FR4, mutations in frameworks and HVR/CDRs are allowed) compared to the VH of the reference B-cell binders. Totally 441 diverse VH variants could be identified, with different distribution for the 4 binders and for the NGS sample delivering the variants, as shown in the following Table.

**Table**: Related VHs, identified in NGS samples

| clone | number of related VHs, identified in NGS samples, with ≤6 AA replacements in VH (FR1 to FR4) | from |
|---|---|---|
| BCC.755 | 322 | antigen enriched B-cells |
| BCC.763 | 7 | PBMC |
| BCC.770 | 75 | antigen enriched B-cells |
| BCC.776 | 37 | PBMC and antigen enriched B-cells |

*Structure-guided Clustering and Ranking of Antibody Variants (SCaRAb)*

As outlined above a clustering of the identified variants had been performed. The results are presented in the following tables. Note that variants representing cluster medoids, i.e., the variant in the cluster whose average dist _ABangle to all other variants in the cluster is minimal, have been highlighted with a grey background. The generally known k-medoids-based-clustering has been extended in the method as reported herein by using a distance-related function. These variants can be interpreted as the representative or exemplar of a given cluster of variants.

VH variants of antibody 755 (40 clusters)

| VH variant name | number of mutations | mutation tuple | risk score | ABangle distance | free cysteine | Cluster index |
|---|---|---|---|---|---|---|
| **1117:26048:17440 1:N:0:4** | **3** | **S305P,S307T,A394G** | **-13.80** | **0.00** | **N** | **0** |
| 2107:3548:16900 1:N:0:3 | 3 | S307T,F329C,A394G | -18.40 | 0.19 | Y | 0 |
| 2114:20056:25187 1:N:0:3 | 3 | S307T,C330V,A394G | -20.40 | 0.00 | Y | 0 |
| 2116:13408:1588 1:N:0:3 | 3 | F302C,S307T,A394G | -13.20 | 0.00 | Y | 0 |
| 1104:8159:14989 1:N:0:4 | 3 | S307T,C330R,A394G | -28.40 | 0.00 | Y | 0 |
| 1107:18832:6130 1:N:0:4 | 3 | S307T,C330W,A394G | -24.40 | 0.00 | Y | 0 |

| VH variant name | number of mutations | mutation tuple | risk score | ABangle distance | free cysteine | Cluster index |
|---|---|---|---|---|---|---|
| 1110:14978:9932 1:N:0:4 | 3 | S307T,C330Y,A394G | -24.40 | 0.00 | Y | 0 |
| 1111:19785:1275 1:N:0:4 | 3 | S307T,S319R,A394C | -8.70 | 0.00 | Y | 0 |
| 2112:23323:1570 1:N:0:4 | 3 | Y293C,S307T,A394G | -22.80 | 0.00 | Y | 0 |
| 2113:10625:13290 1:N:0:4 | 3 | W296C,S307T,A394G | -12.40 | 0.28 | Y | 0 |
| 2105:4626:5248 1:N:0:1 | 3 | S307T,S319R,A394G | -8.70 | 0.00 | N | 0 |
| 2111:25558:19928 1:N:0:1 | 3 | D314N,S319T,A394G | -6.20 | 0.00 | N | 0 |
| 1113:18172:23110 1:N:0:2 | 3 | S307T,A331S,A394G | -11.30 | 0.00 | N | 0 |
| 2103:22100:5261 1:N:0:2 | 3 | S307T,A318V,A394G | -10.00 | 0.00 | N | 0 |
| 1101:9654:14883 1:N:0:3 | 3 | S307T,S308T,A394G | -8.40 | 0.00 | N | 0 |
| 1103:11925:7788 1:N:0:3 | 3 | S307T,F329V,A394G | -15.90 | 0.19 | N | 0 |
| 1103:18092:23861 1:N:0:3 | 3 | S307T,T311N,A394G | -10.20 | 0.00 | N | 0 |
| 1104:18405:16109 1:N:0:3 | 3 | F302L,S307T,A394G | -10.80 | 0.00 | N | 0 |
| 1106:26206:7674 1:N:0:3 | 3 | I304N,S307T,A394G | -9.90 | 0.00 | N | 0 |
| 1106:22579:20023 1:N:0:3 | 3 | S307T,A318D,A394G | -11.60 | 0.00 | N | 0 |
| 1107:23439:5644 1:N:0:3 | 3 | S307T,L315Q,A394G | -14.40 | 0.00 | N | 0 |
| 1107:18066:9061 1:N:0:3 | 3 | S307T,E323A,A394G | -9.00 | 0.00 | N | 0 |
| 1109:10987:15579 1:N:0:3 | 3 | F302V,S307T,A394G | -12.00 | 0.00 | N | 0 |
| 1111:4272:5585 1:N:0:3 | 3 | S307T,T325P,A394G | -10.20 | 0.00 | N | 0 |
| 1111:22960:14878 1:N:0:3 | 3 | S307T,D324A,A394G | -15.60 | 0.00 | N | 0 |
| 1115:9261:11684 1:N:0:3 | 3 | S307T,M317L,A394G | -9.15 | 0.00 | N | 0 |
| 1117:8419:21526 1:N:0:3 | 3 | S307T,E323D,A394G | -8.50 | 0.00 | N | 0 |
| 2101:21589:9999 1:N:0:3 | 3 | F302S,S307T,A394G | -13.20 | 0.00 | N | 0 |

| VH variant name | number of mutations | mutation tuple | risk score | ABangle distance | free cysteine | Cluster index |
|---|---|---|---|---|---|---|
| 2104:28355:8492 1:N:0:3 | 3 | S307T,T327A,A394G | -9.60 | 0.36 | N | 0 |
| 2104:10919:17808 1:N:0:3 | 3 | S307T,L320I,A394G | -9.10 | 0.00 | N | 0 |
| 2105:5190:16629 1:N:0:3 | 3 | S307T,T321A,A394G | -8.80 | 0.00 | N | 0 |
| 2106:3273:11170 1:N:0:3 | 3 | S307T,L320P,A394G | -15.40 | 0.00 | N | 0 |
| 2106:2261:14587 1:N:0:3 | 3 | S307T,S319G,A394G | -8.60 | 0.00 | N | 0 |
| 2108:8221:22107 1:N:0:3 | 3 | S307T,R332G,A394G | -19.60 | 0.00 | N | 0 |
| 2110:7760:22092 1:N:0:3 | 3 | S307T,T327S,A394G | -9.00 | 0.36 | N | 0 |
| 2111:22747:14477 1:N:0:3 | 3 | S307T,A326D,A394G | -17.20 | 0.00 | N | 0 |
| 2117:13720:12053 1:N:0:3 | 3 | S307T,A326S,A394G | -10.60 | 0.00 | N | 0 |
| 1102:19406:3778 1:N:0:4 | 3 | S307T,D324E,A394G | -9.30 | 0.00 | N | 0 |
| 1102:25932:14332 1:N:0:4 | 3 | S305T,S307T,A394G | -10.20 | 0.00 | N | 0 |
| 1105:10046:21225 1:N:0:4 | 3 | S307T,T325A,A394G | -9.60 | 0.00 | N | 0 |
| 1108:3516:11106 1:N:0:4 | 3 | R301L,S307T,A394G | -8.80 | 0.00 | N | 0 |
| 1110:29049:9990 1:N:0:4 | 3 | S307T,D324N,A394G | -10.20 | 0.00 | N | 0 |
| 1111:15125:8276 1:N:0:4 | 3 | S307T,S309T,A394G | -9.60 | 0.00 | N | 0 |
| 1112:15902:11470 1:N:0:4 | 3 | S307T,D314E,A394G | -8.45 | 0.00 | N | 0 |
| 2101:14383:9526 1:N:0:4 | 3 | S307T,T325K,A394G | -10.20 | 0.00 | N | 0 |
| 2101:22864:22719 1:N:0:4 | 3 | S307T,S319T,A394G | -8.50 | 0.00 | N | 0 |
| 2102:7971:7987 1:N:0:4 | 3 | S307T,V313L,A394G | -10.90 | 0.00 | N | 0 |
| 2102:11457:11982 1:N:0:4 | 3 | S307T,D314V,A394G | -8.90 | 0.00 | N | 0 |
| 2103:27670:6132 1:N:0:4 | 3 | I304T,S307T,A394G | -9.30 | 0.00 | N | 0 |
| 2103:5454:17222 1:N:0:4 | 3 | S307T,L315R,A394G | -14.40 | 0.00 | N | 0 |

| VH variant name | number of mutations | mutation tuple | risk score | ABangle distance | free cysteine | Cluster index |
|---|---|---|---|---|---|---|
| 2105:17113:5256 1:N:0:4 | 3 | S307T,Y328H,A394G | -9.90 | 0.00 | N | 0 |
| 2108:19861:3706 1:N:0:4 | 3 | S307T,D314G,A394G | -8.70 | 0.00 | N | 0 |
| 2108:27997:9341 1:N:0:4 | 3 | F302I,S307T,A394G | -10.80 | 0.00 | N | 0 |
| 2110:8959:12221 1:N:0:4 | 3 | R301G,S307T,A394G | -8.80 | 0.00 | N | 0 |
| 2110:8552:14278 1:N:0:4 | 3 | T303S,S307T,A394G | -10.10 | 0.00 | N | 0 |
| 2113:10355:19267 1:N:0:4 | 3 | S307T,D314K,A394G | -8.70 | 0.00 | N | 0 |
| 2116:3784:9465 1:N:0:4 | 3 | S307T,R332S,A394G | -16.80 | 0.00 | N | 0 |
| 2105:19779:1879 1:N:0:2 | 3 | T291K,S307T,A394G | -19.50 | 0.00 | N | 0 |
| 1101:6229:8183 1:N:0:3 | 3 | K298T,S307T,A394G | -15.60 | 0.00 | N | 0 |
| 1104:13381:24822 1:N:0:3 | 3 | W296G,S307T,A394G | -12.40 | 0.28 | N | 0 |
| 1105:13132:24432 1:N:0:3 | 3 | T290N,S307T,A394G | -15.20 | 0.15 | N | 0 |
| 1106:8967:21515 1:N:0:3 | 3 | Y293D,S307T,A394G | -26.40 | 0.00 | N | 0 |
| 1107:21991:4193 1:N:0:3 | 3 | N295T,S307T,A394G | -13.00 | 0.13 | N | 0 |
| 1110:20533:2171 1:N:0:3 | 3 | A297S,S307T,A394G | -9.60 | 0.00 | N | 0 |
| 1111:6033:18835 1:N:0:3 | 3 | Y293F,S307T,A394G | -9.60 | 0.00 | N | 0 |
| 1112:4770:17947 1:N:0:3 | 3 | K298I,S307T,A394G | -20.40 | 0.00 | N | 0 |
| 1118:25241:10586 1:N:0:3 | 3 | T291N,S307T,A394G | -15.80 | 0.00 | N | 0 |
| 1118:15224:20798 1:N:0:3 | 3 | K298N,S307T,A394G | -13.20 | 0.00 | N | 0 |
| 2102:20616:18534 1:N:0:3 | 3 | T291P,S307T,A394G | -19.50 | 0.00 | N | 0 |
| 2105:24474:8007 1:N:0:3 | 3 | A297D,S307T,A394G | -13.20 | 0.00 | N | 0 |
| 2109:12894:5370 1:N:0:3 | 3 | T291S,S307T,A394G | -12.10 | 0.00 | N | 0 |
| 2113:16261:20702 1:N:0:3 | 3 | A297E,S307T,A394G | -12.00 | 0.00 | N | 0 |

| VH variant name | number of mutations | mutation tuple | risk score | ABangle distance | free cysteine | Cluster index |
|---|---|---|---|---|---|---|
| 2117:19754:4077 1:N:0:3 | 3 | Y292S,S307T,A394G | -16.80 | 0.21 | N | 0 |
| 1101:21506:14114 1:N:0:4 | 3 | S288R,S307T,A394G | -20.40 | 0.00 | N | 0 |
| 1105:16900:24181 1:N:0:4 | 3 | G299S,S307T,A394G | -9.40 | 0.00 | N | 0 |
| 1107:16371:23843 1:N:0:4 | 3 | A297G,S307T,A394G | -10.80 | 0.00 | N | 0 |
| 1109:15849:20837 1:N:0:4 | 3 | N295Y,S307T,A394G | -17.60 | 0.00 | N | 0 |
| 1112:15276:22867 1:N:0:4 | 3 | W296R,S307T,A394G | -13.40 | 0.30 | N | 0 |
| 1117:27611:5863 1:N:0:4 | 3 | N295K,S307T,A394G | -13.00 | 0.00 | N | 0 |
| 2105:22497:25166 1:N:0:4 | 3 | A294V,S307T,A394G | -13.00 | 0.34 | N | 0 |
| 2108:13105:4949 1:N:0:4 | 3 | W296L,S307T,A394G | -12.40 | 0.28 | N | 0 |
| 2110:13214:4092 1:N:0:4 | 3 | K298Q,S307T,A394G | -10.80 | 0.00 | N | 0 |
| 2112:3904:12465 1:N:0:4 | 3 | Y292T,S307T,A394G | -16.80 | 1.13 | N | 0 |
| 2114:8546:13514 1:N:0:4 | 3 | K298R,S307T,A394G | -9.60 | 0.00 | N | 0 |
| **2113:22155:9432 1:N:0:4** | **5** | **N155S,S156V,D196Y,R197G,G199T** | **-62.40** | **0.25** | **N** | **1** |
| 2105:20717:17101 1:N:0:4 | 5 | V104L,N155S,D196Y,R197V,G199S | -45.20 | 0.07 | N | 1 |
| 2117:26776:18522 1:N:0:3 | 5 | I152F,D153S,N155S,D196Y,R197A | -37.70 | 0.17 | N | 1 |
| **2111:21535:5980 1:N:0:3** | **1** | **P117L** | **-0.50** | **0.00** | **N** | **2** |
| 1107:22265:17831 1:N:0:4 | 1 | C122R | -20.00 | 0.00 | Y | 2 |
| 2109:15764:10046 1:N:0:3 | 1 | Q102P | -3.30 | 0.00 | N | 2 |
| 2109:11666:22695 1:N:0:3 | 1 | V104L | -0.50 | 0.00 | N | 2 |
| 2115:18433:20332 1:N:0:3 | 1 | T123I | 0.00 | 0.00 | N | 2 |
| 1110:4244:13646 1:N:0:4 | 1 | V112D | -2.00 | 0.00 | N | 2 |
| 2114:5742:18170 1:N:0:4 | 1 | L118M | -0.10 | 0.00 | N | 2 |

| VH variant name | number of mutations | mutation tuple | risk score | ABangle distance | free cysteine | Cluster index |
|---|---|---|---|---|---|---|
| **2119:28171:16213 1:N:0:4** | **5** | **S307T,S355C,A394G, F397L,G402C** | **-35.90** | **0.32** | **Y** | **3** |
| 1103:11280:23651 1:N:0:4 | 5 | S307T,S352C,A394G, F397C,N398D | -34.40 | 0.38 | Y | 3 |
| **1117:21523:9244 1:N:0:4** | **3** | **T116G,P117T,I152F** | **-5.90** | **0.00** | **N** | **4** |
| **1112:17135:11276 1:N:0:3** | **4** | **E105A,T123P,S307T, A394G** | **-9.00** | **0.00** | **N** | **5** |
| 1117:15314:17723 1:N:0:4 | 4 | V104L,T113M,S307T, A394G | -8.90 | 0.00 | N | 5 |
| 2103:5849:10044 1:N:0:4 | 4 | V104L,P117T,S307T, A394G | -9.20 | 0.00 | N | 5 |
| 2105:28283:12169 1:N:0:4 | 4 | R110G,N155K,S307T, A394G | -13.00 | 0.00 | N | 5 |
| 2113:9804:21625 1:N:0:4 | 4 | V104L,N155S,S307T, A394G | -10.80 | 0.00 | N | 5 |
| **2101:5682:22204 1:N:0:4** | **2** | **I213L,T291P** | **-11.55** | **0.00** | **N** | **6** |
| **1118:21273:1232 1:N:0:4** | **4** | **S307T,T312P,A318X, A394G** | **-12.40** | **0.00** | **N** | **7** |
| 1101:25753:6416 1:N:0:2 | 4 | S307T,C330W,A331S, A394G | -27.30 | 0.00 | Y | 7 |
| 1101:15616:11852 1:N:0:4 | 4 | S307T,L320M,C330W ,A394G | -25.10 | 0.00 | Y | 7 |
| 2108:8173:23018 1:N:0:4 | 4 | S307T,A326D,C330R, A394G | -37.20 | 0.00 | Y | 7 |
| 2103:21863:7444 1:N:0:4 | 4 | S307T,C330G,Y351D, A394G | -48.40 | 0.27 | Y | 7 |
| 2117:7027:12367 1:N:0:4 | 4 | S307T,C330G,N354Y, A394G | -42.40 | 0.08 | Y | 7 |
| 1109:12708:24556 1:N:0:2 | 4 | S305T,S307T,T321P, A394G | -10.80 | 0.00 | N | 7 |
| 2105:10840:18512 1:N:0:3 | 4 | S305A,S307T,A318D, A394G | -13.40 | 0.00 | N | 7 |
| 2119:17571:8893 1:N:0:3 | 4 | S307T,T322N,F329L, A394G | -14.00 | 0.19 | N | 7 |
| 1101:27737:14174 1:N:0:4 | 4 | S307T,A318G,A326D, A394G | -18.80 | 0.00 | N | 7 |
| 1103:15990:16033 1:N:0:4 | 4 | S307T,D314V,R332G, A394G | -20.10 | 0.00 | N | 7 |
| 1103:15010:16228 1:N:0:4 | 4 | S305T,S307T,S319R, A394G | -10.50 | 0.00 | N | 7 |
| 1109:28899:17376 1:N:0:4 | 4 | S305P,S307T,S319R, A394G | -14.10 | 0.00 | N | 7 |

| VH variant name | number of mutations | mutation tuple | risk score | ABangle distance | free cysteine | Cluster index |
|---|---|---|---|---|---|---|
| 1114:14635:23670 1:N:0:4 | 4 | S307T,S319G,T325P, A394G | -10.40 | 0.00 | N | 7 |
| 2108:14119:4451 1:N:0:4 | 4 | S307T,D314E,T321A, A394G | -8.85 | 0.00 | N | 7 |
| 1110:18648:2202 1:N:0:2 | 4 | Y293S,F302V,S307T, A394G | -26.40 | 0.00 | N | 7 |
| 2105:16971:5020 1:N:0:2 | 4 | G299S,S307T,L315P, A394G | -16.90 | 0.00 | N | 7 |
| 2114:12873:15745 1:N:0:2 | 4 | N295K,S307T,D314Y, A394G | -13.50 | 0.00 | N | 7 |
| 1102:21561:2675 1:N:0:3 | 4 | T291H,S307T,D314G, A394G | -23.50 | 0.00 | N | 7 |
| 1103:15418:10210 1:N:0:3 | 4 | A294G,F302Y,S307T, A394G | -13.40 | 0.36 | N | 7 |
| 1116:13513:17404 1:N:0:3 | 4 | Y293D,F302Y,S307T, A394G | -26.80 | 0.00 | N | 7 |
| 1117:14122:24446 1:N:0:3 | 4 | S288R,S305P,S307T, A394G | -25.80 | 0.00 | N | 7 |
| 1118:12162:22228 1:N:0:3 | 4 | A297E,F302I,S307T,A394G | -14.40 | 0.00 | N | 7 |
| 2101:28389:16276 1:N:0:3 | 4 | K298N,F302L,S307T, A394G | -15.60 | 0.00 | N | 7 |
| 2106:26242:21852 1:N:0:3 | 4 | K298N,R301L,S307T, A394G | -13.60 | 0.00 | N | 7 |
| 2113:6396:6995 1:N:0:3 | 4 | N295T,F302I,S307T,A394G | -15.40 | 0.13 | N | 7 |
| 1113:7787:14034 1:N:0:4 | 4 | K298E,F302I,S307T,A394G | -13.20 | 0.00 | N | 7 |
| 2108:16555:20565 1:N:0:4 | 4 | S288G,S307T,D314E, A394G | -16.45 | 0.00 | N | 7 |
| 2116:26684:6924 1:N:0:4 | 4 | K298N,F302I,S307T, A394G | -15.60 | 0.00 | N | 7 |
| 2114:14700:23845 1:N:0:5 | 4 | W296G,F302T,S307T, A394G | -17.20 | 0.28 | N | 7 |
| 1106:4127:15400 1:N:0:3 | 4 | N295H,K298R,S307I, A394G | -19.10 | 0.00 | N | 7 |
| 1106:16406:15444 1:N:0:3 | 4 | N295K,A297S,S307T, A394G | -14.20 | 0.00 | N | 7 |
| 1113:12273:21196 1:N:0:3 | 4 | S288R,T290N,S307T, A394G | -27.20 | 0.15 | N | 7 |
| 1106:9726:22782 1:N:0:4 | 4 | A294S,G299A,S307T, A394G | -11.70 | 0.35 | N | 7 |
| 2107:10746:22307 1:N:0:4 | 4 | T291P,N295T,S307T, A394G | -24.10 | 0.13 | N | 7 |

| VH variant name | number of mutations | mutation tuple | risk score | ABangle distance | free cysteine | Cluster index |
|---|---|---|---|---|---|---|
| 2101:25197:3708 1:N:0:3 | 4 | S307T,S319R,N354K, A394G | -15.70 | 0.03 | N | 7 |
| 2105:4942:7894 1:N:0:3 | 4 | S307T,E323A,Y351L, A394G | -21.00 | 0.17 | N | 7 |
| 2112:4654:6406 1:N:0:3 | 4 | S307T,E323G,N354I, A394G | -26.70 | 0.08 | N | 7 |
| 1101:27729:14154 1:N:0:4 | 4 | S307T,T322P,T353P, A394G | -21.30 | 0.00 | N | 7 |
| 1109:26370:4240 1:N:0:5 | 4 | S307T,L320R,S355R, A394V | -24.50 | 0.22 | N | 7 |
| 2117:9077:16439 1:N:0:5 | 4 | S307T,T325L,N354D, A394G | -13.70 | 0.17 | N | 7 |
| **1117:9244:19295 1:N:0:4** | **5** | **Q102R,S307T,S319R, T327S,A394G** | **-10.40** | **0.36** | **N** | **8** |
| 1107:23167:3797 1:N:0:3 | 5 | P117L,Y292S,S307T, T311P,A394G | -20.00 | 0.21 | N | 8 |
| **2112:28984:9687 1:N:0:4** | **1** | **L399F** | **-6.00** | **0.02** | **N** | **9** |
| 1113:10964:14412 1:N:0:3 | 1 | P403Q | -1.50 | 0.70 | N | 9 |
| 1118:15879:20254 1:N:0:3 | 1 | A394T | -6.00 | 0.00 | N | 9 |
| **1116:8054:13480 1:N:0:4** | **5** | **V104L,T116G,P117S, S307T,A394G** | **-9.60** | **0.00** | **N** | **10** |
| 2115:26912:16126 1:N:0:4 | 5 | T116G,P117T,T123I,S307T,A394G | -9.10 | 0.00 | N | 10 |
| **2110:7379:5032 1:N:0:4** | **4** | **R110H,S307T,A318D, A394G** | **-12.00** | **0.00** | **N** | **11** |
| 1119:1879:13689 1:N:0:4 | 4 | E105V,S307T,C330Y, A394G | -25.20 | 0.00 | Y | 11 |
| 1104:18713:2981 1:N:0:3 | 4 | G108R,S307T,T325A, A394G | -12.80 | 0.00 | N | 11 |
| 1114:19267:19071 1:N:0:3 | 4 | S103L,F302Y,S307T, A394G | -8.80 | 0.00 | N | 11 |
| 2110:27515:12485 1:N:0:4 | 4 | G108R,S307T,Y328D, A394G | -26.60 | 0.00 | N | 11 |
| 2115:9097:4698 1:N:0:4 | 4 | V104L,S307T,T321K, A394G | -9.50 | 0.00 | N | 11 |
| 1107:23186:3788 1:N:0:3 | 4 | P117L,K298Q,S307T, A394G | -11.30 | 0.00 | N | 11 |
| 1114:11441:20252 1:N:0:3 | 4 | S103L,T291A,S307T, A394G | -15.80 | 0.00 | N | 11 |
| 2115:11654:10133 1:N:0:3 | 4 | T123I,K298N,S307T, A394G | -13.20 | 0.00 | N | 11 |

| VH variant name | number of mutations | mutation tuple | risk score | ABangle distance | free cysteine | Cluster index |
|---|---|---|---|---|---|---|
| 1114:25898:20164 1:N:0:4 | 4 | S103L,S288R,S307T, A394G | -20.40 | 0.00 | N | 11 |
| **1101:18779:19164 1:N:0:4** | **5** | **Q102P,V104L,S307T, D324E,A394G** | **-13.10** | **0.00** | **N** | **12** |
| 2104:14172:22246 1:N:0:3 | 5 | V104L,N155S,S305A, S307T,A394G | -12.60 | 0.00 | N | 12 |
| **2103:21736:11070 1:N:0:4** | **4** | **C122S,W212G,I213M ,A331Y** | **-36.90** | **0.23** | **Y** | **13** |
| **1111:20770:14582 1:N:0:1** | **2** | **S307T,A394G** | **-8.40** | **0.00** | **N** | **14** |
| 1105:10148:19074 1:N:0:4 | 2 | S307T,A394C | -8.40 | 0.00 | Y | 14 |
| 2116:23124:4613 1:N:0:3 | 2 | A331S,V409G | -7.90 | 0.00 | N | 14 |
| 2118:4192:17694 1:N:0:4 | 2 | A326V,T405S | -4.70 | 0.00 | N | 14 |
| 1101:2409:16339 1:N:0:3 | 2 | S319R,N354I | -17.80 | 0.08 | N | 14 |
| 1108:2650:10812 1:N:0:3 | 2 | S307T,A394V | -8.40 | 0.00 | N | 14 |
| 1113:10966:14429 1:N:0:3 | 2 | Y351D,P403Q | -21.50 | 0.78 | N | 14 |
| 1117:18531:19527 1:N:0:4 | 2 | S307I,A394G | -15.60 | 0.00 | N | 14 |
| **1101:19661:1424 1:N:0:4** | **2** | **L120F,L154P** | **-13.90** | **0.00** | **N** | **15** |
| 1111:4052:19173 1:N:0:4 | 2 | P117H,E208G | -3.20 | 0.09 | N | 15 |
| 1107:7761:7540 1:N:0:3 | 2 | V124A,I152F | -9.20 | 0.00 | N | 15 |
| 2112:22110:23423 1:N:0:4 | 2 | Q102K,N155H | -3.00 | 0.00 | N | 15 |
| **2101:5252:21433 1:N:0:3** | **3** | **T113M,S307T,A394G** | **-8.40** | **0.00** | **N** | **16** |
| 1101:23578:3670 1:N:0:3 | 3 | V124A,S307T,A394G | -12.40 | 0.00 | N | 16 |
| 1102:17479:4575 1:N:0:3 | 3 | V124I,S307T,A394G | -9.07 | 0.00 | N | 16 |
| 1102:17819:7207 1:N:0:3 | 3 | Q102P,S307T,A394G | -11.70 | 0.00 | N | 16 |
| 1103:14565:9892 1:N:0:3 | 3 | T123I,S307T,A394G | -8.40 | 0.00 | N | 16 |
| 1110:5480:9843 1:N:0:3 | 3 | V104L,S307T,A394G | -8.90 | 0.00 | N | 16 |

| VH variant name | number of mutations | mutation tuple | risk score | ABangle distance | free cysteine | Cluster index |
|---|---|---|---|---|---|---|
| 1112:19316:19410 1:N:0:3 | 3 | G108W,S307T,A394G | -11.60 | 0.00 | N | 16 |
| 1114:2899:8564 1:N:0:3 | 3 | S103L,S307T,A394G | -8.40 | 0.00 | N | 16 |
| 1115:19772:7379 1:N:0:3 | 3 | L111P,S307T,A394G | -8.40 | 0.00 | N | 16 |
| 1116:6924:6457 1:N:0:3 | 3 | V112A,S307T,A394G | -9.20 | 0.00 | N | 16 |
| 2102:22485:8517 1:N:0:3 | 3 | E105A,S307T,A394G | -9.00 | 0.00 | N | 16 |
| 2105:16948:18514 1:N:0:3 | 3 | E106K,S307T,A394G | -9.20 | 0.00 | N | 16 |
| 2106:20355:22838 1:N:0:3 | 3 | V104M,S307T,A394G | -8.90 | 0.00 | N | 16 |
| 1102:17194:9315 1:N:0:4 | 3 | G115R,S307T,A394G | -10.80 | 0.00 | N | 16 |
| 1106:14178:11856 1:N:0:4 | 3 | L118P,S307T,A394G | -9.40 | 0.00 | N | 16 |
| 1108:15665:2204 1:N:0:4 | 3 | P114L,S307T,A394G | -8.90 | 0.00 | N | 16 |
| 2108:4840:19320 1:N:0:4 | 3 | G108R,S307T,A394G | -11.60 | 0.00 | N | 16 |
| 1111:28917:12309 1:N:0:1 | 3 | D153N,S307T,A394G | -9.60 | 0.00 | N | 16 |
| 2114:3507:15988 1:N:0:3 | 3 | N155T,S307T,A394G | -12.20 | 0.00 | N | 16 |
| 1108:15399:17590 1:N:0:4 | 3 | N155K,S307T,A394G | -12.20 | 0.00 | N | 16 |
| 2108:28274:15542 1:N:0:4 | 3 | N155S,S307T,A394G | -10.30 | 0.00 | N | 16 |
| **1112:20537:6359 1:N:0:3** | **5** | **Y292S,A294S,A297E,G299R,F302C** | **-21.10** | **0.48** | **Y** | **17** |
| **2104:22776:23560 1:N:0:4** | **4** | **V251A,I252L,Y292S,A294P** | **-23.85** | **0.64** | **N** | **18** |
| **1116:20157:15465 1:N:0:4** | **3** | **G209W,K298Q,I304T** | **-9.70** | **0.66** | **N** | **19** |
| 2104:17031:8630 1:N:0:3 | 3 | V251G,V253F,T303P | -36.10 | 0.26 | N | 19 |
| **2115:16487:5068 1:N:0:4** | **5** | **E208G,F329V,C330W,A394G,N398D** | **-33.80** | **0.18** | **Y** | **20** |
| 1114:13316:5473 1:N:0:4 | 5 | E208G,S307T,S355G,A394G,F397C | -32.20 | 0.30 | Y | 20 |
| **1113:7949:2267 1:N:0:4** | **5** | **S307T,S319I,T322P,A331S,A394G** | **-12.60** | **0.00** | **N** | **21** |

| VH variant name | number of mutations | mutation tuple | risk score | ABangle distance | free cysteine | Cluster index |
|---|---|---|---|---|---|---|
| 1112:23132:24993 1:N:0:3 | 5 | S307T,D324E,C330R,R332S,A394G | -37.70 | 0.00 | Y | 21 |
| 2112:6426:6616 1:N:0:4 | 5 | A294V,K298N,R301C,S307T,A394G | -18.30 | 0.34 | Y | 21 |
| 1102:14155:17596 1:N:0:3 | 5 | S307T,S309P,A318D,L320M,A394G | -15.90 | 0.00 | N | 21 |
| 2111:15176:7641 1:N:0:3 | 5 | S307T,K316I,T327A,Y328S,A394G | -21.60 | 0.36 | N | 21 |
| 1108:5048:19993 1:N:0:4 | 5 | S307T,E323A,A331S,R332S,A394G | -20.30 | 0.00 | N | 21 |
| 2109:22371:4136 1:N:0:4 | 5 | S307T,T325K,F329Y,R332I,A394G | -25.03 | 0.22 | N | 21 |
| 2117:25398:6020 1:N:0:3 | 5 | N295K,F302I,S307T,D314E,A394G | -15.45 | 0.00 | N | 21 |
| 1110:14855:2972 1:N:0:4 | 5 | N295K,W296R,F302S,S307T,A394G | -22.80 | 0.30 | N | 21 |
| 2114:9301:5365 1:N:0:4 | 5 | A297P,K298N,S305T,S307T,A394G | -18.60 | 0.00 | N | 21 |
| 2101:12534:15438 1:N:0:3 | 5 | S305P,S307T,E323G,S352R,A394G | -25.10 | 0.09 | N | 21 |
| 2119:15108:2242 1:N:0:3 | 5 | S307T,L320R,S352I,T353A,A394G | -36.00 | 0.08 | N | 21 |
| **2119:9570:2597 1:N:0:4** | **4** | **D153A,V251G,S307T,A394G** | **-32.20** | **0.26** | **N** | **22** |
| 2105:23971:7940 1:N:0:3 | 4 | G209V,G214V,S307T,A394G | -31.90 | 0.66 | N | 22 |
| 2104:13560:23700 1:N:0:3 | 4 | D153E,R197G,S307T,A394G | -24.60 | 0.05 | N | 22 |
| 2105:19440:21172 1:N:0:3 | 4 | N155I,V251A,S307T,A394G | -25.50 | 0.23 | N | 22 |
| **2102:17483:2532 1:N:0:3** | **3** | **S307T,A394G,A396G** | **-14.40** | **0.16** | **N** | **23** |
| 1111:10546:14497 1:N:0:4 | 3 | S307T,A394G,W401C | -22.40 | 0.00 | Y | 23 |
| 1107:23593:21806 1:N:0:2 | 3 | S307T,A394G,T405A | -9.00 | 0.00 | N | 23 |
| 1113:2847:16642 1:N:0:3 | 3 | S307T,A394G,T408S | -8.50 | 0.00 | N | 23 |
| 2104:4801:14088 1:N:0:3 | 3 | S307T,A394G,L406M | -8.45 | 0.00 | N | 23 |
| 2114:4974:20311 1:N:0:3 | 3 | S307T,A394G,W401R | -25.90 | 0.00 | N | 23 |
| 1115:6244:10522 1:N:0:3 | 3 | S307T,S355R,A394G | -18.90 | 0.22 | N | 23 |

| VH variant name | number of mutations | mutation tuple | risk score | ABangle distance | free cysteine | Cluster index |
|---|---|---|---|---|---|---|
| 1118:5143:5064 1:N:0:3 | 3 | S307T,S352I,A394G | -22.40 | 0.08 | N | 23 |
| 2105:22801:10153 1:N:0:3 | 3 | S307T,A394G,Y395F | -9.57 | 0.11 | N | 23 |
| 1116:17720:9959 1:N:0:4 | 3 | S307T,N354T,A394G | -15.40 | 0.05 | N | 23 |
| 2102:17926:22781 1:N:0:4 | 3 | S307T,S355I,A394G | -22.40 | 0.08 | N | 23 |
| 2103:22390:16654 1:N:0:4 | 3 | S307T,A394G,F397L | -15.40 | 0.32 | N | 23 |
| 2104:8414:6123 1:N:0:4 | 3 | S307T,A394G,F397V | -18.90 | 0.28 | N | 23 |
| 2109:21276:16256 1:N:0:4 | 3 | S307T,A394G,A396D | -20.40 | 0.09 | N | 23 |
| 2116:11277:9861 1:N:0:4 | 3 | S307T,S355G,A394G | -15.40 | 0.08 | N | 23 |
| 2119:29036:9615 1:N:0:4 | 3 | S307T,N354M,A394G | -22.40 | 0.03 | N | 23 |
| 2106:27137:9548 1:N:0:5 | 3 | S307T,A394G,N398D | -9.90 | 0.13 | N | 23 |
| 1116:17833:25032 1:N:0:4 | 3 | S352N,A394G,A396S | -12.50 | 0.14 | N | 23 |
| **2102:28708:12170 1:N:0:3** | **1** | **E323A** | **-0.60** | **0.00** | **N** | **24** |
| 1117:14435:23923 1:N:0:3 | 1 | C330W | -16.00 | 0.00 | Y | 24 |
| 2112:17648:13944 1:N:0:3 | 1 | F329C | -10.00 | 0.19 | Y | 24 |
| 2111:13503:16690 1:N:0:3 | 1 | Y351C | -16.00 | 0.19 | Y | 24 |
| 1102:7658:4808 1:N:0:3 | 1 | R332S | -8.40 | 0.00 | N | 24 |
| 1103:2392:12352 1:N:0:3 | 1 | F329L | -5.00 | 0.19 | N | 24 |
| 1107:28592:14825 1:N:0:3 | 1 | A326S | -2.20 | 0.00 | N | 24 |
| 2104:18926:18007 1:N:0:3 | 1 | F302V | -3.60 | 0.00 | N | 24 |
| 2105:25911:14756 1:N:0:3 | 1 | E323D | -0.10 | 0.00 | N | 24 |
| 2113:14965:7732 1:N:0:3 | 1 | R301Q | -0.10 | 0.00 | N | 24 |
| 1113:6574:13048 1:N:0:4 | 1 | F302L | -2.40 | 0.00 | N | 24 |

| VH variant name | number of mutations | mutation tuple | risk score | ABangle distance | free cysteine | Cluster index |
|---|---|---|---|---|---|---|
| 2107:16580:5388 1:N:0:4 | 1 | Y328H | -1.50 | 0.00 | N | 24 |
| 2115:20613:5973 1:N:0:4 | 1 | S319R | -0.30 | 0.00 | N | 24 |
| 1116:5071:15508 1:N:0:3 | 1 | Y293D | -18.00 | 0.00 | N | 24 |
| 1108:9327:9648 1:N:0:4 | 1 | K298E | -2.40 | 0.00 | N | 24 |
| 2101:26499:9008 1:N:0:4 | 1 | K298N | -4.80 | 0.00 | N | 24 |
| 2111:18866:9328 1:N:0:4 | 1 | A294P | -6.90 | 0.66 | N | 24 |
| 2114:12755:23738 1:N:0:4 | 1 | A297S | -1.20 | 0.00 | N | 24 |
| 1118:21354:17316 1:N:0:3 | 1 | N354T | -7.00 | 0.05 | N | 24 |
| **2114:20534:11604 1:N:0:4** | **5** | **S255G,S307T,A318D,A326G,A394G** | **-23.20** | **0.00** | **N** | **25** |
| 1112:20714:24469 1:N:0:4 | 5 | S255I,W296L,G299C,S307T,A394G | -29.30 | 0.28 | Y | 25 |
| 1101:28349:16016 1:N:0:3 | 5 | E208G,K306I,S307T,S319R,A394G | -11.50 | 0.09 | N | 25 |
| 2105:9076:19470 1:N:0:3 | 5 | I213N,A294D,S307T,K316Q,A394G | -22.10 | 0.34 | N | 25 |
| 2102:26984:5065 1:N:0:4 | 5 | T254H,N295K,S307T,S319R,A394G | -28.50 | 0.00 | N | 25 |
| 1111:26510:7666 1:N:0:3 | 5 | V253A,T291P,K298N,S307T,A394G | -32.30 | 0.00 | N | 25 |
| 1106:10308:2576 1:N:0:1 | 5 | T254S,S307T,A318T,N354D,N398S | -12.80 | 0.16 | N | 25 |
| 1106:18041:23428 1:N:0:4 | 5 | T254S,S307T,A318P,N354D,N398S | -13.60 | 0.16 | N | 25 |
| **1101:21776:24081 1:N:0:4** | **2** | **G299A,S305P** | **-6.40** | **0.00** | **N** | **26** |
| 1101:18416:3880 1:N:0:3 | 2 | D314V,C330G | -20.50 | 0.00 | Y | 26 |
| 1110:23623:3014 1:N:0:4 | 2 | Y293C,E323D | -14.50 | 0.00 | Y | 26 |
| 2105:11893:7688 1:N:0:4 | 2 | D314E,E323A | -0.65 | 0.00 | N | 26 |
| 1102:9925:6735 1:N:0:4 | 2 | G299R,F302I | -4.40 | 0.00 | N | 26 |
| **2116:5079:18887 1:N:0:3** | **5** | **T123P,E208A,S307T,S309L,A394G** | **-15.30** | **0.06** | **N** | **27** |

| VH variant name | number of mutations | mutation tuple | risk score | ABangle distance | free cysteine | Cluster index |
|---|---|---|---|---|---|---|
| 1114:21228:6267 1:N:0:3 | 5 | S103L,V251A,A294S,S307T,A394G | -18.30 | 0.38 | N | 27 |
| **1104:29248:10613 1:N:0:4** | **4** | **I213T,S307T,E323A,A394G** | **-11.70** | **0.00** | **N** | **28** |
| 1102:22954:1504 1:N:0:4 | 4 | V251G,F302C,S307T,A394G | -32.20 | 0.26 | Y | 28 |
| 2109:26695:4723 1:N:0:4 | 4 | D196E,S307T,C330G,A394G | -30.05 | 0.00 | Y | 28 |
| 1106:26861:11322 1:N:0:3 | 4 | G209V,S307T,L320R,A394G | -22.00 | 0.66 | N | 28 |
| 2112:7769:24277 1:N:0:3 | 4 | T254A,S307T,T327P,A394G | -17.80 | 0.36 | N | 28 |
| 1106:14651:9283 1:N:0:4 | 4 | V253F,S307T,D314E,A394G | -20.45 | 0.00 | N | 28 |
| 1106:25426:12341 1:N:0:4 | 4 | V251G,I304S,S307T,A394G | -28.60 | 0.26 | N | 28 |
| 2102:13541:10881 1:N:0:4 | 4 | E208G,S288G,S307T,A394G | -19.20 | 0.09 | N | 28 |
| 2106:17372:24874 1:N:0:4 | 4 | V253G,S307T,L320R,A394G | -34.00 | 0.00 | N | 28 |
| 2109:7533:20614 1:N:0:4 | 4 | E208A,T291K,S307T,A394G | -21.60 | 0.06 | N | 28 |
| 2113:15085:5712 1:N:0:4 | 4 | G199S,K298N,S307T,A394G | -20.00 | 0.14 | N | 28 |
| **2115:16505:5070 1:N:0:4** | **4** | **S305P,K306T,K316T,L320R** | **-11.00** | **0.00** | **N** | **29** |
| 1103:22282:19571 1:N:0:3 | 4 | T322A,E323A,C330S,A331S | -16.10 | 0.00 | Y | 29 |
| 2117:21062:11524 1:N:0:4 | 4 | S288R,T291A,T312K,Y328D | -36.80 | 0.00 | N | 29 |
| **1102:23311:8375 1:N:0:4** | **3** | **E208G,S307T,A394G** | **-11.20** | **0.09** | **N** | **30** |
| 2101:3566:14679 1:N:0:3 | 3 | I213N,S307T,A394G | -12.90 | 0.00 | N | 30 |
| 2101:10234:16082 1:N:0:3 | 3 | E208K,S307T,A394G | -9.10 | 0.55 | N | 30 |
| 2106:29060:12886 1:N:0:3 | 3 | Q204L,S307T,A394G | -15.20 | 0.23 | N | 30 |
| 2106:25797:16513 1:N:0:3 | 3 | V202G,S307T,A394G | -21.40 | 0.22 | N | 30 |
| 2112:16946:9038 1:N:0:3 | 3 | E211V,S307T,A394G | -13.60 | 0.17 | N | 30 |
| 2117:7810:4136 1:N:0:3 | 3 | E208A,S307P,A394G | -15.30 | 0.06 | N | 30 |

| VH variant name | number of mutations | mutation tuple | risk score | ABangle distance | free cysteine | Cluster index |
|---|---|---|---|---|---|---|
| 1102:3683:11930 1:N:0:4 | 3 | I213S,S307T,A394G | -12.00 | 0.00 | N | 30 |
| 1104:29232:10626 1:N:0:4 | 3 | I213T,S307T,A394G | -11.10 | 0.00 | N | 30 |
| 1106:14147:11888 1:N:0:4 | 3 | W201S,S307T,A394G | -28.40 | 0.00 | N | 30 |
| 1109:16238:15426 1:N:0:4 | 3 | R203H,S307T,A394G | -12.40 | 0.00 | N | 30 |
| 1102:19092:21785 1:N:0:3 | 3 | V251G,S307T,A394G | -27.40 | 0.26 | N | 30 |
| 1104:15958:6342 1:N:0:3 | 3 | G199V,S307T,A394G | -25.40 | 0.07 | N | 30 |
| 1109:13663:15593 1:N:0:3 | 3 | T254S,S307T,A394G | -12.20 | 0.00 | N | 30 |
| 2101:22839:4783 1:N:0:3 | 3 | D196E,S307T,A394G | -10.05 | 0.00 | N | 30 |
| 2102:27837:7005 1:N:0:3 | 3 | I252L,S307T,A394G | -9.35 | 0.00 | N | 30 |
| 2107:23079:21136 1:N:0:3 | 3 | R197G,S307T,A394G | -24.00 | 0.05 | N | 30 |
| 1114:14583:6584 1:N:0:4 | 3 | D196N,S307T,A394G | -11.70 | 0.00 | N | 30 |
| 2107:10102:20753 1:N:0:4 | 3 | M198V,S307T,A394G | -11.00 | 0.00 | N | 30 |
| 2118:21108:17365 1:N:0:4 | 3 | V253G,S307T,A394G | -28.40 | 0.00 | N | 30 |
| **2109:8147:21805 1:N:0:3** | **4** | **W296C,S307T,A394G,G404C** | **-17.40** | **0.28** | **Y** | **31** |
| 2118:6440:18144 1:N:0:4 | 4 | S307T,S308A,A394G,S411A | -8.50 | 0.00 | N | 31 |
| 1107:24338:7886 1:N:0:4 | 4 | V251G,S307T,A394G,S410F | -27.80 | 0.26 | N | 31 |
| **2107:29166:13125 1:N:0:3** | **4** | **S307T,Y351C,A394G,A396S** | **-27.40** | **0.17** | **Y** | **32** |
| 1108:5019:18802 1:N:0:3 | 4 | S307T,A394G,L399F,G404R | -18.40 | 0.02 | N | 32 |
| 1110:24949:5805 1:N:0:4 | 4 | S307T,Y328V,A394G,A396D | -29.40 | 0.09 | N | 32 |
| 2103:24484:22076 1:N:0:4 | 4 | S307T,S355R,A394G,W401G | -32.90 | 0.22 | N | 32 |
| **2115:3097:18045 1:N:0:3** | **5** | **T123I,R301P,S307T,A394G,V407A** | **-10.80** | **0.00** | **N** | **33** |
| **1115:13289:24928 1:N:0:4** | **5** | **E208G,N295V,W296G,K298T,G299V** | **-28.00** | **0.29** | **N** | **34** |

| VH variant name | number of mutations | mutation tuple | risk score | ABangle distance | free cysteine | Cluster index |
|---|---|---|---|---|---|---|
| **1110:21663:11508 1:N:0:1** | **5** | **M198L,S255K,S307T,A394G,N398T** | **-19.90** | **0.05** | **N** | **35** |
| **1115:4917:17781 1:N:0:3** | **5** | **S305T,S307T,Y351N,A394G,L399V** | **-29.20** | **0.16** | **N** | **36** |
| **1107:25988:22599 1:N:0:4** | **4** | **C122W,S307T,A394G,G404C** | **-29.40** | **0.00** | **Y** | **37** |
| **2103:7871:5414 1:N:0:3** | **5** | **I152F,D153S,N155S,S307T,A394G** | **-17.90** | **0.00** | **N** | **38** |
| **1104:5907:19927 1:N:0:3** | **3** | **S288G,N295T,E323A** | **-13.20** | **0.13** | **N** | **39** |
| 2112:27082:7147 1:N:0:3 | 3 | T290S,L320P,F329C | -20.40 | 0.22 | Y | 39 |
| 2105:19134:23323 1:N:0:4 | 3 | Y293D,K298N,K306N | -22.80 | 0.00 | N | 39 |

VH variants of antibody 763 (4 clusters)

| VH variant name | number of mutations | mutation tuple | risk score | ABangle distance | free cysteine | Cluster Index |
|---|---|---|---|---|---|---|
| **1114:12508:8829 1:N:0:1** | **5** | **Q102P,S103P,S125P,Y196S,R306K** | **-18.00** | **0.00** | **N** | **0** |
| 1114:28111:18680 1:N:0:1 | 5 | S103L,N197K,T322P,F329V,V331G | -30.70 | 0.07 | N | 0 |
| **1101:11801:20894 1:N:0:1** | **1** | **T322P** | **-0.90** | **0.00** | **N** | **1** |
| 2106:2910:18342 1:N:0:1 | 1 | R306K | 0.00 | 0.00 | N | 1 |
| **1101:1836:14971 1:N:0:1** | **3** | **Y293D,S295N,T322P** | **-21.20** | **0.18** | **N** | **2** |
| **2103:7194:16368 1:N:0:1** | **2** | **E211A,R306K** | **-3.90** | **0.15** | **N** | **3** |

VH variants of antibody 770 (10 clusters)

| VH variant name | number of mutations | mutation tuple | risk score | ABangle distance | free cysteine | Cluster Index |
|---|---|---|---|---|---|---|
| **2111:12893:24351 1:N:0:4** | **3** | **A124V,F152I,S153D** | **-11.60** | **0.00** | **N** | **0** |
| 1119:25404:18606 1:N:0:4 | 3 | T116G,P117T,A124V | -4.70 | 0.00 | N | 0 |
| 1106:8006:17093 1:N:0:3 | 3 | T113M,A124V,S156T | -7.70 | 0.00 | N | 0 |
| 2118:26397:7206 1:N:0:3 | 3 | F152S,E211D,Y292S | -19.45 | 0.26 | N | 0 |
| **1108:9880:9108 1:N:0:3** | **1** | **K298N** | **-4.80** | **0.00** | **N** | **1** |
| 2104:2150:10503 1:N:0:3 | 1 | L406M | -0.05 | 0.00 | N | 1 |
| 2116:18898:16116 1:N:0:3 | 1 | V313A | -5.00 | 0.00 | N | 1 |
| 1106:12880:25089 1:N:0:4 | 1 | S319G | -0.20 | 0.00 | N | 1 |
| 2103:27776:11682 1:N:0:4 | 1 | I213L | -0.45 | 0.00 | N | 1 |
| 2118:19944:2771 1:N:0:4 | 1 | T405N | -0.60 | 0.00 | N | 1 |
| 1110:19477:24960 1:N:0:3 | 1 | S295N | -2.30 | 0.17 | N | 1 |
| 1118:19369:6823 1:N:0:3 | 1 | Y292S | -8.40 | 0.19 | N | 1 |
| 2118:25093:13289 1:N:0:3 | 1 | W296G | -4.00 | 0.26 | N | 1 |
| 1110:22596:20720 1:N:0:4 | 1 | Y293D | -18.00 | 0.00 | N | 1 |
| 1115:13276:12164 1:N:0:4 | 1 | T291K | -11.10 | 0.00 | N | 1 |
| 1115:26242:17606 1:N:0:4 | 1 | T291A | -7.40 | 0.00 | N | 1 |
| 2107:14816:5418 1:N:0:3 | 1 | D352V | -17.50 | 0.10 | N | 1 |
| **2101:7723:10903 1:N:0:4** | **5** | **V104L,V252I,R301P,T325P,A353K** | **-15.33** | **0.00** | **N** | **2** |
| 1102:27934:6512 1:N:0:3 | 5 | V251F,S254R,D289A,T291P,A326D | -58.30 | 0.34 | N | 2 |
| 1104:21790:9830 1:N:0:4 | 5 | V104L,V252I,R301P,R332S,A353K | -21.93 | 0.00 | N | 2 |
| 1118:12558:2046 1:N:0:4 | 5 | V104L,V252I,R301P,T303P,A353K | -18.63 | 0.00 | N | 2 |

| VH variant name | number of mutations | mutation tuple | risk score | ABangle distance | free cysteine | Cluster Index |
|---|---|---|---|---|---|---|
| 2104:22350:22387 1:N:0:4 | 5 | V104L,V252I,R301P, T321K,A353E | -14.13 | 0.00 | N | 2 |
| 2105:12294:18712 1:N:0:4 | 5 | V104L,V252I,R301P, T325M,A353K | -15.33 | 0.00 | N | 2 |
| 2110:9944:15110 1:N:0:4 | 5 | V104L,V252I,R301P, A331S,A353K | -16.43 | 0.00 | N | 2 |
| 2118:6852:18638 1:N:0:4 | 5 | V104L,V252I,R301P, D324E,A353K | -14.43 | 0.00 | N | 2 |
| 1112:26205:14133 1:N:0:3 | 5 | V104L,V252I,D289G, R301P,A353K | -25.23 | 0.14 | N | 2 |
| 2103:3741:16519 1:N:0:4 | 5 | V104L,V252I,G299D, R301P,A353K | -15.03 | 0.00 | N | 2 |
| 2118:8662:22920 1:N:0:4 | 5 | V104L,V252I,A294E, R301P,A353K | -20.43 | 0.17 | N | 2 |
| 2112:9951:11009 1:N:0:3 | 5 | V252I,A294E,S295T, R301P,A353K | -22.23 | 0.31 | N | 2 |
| 1101:3865:19178 1:N:0:4 | 5 | V104L,V252I,R301P, Y351D,A353K | -33.53 | 0.21 | N | 2 |
| 2114:9668:14956 1:N:0:4 | 5 | V104L,V252I,R301P, A353K,A396S | -16.53 | 0.11 | N | 2 |
| **1115:10270:14756 1:N:0:3** | **5** | **T123A,A124V,S156T, G197A,G199S** | **-22.30** | **0.17** | **N** | **3** |
| 2119:20444:7869 1:N:0:3 | 5 | C101Q,Q102E,S103Q, V104L,E105V | -2.85 | 0.00 | Y | 3 |
| 2112:7171:5737 1:N:0:4 | 5 | V104M,A124V,G197V,M198V,G199S | -33.40 | 0.15 | N | 3 |
| **1109:17331:3206 1:N:0:3** | **3** | **T321K,R332G,Y351D** | **-31.80** | **0.21** | **N** | **4** |
| 2105:3795:9269 1:N:0:4 | 3 | C330G,R332S,V407A | -30.40 | 0.00 | Y | 4 |
| 2111:23175:14951 1:N:0:3 | 3 | V252I,R301P,A353K | -13.03 | 0.00 | N | 4 |
| **1101:11665:4235 1:N:0:3** | **4** | **V104L,V252I,R301P, A353K** | **-13.53** | **0.00** | **N** | **5** |
| 1113:15979:20411 1:N:0:3 | 4 | A124V,S156R,G197A, G199I | -43.30 | 0.17 | N | 5 |
| 1112:18176:24346 1:N:0:4 | 4 | A124V,Y196F,G197A, G199D | -23.10 | 0.16 | N | 5 |
| 2107:13654:18311 1:N:0:3 | 4 | A124V,V252I,R301P, A353K | -17.03 | 0.00 | N | 5 |
| 2116:13810:7307 1:N:0:4 | 4 | V104M,V252I,R301P, A353K | -13.53 | 0.00 | N | 5 |
| **2117:26896:17598 1:N:0:4** | **2** | **Y292H,R301P** | **-1.45** | **0.12** | **N** | **6** |

| VH variant name | number of mutations | mutation tuple | risk score | ABangle distance | free cysteine | Cluster Index |
|---|---|---|---|---|---|---|
| 1111:21746:5128 1:N:0:3 | 2 | V104L,T116P | -0.80 | 0.00 | N | 6 |
| 1113:9389:22434 1:N:0:3 | 2 | I322N,R332G | -12.70 | 0.00 | N | 6 |
| 2103:16223:23064 1:N:0:3 | 2 | K208Q,L315P | -8.20 | 0.86 | N | 6 |
| 2105:3619:15924 1:N:0:3 | 2 | V202I,I213V | -1.17 | 0.29 | N | 6 |
| 2106:2580:14766 1:N:0:4 | 2 | S308A,S410A | -0.10 | 0.00 | N | 6 |
| 1106:23729:2056 1:N:0:4 | 2 | I213S,W296V | -8.60 | 0.26 | N | 6 |
| 2105:4288:18963 1:N:0:4 | 2 | T291N,I322S | -8.60 | 0.00 | N | 6 |
| 2104:6077:14499 1:N:0:4 | 2 | V251G,Y292F | -19.70 | 0.51 | N | 6 |
| 1119:11057:10711 1:N:0:3 | 2 | T321K,Y351A | -16.60 | 0.20 | N | 6 |
| 2110:10010:2769 1:N:0:4 | 2 | R332S,Y395S | -22.40 | 0.17 | N | 6 |
| 2111:27401:9934 1:N:0:4 | 2 | G355R,D394V | -29.00 | 0.24 | N | 6 |
| **2115:12441:16084 1:N:0:3** | **1** | **T123I** | **0.00** | **0.00** | **N** | **7** |
| 1103:19179:13949 1:N:0:3 | 1 | G207V | 0.00 | 0.00 | N | 7 |
| 1106:18574:16375 1:N:0:3 | 1 | S103P | 0.00 | 0.00 | N | 7 |
| 1114:23251:5565 1:N:0:3 | 1 | S103L | 0.00 | 0.00 | N | 7 |
| 1118:25289:12604 1:N:0:3 | 1 | A124T | -4.00 | 0.00 | N | 7 |
| 1118:20338:15736 1:N:0:3 | 1 | V104M | -0.50 | 0.00 | N | 7 |
| 2104:11879:9882 1:N:0:3 | 1 | V104L | -0.50 | 0.00 | N | 7 |
| 2110:10996:2922 1:N:0:3 | 1 | P206L | 0.00 | 0.00 | N | 7 |
| 2102:5275:22352 1:N:0:4 | 1 | Q204H | -3.40 | 0.15 | N | 7 |
| **2101:15918:20591 1:N:0:4** | **4** | **M317I,I322T,R332V, Y351N** | **-32.40** | **0.15** | **N** | **8** |
| 1115:28168:18911 1:N:0:4 | 4 | G209A,I322S,Y328S, D352A | -30.40 | 0.37 | N | 8 |

| VH variant name | number of mutations | mutation tuple | risk score | ABangle distance | free cysteine | Cluster Index |
|---|---|---|---|---|---|---|
| 1119:16081:22926 1:N:0:3 | 4 | T290N,T291M,G355M,F397L | -42.40 | 0.40 | N | 8 |
| **1108:27622:11116 1:N:0:4** | **5** | **V104L,L118Q,V252I,R301P,A353K** | **-14.33** | **0.00** | **N** | **9** |
| 1115:27661:8185 1:N:0:4 | 5 | A124V,S156N,G197A,G199S,F329C | -32.30 | 0.24 | Y | 9 |
| 1105:27684:15476 1:N:0:4 | 5 | V104L,V112F,V252I,R301P,A353K | -14.73 | 0.00 | N | 9 |
| 1114:18185:11772 1:N:0:4 | 5 | S103L,V104L,V252I,R301P,A353K | -13.53 | 0.00 | N | 9 |
| 2103:14314:17267 1:N:0:4 | 5 | V104L,Q204R,V252I,R301P,A353K | -15.23 | 0.15 | N | 9 |
| 2106:8058:9673 1:N:0:4 | 5 | V104L,R110G,V252I,R301P,A353K | -14.33 | 0.00 | N | 9 |
| 2114:26957:9620 1:N:0:4 | 5 | P117A,A124V,V252I,R301P,A353K | -17.33 | 0.00 | N | 9 |
| 2115:2997:9890 1:N:0:4 | 5 | V104L,T123I,V252I,R301P,A353K | -13.53 | 0.00 | N | 9 |

VH variants of antibody 776 (7 clusters)

| VH variant name | number of mutations | mutation tuple | risk score | ABangle distance | free cysteine | Cluster index |
|---|---|---|---|---|---|---|
| **1117:25709:6429 1:N:0:2** | **3** | **L104V,I304S,T322I** | **-2.60** | **0.00** | **N** | **0** |
| 2109:5738:21603 1:N:0:3 | 3 | Q102R,T322I,C330W | -18.00 | 0.00 | Y | 0 |
| 1111:2345:17106 1:N:0:2 | 3 | Q102R,T322I,D324N | -3.80 | 0.00 | N | 0 |
| 1115:10228:22509 1:N:0:2 | 3 | Q102R,W201S,T322I | -22.00 | 0.00 | N | 0 |
| 1117:25724:6418 1:N:0:2 | 3 | L104V,T322I,S410P | -1.70 | 0.00 | N | 0 |
| 2101:26149:12052 1:N:0:2 | 3 | Q102R,I304N,T322I | -3.50 | 0.00 | N | 0 |
| 2115:14482:21163 1:N:0:2 | 3 | L104V,T123I,T322I | -1.40 | 0.00 | N | 0 |
| 1113:27497:17512 1:N:0:3 | 3 | Q102R,V313E,T322I | -12.00 | 0.00 | N | 0 |
| 2104:4023:13062 1:N:0:3 | 3 | Q102R,V202D,T322I | -15.00 | 0.25 | N | 0 |

| VH variant name | number of mutations | mutation tuple | risk score | ABangle distance | free cysteine | Cluster index |
|---|---|---|---|---|---|---|
| 2110:8209:7451 1:N:0:3 | 3 | Q102R,T119P,T322I | -2.60 | 0.00 | N | 0 |
| 1115:9216:7842 1:N:0:4 | 3 | Q102R,T322I,Y328F | -3.00 | 0.00 | N | 0 |
| 1112:5455:8335 1:N:0:5 | 3 | Q102R,T322I,T325P | -3.80 | 0.00 | N | 0 |
| 2104:16530:3365 1:N:0:5 | 3 | Q102R,I321K,T322I | -3.00 | 0.00 | N | 0 |
| 2104:13191:17970 1:N:0:5 | 3 | Q102R,T322I,W401R | -19.50 | 0.00 | N | 0 |
| 2115:14608:23050 1:N:0:5 | 3 | Q102R,T123I,T322I | -2.00 | 0.00 | N | 0 |
| 2109:26527:12633 1:N:0:1 | 3 | Q102R,A297T,T322I | -4.40 | 0.00 | N | 0 |
| 1109:17671:16866 1:N:0:2 | 3 | Q102R,S156I,T322I | -16.80 | 0.00 | N | 0 |
| 1116:15403:9100 1:N:0:2 | 3 | Q102R,N199T,T322I | -8.80 | 0.17 | N | 0 |
| 1111:24306:15673 1:N:0:4 | 3 | Q102R,I251S,T322I | -17.20 | 0.37 | N | 0 |
| 1109:15266:10649 1:N:0:5 | 3 | Q102R,W296R,T322I | -7.00 | 0.31 | N | 0 |
| 1113:4091:16678 1:N:0:5 | 3 | Q102R,R253S,T322I | -14.00 | 0.00 | N | 0 |
| 1117:17821:20921 1:N:0:5 | 3 | Q102R,K298E,T322I | -4.40 | 0.00 | N | 0 |
| 1119:22336:24364 1:N:0:4 | 3 | Q102R,T322I,P399L | -17.00 | 0.07 | N | 0 |
| **2111:7168:17245 1:N:0:5** | **5** | **R110D,T113K,T116A,P117S,T322I** | **-2.20** | **0.00** | **N** | **1** |
| 1107:18024:13209 1:N:0:3 | 5 | T113M,T116G,P117S,T155A,T322I | -5.40 | 0.00 | N | 1 |
| **1109:2895:14057 1:N:0:2** | **1** | **T322I** | **-0.90** | **0.00** | **N** | **2** |
| 1106:12016:11024 1:N:0:3 | 1 | Y292N | -8.40 | 0.27 | N | 2 |
| 2102:18888:2945 1:N:0:3 | 1 | F397V | -10.50 | 0.11 | N | 2 |
| **2115:18009:16858 1:N:0:4** | **4** | **Q102R,T123I,D314E,T322I** | **-2.05** | **0.00** | **N** | **3** |
| 1114:22845:6504 1:N:0:3 | 4 | Q102R,I251L,T322I,F329C | -13.90 | 0.29 | Y | 3 |
| 2117:27848:10451 1:N:0:5 | 4 | Q102R,R203C,N290K,T322I | -18.80 | 0.06 | Y | 3 |

| VH variant name | number of mutations | mutation tuple | risk score | ABangle distance | free cysteine | Cluster index |
|---|---|---|---|---|---|---|
| 1112:9308:6605 1:N:0:2 | 4 | Q102R,G108R,T155M,T322I | -10.90 | 0.00 | N | 3 |
| **1112:3580:16133 1:N:0:1** | **2** | **Q102R,T322I** | **-2.00** | **0.00** | **N** | **4** |
| **2107:19369:12039 1:N:0:3** | **1** | **L104V** | **-0.50** | **0.00** | **N** | **5** |
| **2116:22283:22116 1:N:0:2** | **4** | **Q102R,R301Q,T322I,L406M** | **-2.15** | **0.00** | **N** | **6** |
| 2112:8356:23198 1:N:0:3 | 4 | Q102R,T322I,T325K,F329L | -8.80 | 0.11 | N | 6 |

For the final selection of NGS variants for DNA synthesis and HEK transient transfection with parental VK, all "medoids" VHs were selected that had more than 1 amino acid replacement, which resulted in AB angle Distance ≤ 0.5 and had no free cysteine. Totally 31 VH have been selected for clone 755, 4 for clone 763, 10 for clone 770 and 9 for clone 776 (see Table below). As shown in the following Table selected variants were delivered either by the pool of PBMC or by the pool of antigen-enriched PBMC.

**Table:** Final selection of NGS VH variants for gene synthesis and transient transfection with parental VK.

AP: After Panning cell pool; BP: PBMC cell pool before panning

| clone variant | from | VH variant name | ABangle Distance | number of mutations | mutations | risk score | free cysteine | cluster index |
|---|---|---|---|---|---|---|---|---|
| BCC.755-1 | AP | 1101:18779:19164 1:N:0:4 | 0.00 | 5 | Q102P,V104L,S307T,D324E,A394G | -13.10 | N | 12 |
| BCC.755-2 | AP | 1101:19661:1424 1:N:0:4 | 0.00 | 2 | L120F,L154P | -13.90 | N | 15 |
| BCC.755-3 | AP | 1101:21776:24081 1:N:0:4 | 0.00 | 2 | G299A,S305P | -6.40 | N | 26 |
| BCC.755-4 | AP | 1102:23311:8375 1:N:0:4 | 0.09 | 3 | E208G,S307T,A394G | -11.20 | N | 30 |
| BCC.755-5 | AP | 1104:29248:10613 1:N:0:4 | 0.00 | 4 | I213T,S307T,E323A,A394G | -11.70 | N | 28 |

| clone variant | from | VH variant name | ABangle Distance | number of mutations | mutations | risk score | free cysteine | cluster index |
|---|---|---|---|---|---|---|---|---|
| BCC.755-6 | AP | 1104:5907:19927 1:N:0:3 | 0.13 | 3 | S288G,N295T,E323A | -13.20 | N | 39 |
| BCC.755-7 | BP | 1110:21663:11508 1:N:0:1 | 0.05 | 5 | M198L,S255K,S307T,A394G,N398T | -19.90 | N | 35 |
| BCC.755-8 | BP | 1111:20770:14582 1:N:0:1 | 0.00 | 2 | S307T,A394G | -8.40 | N | 14 |
| BCC.755-9 | AP | 1112:17135:11276 1:N:0:3 | 0.00 | 4 | E105A,T123P,S307T,A394G | -9.00 | N | 5 |
| BCC.755-10 | AP | 1113:7949:2267 1:N:0:4 | 0.00 | 5 | S307T,S319I,T322P,A331S,A394G | -12.60 | N | 21 |
| BCC.755-11 | AP | 1115:13289:24928 1:N:0:4 | 0.29 | 5 | E208G,N295V,W296G,K298T,G299V | -28.00 | N | 34 |
| BCC.755-12 | AP | 1115:4917:17781 1:N:0:3 | 0.16 | 5 | S305T,S307T,Y351N,A394G,L399V | -29.20 | N | 36 |
| BCC.755-13 | AP | 1116:8054:13480 1:N:0:4 | 0.00 | 5 | V104L,T116G,P117S,S307T,A394G | -9.60 | N | 10 |
| BCC.755-14 | AP | 1117:21523:9244 1:N:0:4 | 0.00 | 3 | T116G,P117T,I152F | -5.90 | N | 4 |
| BCC.755-15 | AP | 1117:26048:17440 1:N:0:4 | 0.00 | 3 | S305P,S307T,A394G | -13.80 | N | 0 |
| BCC.755-16 | AP | 1117:9244:19295 1:N:0:4 | 0.36 | 5 | Q102R,S307T,S319R,T327S,A394G | -10.40 | N | 8 |
| BCC.755-17 | AP | 1118:21273:1232 1:N:0:4 | 0.00 | 4 | S307T,T312P,A318X,A394G | -12.40 | N | 7 |
| BCC.755-18 | AP | 2101:5252:21433 1:N:0:3 | 0.00 | 3 | T113M,S307T,A394G | -8.40 | N | 16 |
| BCC.755-19 | AP | 2101:5682:22204 1:N:0:4 | 0.00 | 2 | I213L,T291P | -11.55 | N | 6 |
| BCC.755-20 | AP | 2102:17483:2532 1:N:0:3 | 0.16 | 3 | S307T,A394G,A396G | -14.40 | N | 23 |
| BCC.755-21 | AP | 2102:28708:12170 1:N:0:3 | 0.00 | 1 | E323A | -0.60 | N | 24 |
| BCC.755-22 | AP | 2103:7871:5414 1:N:0:3 | 0.00 | 5 | I152F,D153S,N155S,S307T,A394G | -17.90 | N | 38 |

| clone variant | from | VH variant name | ABangle Distance | number of mutations | mutations | risk score | free cysteine | cluster index |
|---|---|---|---|---|---|---|---|---|
| BCC.755-23 | AP | 2110:7379:5032 1:N:0:4 | 0.00 | 4 | R110H,S307T, A318D,A394G | -12.00 | N | 11 |
| BCC.755-24 | AP | 2111:21535:5980 1:N:0:3 | 0.00 | 1 | P117L | -0.50 | N | 2 |
| BCC.755-25 | AP | 2112:28984:9687 1:N:0:4 | 0.02 | 1 | L399F | -6.00 | N | 9 |
| BCC.755-26 | AP | 2113:22155:9432 1:N:0:4 | 0.25 | 5 | N155S,S156V, D196Y,R197G ,G199T | -62.40 | N | 1 |
| BCC.755-27 | AP | 2114:20534:11604 1:N:0:4 | 0.00 | 5 | S255G,S307T, A318D,A326G ,A394G | -23.20 | N | 25 |
| BCC.755-28 | AP | 2115:16505:5070 1:N:0:4 | 0.00 | 4 | S305P,K306T, K316T,L320R | -11.00 | N | 29 |
| BCC.755-29 | AP | 2115:3097:18045 1:N:0:3 | 0.00 | 5 | T123I,R301P, S307T,A394G, V407A | -10.80 | N | 33 |
| BCC.755-30 | AP | 2116:5079:18887 1:N:0:3 | 0.06 | 5 | T123P,E208A, S307T,S309L, A394G | -15.30 | N | 27 |
| BCC.755-31 | AP | 2119:9570:2597 1:N:0:4 | 0.26 | 4 | D153A,V251G ,S307T,A394G | -32.20 | N | 22 |
| BCC.763-1 | BP | 1101:11801:20894 1:N:0:1 | 0.00 | 1 | T322P | -0.90 | N | 1 |
| BCC.763-2 | BP | 2103:7194:16368 1:N:0:1 | 0.15 | 2 | E211A,R306K | -3.90 | N | 3 |
| BCC.763-3 | BP | 1114:12508:8829 1:N:0:1 | 0.00 | 5 | Q102P,S103P, S125P,Y196S, R306K | -18.00 | N | 0 |
| BCC.763-4 | BP | 1101:1836:14971 1:N:0:1 | 0.18 | 3 | Y293D,S295N ,T322P | -21.20 | N | 2 |
| BCC.770-1 | AP | 1101:11665:4235 1:N:0:3 | 0.00 | 4 | V104L,V252I, R301P,A353K | -13.53 | N | 5 |
| BCC.770-10 | AP | 2117:26896:17598 1:N:0:4 | 0.12 | 2 | Y292H,R301P | -1.45 | N | 6 |
| BCC.770-2 | AP | 1108:27622:11116 1:N:0:4 | 0.00 | 5 | V104L,L118Q, V252I,R301P, A353K | -14.33 | N | 9 |
| BCC.770-3 | AP | 1108:9880:9108 1:N:0:3 | 0.00 | 1 | K298N | -4.80 | N | 1 |

| clone variant | from | VH variant name | ABangle Distance | number of mutations | mutations | risk score | free cysteine | cluster index |
|---|---|---|---|---|---|---|---|---|
| BCC.770-4 | AP | 1109:17331:3206 1:N:0:3 | 0.21 | 3 | T321K,R332G,Y351D | -31.80 | N | 4 |
| BCC.770-5 | AP | 1115:10270:14756 1:N:0:3 | 0.17 | 5 | T123A,A124V,S156T,G197A,G199S | -22.30 | N | 3 |
| BCC.770-6 | AP | 2101:15918:20591 1:N:0:4 | 0.15 | 4 | M317I,I322T,R332V,Y351N | -32.40 | N | 8 |
| BCC.770-7 | AP | 2101:7723:10903 1:N:0:4 | 0.00 | 5 | V104L,V252I,R301P,T325P,A353K | -15.33 | N | 2 |
| BCC.770-8 | AP | 2111:12893:24351 1:N:0:4 | 0.00 | 3 | A124V,F152I,S153D | -11.60 | N | 0 |
| BCC.770-9 | AP | 2115:12441:16084 1:N:0:3 | 0.00 | 1 | T123I | 0.00 | N | 7 |
| BCC.776-1 | AP | 1106:12016:11024 1:N:0:3 | 0.27 | 1 | Y292N | -8.40 | N | 2 |
| BCC.776-2 | BP | 1109:2895:14057 1:N:0:2 | 0.00 | 1 | T322I | -0.90 | N | 2 |
| BCC.776-3 | AP | 1111:24306:15673 1:N:0:4 | 0.37 | 3 | Q102R,I251S,T322I | -17.20 | N | 0 |
| BCC.776-4 | BP | 1112:3580:16133 1:N:0:1 | 0.00 | 2 | Q102R,T322I | -2.00 | N | 4 |
| BCC.776-5 | BP | 1117:25709:6429 1:N:0:2 | 0.00 | 3 | L104V,I304S,T322I | -2.60 | N | 0 |
| BCC.776-6 | AP | 2107:19369:12039 1:N:0:3 | 0.00 | 1 | L104V | -0.50 | N | 5 |
| BCC.776-7 | BP | 2111:7168:17245 1:N:0:5 | 0.00 | 5 | R110D,T113K,T116A,P117S,T322I | -2.20 | N | 1 |
| BCC.776-8 | AP | 2115:18009:16858 1:N:0:4 | 0.00 | 4 | Q102R,T123I,D314E,T322I | -2.05 | N | 3 |
| BCC.776-9 | BP | 2116:22283:22116 1:N:0:2 | 0.00 | 4 | Q102R,R301Q,T322I,L406M | -2.15 | N | 6 |

*Dose response curve based binding analysis of NGS-variants*

Variant VH and parental VL plasmids were transiently co-transfected into HEK293 cells. Additionally, the reference B-cell clones were also transfected and used as reference. After purification of supernatant all clones were analyzed for binding to human and murine antigen as described in the experimental section below. The $EC_{50}$-based analysis was carried out in replicates at different occasions to warrant statistical accuracy.

Figure 3 shows a correlation plot of human and murine antigen binding displaying the reference antibody (binder identified by screening after panning) and the respective clones selected by NGS. All biochemical and mutation score data of NGS variants and BCC references are consolidated in the following Table below. The plot data show good correlations regarding binding behavior and relative ranking (see Table data). Most of the variants (60-80%) display binding $EC_{50}$ values to the human antigen comparable to the relative control, and some even a slightly improved $EC_{50}$ value. The same was observed for $EC_{50}$ values for binding to murine antigen for those clones displaying cross reactivity to murine antigen. Variants for BCC 763 were identified in PBMC NGS repertoire (before enrichment) and 80% of the variants displayed binding properties comparable to the reference; for BCC 755 and BCC 776 most of the variants with comparable behavior to the reference were recovered from the enriched pool of B-cells, yet still a couple of good binders could be obtained from total PBMC NGS repertoire. Variants for BCC 770 were recovered from NGS repertoire of enriched pool. It can be seen that for each reference antibody a variant antibody with lower $EC_{50}$ value could be identified with the method s reported herein.

**Table:** Consolidation of biochemical and mutation score data of NGS variants and BCC references, sequences by $EC_{50}$ value with human antigen (lowest first, highest last).

AP: After Panning cell pool; BP: PBMC cell pool before panning

| Variants for BCC 755 | | | | | | | |
|---|---|---|---|---|---|---|---|
| **clone variant** | **$EC_{50}$ human antigen [ng/ml]** | **$EC_{50}$ murine antigen [ng/ml]** | **from** | **ABangle Distance** | **number of mutations** | **all mutations** | **risk score** |
| BCC.755-14 | <20 | >2000 | AP | 0.00 | 3 | T116G,P117T, I152F | -5.90 |
| BCC.755-7 | <20 | >2000 | BP | 0.05 | 5 | M198L,S255K, S307T,A394G, N398T | -19.90 |
| BCC.755-24 | <20 | >2000 | AP | 0.00 | 1 | P117L | -0.50 |
| BCC.755-6 | <20 | >2000 | AP | 0.13 | 3 | S288G,N295T, E323A | -13.20 |

| | | | | | | | |
|---|---|---|---|---|---|---|---|
| **BCC.755 ref 1** | <20 | >2000 | | | | | |
| BCC.755-22 | <20 | >2000 | AP | 0.00 | 5 | I152F,D153S, N155S,S307T, A394G | -17.90 |
| BCC.755-2 | <20 | >2000 | AP | 0.00 | 2 | L120F,L154P | -13.90 |
| BCC.755-4 | <20 | >2000 | AP | 0.09 | 3 | E208G,S307T, A394G | -11.20 |
| BCC.755-13 | <20 | >2000 | AP | 0.00 | 5 | V104L,T116G, P117S,S307T, A394G | -9.60 |
| BCC.755-21 | <20 | >2000 | AP | 0.00 | 1 | E323A | -0.60 |
| BCC.755-27 | <20 | >2000 | AP | 0.00 | 5 | S255G,S307T, A318D,A326G ,A394G | -23.20 |
| BCC.755-11 | <20 | >2000 | AP | 0.29 | 5 | E208G,N295V, W296G,K298T ,G299V | -28.00 |
| BCC.755-3 | <20 | >2000 | AP | 0.00 | 2 | G299A,S305P | -6.40 |
| **BCC.755 ref 2** | <20 | >2000 | | | | | |
| BCC.755-17 | <50 | >2000 | AP | 0.00 | 4 | S307T,T312P, A318X,A394G | -12.40 |
| BCC.755-8 | <50 | >2000 | BP | 0.00 | 2 | S307T,A394G | -8.40 |
| BCC.755-25 | <50 | >2000 | AP | 0.02 | 1 | L399F | -6.00 |
| BCC.755-16 | <50 | >2000 | AP | 0.36 | 5 | Q102R,S307T, S319R,T327S, A394G | -10.40 |
| BCC.755-9 | <50 | >2000 | AP | 0.00 | 4 | E105A,T123P, S307T,A394G | -9.00 |
| BCC.755-19 | <50 | >2000 | AP | 0.00 | 2 | I213L,T291P | -11.55 |
| BCC.755-10 | <50 | >2000 | AP | 0.00 | 5 | S307T,S319I,T 322P,A331S,A 394G | -12.60 |
| BCC.755-5 | <50 | >2000 | AP | 0.00 | 4 | I213T,S307T,E 323A,A394G | -11.70 |
| BCC.755-18 | <50 | >2000 | AP | 0.00 | 3 | T113M,S307T, A394G | -8.40 |
| BCC.755-15 | <50 | >2000 | AP | 0.00 | 3 | S305P,S307T, A394G | -13.80 |
| BCC.755-30 | <50 | >2000 | AP | 0.06 | 5 | T123P,E208A, S307T,S309L, A394G | -15.30 |
| BCC.755-29 | <50 | >2000 | AP | 0.00 | 5 | T123I,R301P,S 307T,A394G,V 407A | -10.80 |
| BCC.755-23 | 50-100 | >2000 | AP | 0.00 | 4 | R110H,S307T, A318D,A394G | -12.00 |

| | | | | | | | |
|---|---|---|---|---|---|---|---|
| BCC.755-1 | >100 | >2000 | AP | 0.00 | 5 | Q102P,V104L, S307T,D324E, A394G | -13.10 |
| BCC.755-20 | >100 | >2000 | AP | 0.16 | 3 | S307T,A394G, A396G | -14.40 |
| BCC.755-12 | >2000 | >2000 | AP | 0.16 | 5 | S305T,S307T, Y351N,A394G ,L399V | -29.20 |
| BCC.755-26 | >2000 | >2000 | AP | 0.25 | 5 | N155S,S156V, D196Y,R197G ,G199T | -62.40 |
| BCC.755-28 | >2000 | >2000 | AP | 0.00 | 4 | S305P,K306T, K316T,L320R | -11.00 |
| BCC.755-31 | >2000 | >2000 | AP | 0.26 | 4 | D153A,V251G ,S307T,A394G | -32.20 |
| **Variants for BCC 763** | | | | | | | |
| **clone variant** | **$EC_{50}$ human antigen [ng/ml]** | **$EC_{50}$ murine antigen [ng/ml]** | **from** | **ABangle Distance** | **number of mutations** | **all mutations** | **risk score** |
| BCC.763-1 | <20 | <20 | BP | 0.00 | 1 | T322P | -0.90 |
| **BCC.763 ref. 1** | <20 | <20 | | | | | |
| **BCC.763 ref. 2** | <20 | <20 | | | | | |
| BCC.763-2 | <20 | <20 | BP | 0.15 | 2 | E211A,R306K | -3.90 |
| BCC.763-4 | <50 | <20 | BP | 0.18 | 3 | Y293D,S295N, T322P | -21.2 |
| BCC.763-3 | >100 | >50 | BP | 0.00 | 5 | Q102P,S103P, S125P,Y196S, R306K | -18.00 |
| **Variants for BCC 770** | | | | | | | |
| **clone variant** | **$EC_{50}$ human antigen [ng/ml]** | **$EC_{50}$ murine antigen [ng/ml]** | **from** | **ABangle Distance** | **number of mutations** | **all mutations** | **risk score** |
| BCC.770-1 | <20 | >2000 | AP | 0.00 | 4 | V104L,V252I, R301P,A353K | -13.53 |
| **BCC.770 ref. 1** | <20 | >2000 | | | | | |
| BCC.770-9 | <20 | >2000 | AP | 0.00 | 1 | T123I | 0.00 |
| BCC.770-2 | <20 | >2000 | AP | 0.00 | 5 | V104L,L118Q, V252I,R301P, A353K | -14.33 |
| **BCC.770 ref. 2** | <20 | >2000 | | | | | |

| | | | | | | | |
|---|---|---|---|---|---|---|---|
| BCC.770-8 | <20 | >2000 | AP | 0.00 | 3 | A124V,F152I, S153D | -11.60 |
| BCC.770-3 | <20 | >2000 | AP | 0.00 | 1 | K298N | -4.80 |
| BCC.770-7 | >100 | >2000 | AP | 0.00 | 5 | V104L,V252I, R301P,T325P, A353K | -15.33 |
| BCC.770-10 | >100 | >2000 | AP | 0.12 | 2 | Y292H,R301P | -1.45 |
| BCC.770-4 | >2000 | >2000 | AP | 0.21 | 3 | T321K,R332G, Y351D | -31.80 |
| BCC.770-5 | >2000 | >2000 | AP | 0.17 | 5 | T123A,A124V, S156T,G197A, G199S | -22.30 |
| BCC.770-6 | >2000 | >2000 | AP | 0.15 | 4 | M317I,I322T, R332V,Y351N | -32.40 |
| **Variants for BCC 776** | | | | | | | |
| **clone variant** | **$EC_{50}$ human antigen [ng/ml]** | **$EC_{50}$ murine antigen [ng/ml]** | **from** | **ABangle Distance** | **number of mutations** | **all mutations** | **risk score** |
| BCC.776-8 | <20 | <20 | AP | 0.00 | 4 | Q102R,T123I, D314E,T322I | -2.05 |
| BCC.776-1 | <20 | <20 | AP | 0.27 | 1 | Y292N | -8.40 |
| BCC.776-9 | <20 | <20 | BP | 0.00 | 4 | Q102R,R301Q, T322I,L406M | -2.15 |
| **BCC.776 ref. 1** | <20 | <20 | | | | | |
| BCC.776-2 | <20 | <20 | BP | 0 | 1 | T322I | -0.9 |
| **BCC.776 ref. 2** | <20 | <20 | | | | | |
| BCC.776-6 | <20 | <20 | AP | 0.00 | 1 | L104V | -0.50 |
| BCC.776-3 | <20 | <20 | AP | 0.37 | 3 | Q102R,I251S, T322I | -17.20 |
| BCC.776-7 | <20 | <20 | BP | 0.00 | 5 | R110D,T113K, T116A,P117S, T322I | -2.20 |
| BCC.776-4 | <20 | <20 | BP | 0.00 | 2 | Q102R,T322I | -2.00 |
| BCC.776-5 | <20 | <20 | BP | 0.00 | 3 | L104V,I304S, T322I | -2.60 |

Thus, with this procedure antigen specific binders could be identified with binding properties comparable (or even improved) to the antigen specific B-cell clones isolated as described. This has been demonstrated by DNA synthesis, recombinant expression and biochemical analysis of sequence-based identified variants. This opens up the way to a completely new application of NGS data.

**EXAMPLE B**

NGS variants for B-cell cloning (BCC) binders bearing developability hot-spots

Totally for seven B-cell cloning binders clonally related binders were identified in the NGS repertoires with the method as reported herein; all clones were isolated by B-cell cloning after hu-CDCP1 specific enrichment (enrichment type is indicated in the Table below) all exhibited specificity for hu-CDCP1 ($EC_{50}$/$IC_{50}$ abs (ng/ml) range below 200 ng/ml) and all VHs bore cysteine or N-glycosylation site spots in HCDRs.

**Table:** properties ofB cell clones selected for NGS variants analysis

| clone | EC/$IC_{50}$ abs [ng/ml] | List Dev. Spots | CDR2 PEP | CDR3 PEP |
|---|---|---|---|---|
| CDCP1_105 | 9.87 | Cys HCDR2 | CIYAGSGRIKYASWAKG | |
| CDCP1_ 223 | 41.23 | Cys HCDR2 | CIYAGSGGATYYASWAKG | |
| CDCP1_236 | 31.59 | N{P}[ST] HCDR2 | IINTSGNTYYANWAKG | |
| CDCP1_284 | 12.69 | Cys HCDR2 | FIGSSGTTYCATWAKG | |
| CDCP1_ 212 | 197.93 | Cys HCDR3 | | GGYACD L |
| CDCP1_ 088 | 46.76 | N{P}[ST] HCDR2 | IINTSGNTYYANWAKG | |
| CDCP1_ 234 | 27.57 | N{P}[ST] HCDR2 | IFYVATNITWYASWAKG | |
| **clone** | | | | **enrichment Type** |
| CDCP1_088 | | | | on plates coated CDCP1 protein |
| CDCP1_105 | | | | on plates coated CDCP1 protein |
| CDCP1_234 | | | | antigen specific sort using biotinylated CDCP1 with the following sortgates: rbIgM⊖/ rbIgG ⊕/ CDCP1⊕ |
| CDCP1_212 | | | | antigen specific sort using biotinylated CDCP1 with the following sortgates: rbIgM⊖/ rbIgG ⊕/ CDCP1⊕ |
| CDCP1_223 | | | | antigen specific sort using biotinylated CDCP1 with the following sortgates: rbIgM⊖/ rbIgG ⊕/ CDCP1⊕ |
| CDCP1_236 | | | | antigen specific sort using biotinylated CDCP1 with the following sortgates: rbIgM⊖/ rbIgG ⊕/ CDCP1⊕ |
| CDCP1_284 | | | | antigen specific sort using biotinylated CDCP1 with the following sortgates: rbIgM⊖/ rbIgG ⊕/ CDCP1⊕ |

NGS repertoire from PBMCs and from antigen enriched B-cells was analyzed for identification of VHs variants with ≤ 11 amino acid replacements in the entire VH (FR1 to FR4) compared to the VH of the reference B-cell binders. Those VH cognate variants with improved developability properties (no Cys and/or N-glycosylation site spots any longer in HCDRs) were gene synthesized, co-transfected with the parental light chain in HEK cells and the expressed antibodies were evaluated for binding properties in comparison with BCC references.

Dose response curve based binding analysis of NGS-variants

Variant VHs and parental VL plasmids were transiently co-transfected into HEK293 cells. Additionally, the seven parental antibodies were transfected and used as reference. After purification of supernatant all clones were analyzed for binding to human CDCP1 antigen as described in the Examples section.

Figure 4 shows binding of NGS variants to human CDCP1 in comparison to the respective parental clones. For each antibody, a different number of NGS sequence variants was tested. At least one variant for each clone could be identified that shows $EC_{50}$ values in the same range as the reference antibody (marked by *).

In the following Table for each BCC clone the VH variants identified, the B-cell source of NGS sample providing the variants, the total number of amino acid replacements in entire VH and the absolute $EC_{50}/IC_{50}$ values are shown.

**Table:** VH variants identified with improved in silico developability properties: the B cell source of NGS sample providing the variants, the total number of amino acid replacements in entire VH and the absolute $EC_{50}/IC_{50}$ values.

| Sample Name | Spot original clone | EC/IC50 abs (ng/ml) | Source | Total AA Mutation compared to reference VH |
|---|---|---|---|---|
| CDCP1_105 | Cys CDR2 | 9.87 | | |
| CDCP1_105-1 | | 26.88 | Enriched B cells | 3 |
| CDCP1_105-2 | | binding lost | Enriched B cells | 10 |
| CDCP1_105-4 | | binding lost | Enriched B cells | 6 |
| CDCP1_105-5 | | binding lost | Enriched B cells | 8 |
| CDCP1_105-6 | | binding lost | Enriched B cells | 11 |
| CDCP1_105-7 | | binding lost | Enriched B cells | 6 |
| CDCP1_105-8 | | 233.92 | Enriched B cells | 3 |
| CDCP1_105-9 | | binding lost | Enriched B cells | 9 |
| CDCP1_105-10 | | 23.56 | Enriched B cells | 2 |
| CDCP1_105-11 | | 17.66 | Enriched B cells | 2 |
| CDCP1_105-12 | | binding lost | Enriched B cells | 3 |
| CDCP1_105-13 | | binding lost | Enriched B cells | 2 |
| CDCP1_105-14 | | 42.93 | Enriched B cells | 2 |
| CDCP1_105-15 | | binding lost | Enriched B cells | 6 |
| CDCP1_105-16 | | 47.46 | Enriched B cells | 5 |
| CDCP1_105-17 | | 133.83 | Enriched B cells | 3 |
| CDCP1_105-18 | | binding lost | Enriched B cells | 6 |
| CDCP1_105-19 | | binding lost | Enriched B cells | 4 |
| CDCP1_105-20 | | binding lost | Enriched B cells | 7 |
| CDCP1_223 | Cys CDR2 | 46.59 | | |
| CDCP1_223-1 | | binding lost | PBMC | 5 |
| CDCP1_223-2 | | 46.11 | PBMC | 4 |
| CDCP1_223-3 | | 991.24 | PBMC | 6 |
| CDCP1_236 | N{P}[ST] CDR2 | 31.59 | | |

(continued)

| Sample Name | Spot original clone | EC/IC50 abs (ng/ml) | Source | Total AA Mutation compared to reference VH |
|---|---|---|---|---|
| CDCP1_236-5 | | 39.48 | PBMC | 10 |
| CDCP1_284 | Cys CDR2 | 12.69 | | |
| CDCP1_284-1 | | 14.23 | Enriched B cells | 11 |
| CDCP1_284-2 | | 12.11 | Enriched B cells | 11 |
| CDCP1_284-3 | | 10.25 | Enriched B cells | 11 |
| CDCP1_284-4 | | 10.56 | Enriched B cells | 11 |
| CDCP1_284-5 | | 14.34 | Enriched B cells | 11 |
| CDCP1_284-6 | | 16.35 | Enriched B cells | 11 |
| CDCP1_284-7 | | 14.67 | Enriched B cells | 11 |
| CDCP1_284-8 | | 13.19 | Enriched B cells | 11 |
| CDCP1_284-9 | | 8.69 | Enriched B cells | 11 |
| CDCP1_284-10 | | 14.02 | Enriched B cells | 6 |
| CDCP1_284-11 | | 8.29 | Enriched B cells | 6 |
| CDCP1_284-12 | | 17.05 | Enriched B cells | 5 |
| CDCP1_284-13 | | 15.32 | Enriched B cells | 7 |
| CDCP1_284-14 | | 16.21 | Enriched B cells | 6 |
| CDCP1_284-15 | | 11.01 | Enriched B cells | 6 |
| Sample Name | Spot original clone | EC/IC50 abs (ng/ml) | Source | Total AA Mutations compared to reference VH |
| | | | | |
| CDCP1_212 | Cys CDR3 | 197.93 | | |
| CDCP1_212-1 | | 448.61 | Enriched B cells | 3 |
| CDCP1_212-2 | | 630.55 | Enriched B cells | 2 |
| CDCP1_212-3 | | binding lost | Enriched B cells | 5 |
| CDCP1_212-4 | | binding lost | Enriched B cells | 3 |
| CDCP1_212-5 | | 189.14 | Enriched B cells | 7 |
| CDCP1_088 | N{P}[ST] CDR2 | 46.76 | | |
| CDCP1_088-1 | | binding lost | PBMC | 3 |
| CDCP1_088-2 | | binding lost | PBMC | 5 |
| CDCP1_088-3 | | 54.38 | PBMC | 10 |
| CDCP1_234 | N{P}[ST] CDR2 | 27.57 | | |
| CDCP1_234-1 | | 43.11 | Enriched B cells | 9 |
| CDCP1_234-2 | | 27.16 | Enriched B cells | 4 |
| CDCP1_234-3 | | 17.57 | Enriched B cells | 3 |
| CDCP1_234-4 | | 22.37 | Enriched B cells | 3 |
| CDCP1_234-5 | | 22.04 | Enriched B cells | 3 |

(continued)

| Sample Name | Spot original clone | EC/IC50 abs (ng/ml) | Source | Total AA Mutations compared to reference VH |
|---|---|---|---|---|
| CDCP1_234-6 | | 29.79 | Enriched B cells | 3 |
| CDCP1_234-8 | | 25.45 | Enriched B cells | 2 |
| CDCP1_234-9 | | 23.09 | Enriched B cells | 3 |
| CDCP1_234-10 | | 22.24 | Enriched B cells | 3 |
| CDCP1_234-11 | | 19.85 | Enriched B cells | 3 |
| CDCP1_234-12 | | 14.61 | Enriched B cells | 3 |
| CDCP1_234-13 | | 21.18 | Enriched B cells | 3 |
| CDCP1_234-14 | | 18.72 | Enriched B cells | 3 |
| CDCP1_234-15 | | 29.18 | Enriched B cells | 3 |

The CDRH3 sequences from confirmed binders found in the NGS pool showed quite distinct features, providing a basis to classify sequences. Some of the ‘best’ binders showed strong homology in the CDRH3 region. It has been found that the NGS repertoire pool can be used to find such variants of good binders.

Analyses with sufficient sequencing depth and optimized normalization conditions are the basis for such a process. Most importantly the complex sequence diversity has to be analyzed on the DNA level to group together sequences with comparable properties, which are likely of the same phylogenetic origin. Especially with rabbits, that not only use somatic hypermutation but also gene conversion during clonal expansion it is very difficult to make this grouping to allow the identification of clonally related VHs.

The NGS sequence pools were screened with the method as reported herein for VH variants that possess identical CDRH3 sequences as the above indicated four BCC binders. The analysis of the complete VH region on a DNA level showed that, in addition to exactly identical VHs, variants can be identified varying from the reference BCCs by numerous mutations on the V region outside CDRH3 (see Table below). Alignments of the sequences indicated that the mutations occurred at various different positions throughout the sequence and were not concentrated to a certain region (data not shown).

Table: Detailed analysis of selected CDRH3 sequences and variants within their VH.

| **CDRH3 Sequence** | **Number of Mutations in total VH (compared to closest Germline) in BCC** | **Number of mutations in total VH of all NGS Samples (compared to closest Germline)** |
|---|---|---|
| DHDTGSHPYNYENMDV (SEQ ID NO: 01) | 6 | 5, 6,7, 8,10 |
| DHDTGSHPYSYENMDV (SEQ ID NO: 02) | 6 | 6,7 |
| DHDTGSSPYNYDNMDV (SEQ ID NO: 03) | 7 | 7,8,9,11,12,18 |
| DSLSYGYAYATNYFNI (SEQ ID NO: 29) | 8 | 4,5,6,8,9,10,11,12,14 |

Beside sequences with identical CDRH3 (and mutations in the VH), the NGS pool can also be screened to identify variants with CDRH3 regions highly homologous to those of reference BCC binders. This can be done by aligning the CDRH3 of the binders with the total NGS repertoire and selecting sequences with high homology (e.g. max. one mutation on the peptide level). The following Table shows an extract of the alignment done with the CDRH3 sequences of the ‘best’ binders 9-11. The CDRH3 sequences S1-S25 found in the NGS pools are closely related to these ’best’ binders.

**Table:** Peptide CDR3 sequences of identified binders 9-11 aligned with an extract of CDR3s of the same length and a high degree of homology (S1-S25).

| | |
|---|---|
| 9<br>DHDT--GSHP-YN-YENMDV (SEQ ID NO: 01) | S12<br>DHDT--GSHP-YS-YENIDV (SEQ ID NO: 15) |
| 10<br>DHDT--GSHP-YS-YENMDV (SEQ ID NO: 02) | S13<br>DNDT --GSHP- YS-- YENMDV (SEQ ID NO: 16) |
| 11<br>DHDT--GSSP-YN-YDNMDV (SEQ ID NO: 03) | S14<br>DHDN--GSHP-YS--YENMDV (SEQ ID NO: 17) |
| S1<br>DHDT --GSSQ- YN- YDNMDV (SEQ ID NO: 04) | S15<br>DHDT --GSHP- YS--HENMDV (SEQ ID NO: 18) |
| S2<br>DHDT--GSNP-YN--YDNMDV (SEQ ID NO: 05) | S16<br>DHDT --GCHP- YS-- YENMDV (SEQ ID NO: 19) |
| S3<br>DHDT--GSSP-YN--YDNMDV (SEQ ID NO: 06) | S17<br>DHDT --GNHP- YS-- YENMDV (SEQ ID NO: 20) |
| S4<br>DHDT --GSHP- YK-- Y ANMDV (SEQ ID NO: 07) | S18<br>DHDT --GSHP- YS-- YENMDV (SEQ ID NO: 21) |
| S5<br>EHDT --GSHP- YC-- YENMDV (SEQ ID NO: 08) | S19<br>DYDT --GSHP- YN-- YENMDV (SEQ ID NO: 22) |
| S6<br>DHDT--GNHP-YN--YENMDV (SEQ ID NO: 09) | S20<br>DHDT --GSHP- YN-- YENLDV (SEQ ID NO: 23) |
| S7<br>DHDT --GSHP- YS-- YENMYV (SEQ ID NO: 10) | S21<br>DHDT--GSHP-YN--YENRDV (SEQ ID NO: 24) |
| S8<br>DHDT --GSHP- YS-- YENMDF (SEQ ID NO: 11) | S22<br>DHDT-GSHP-YN--NENMDV (SEQ ID NO: 25) |
| S9<br>DHET --GSHP- YS-- YENMDV (SEQ ID NO: 12) | S23<br>DHDT--GSHP-YN--YENMDV (SEQ ID NO: 26) |
| S10<br>EHDT --GSHP- YS-- YENMDV (SEQ ID NO: 13) | S24<br>DHDT--GSSP-DN--YDNMEV (SEQ ID NO: 27) |
| S11<br>DHDT--GSHP-YS-YDNMDV (SEQ ID NO: 14) | S25<br>DHDA--GSSP-YN-YDNMDV (SEQ ID NO: 28) |

As mentioned above, four BCC CDRH3s have been found in more than one animal in the NGS samples. It has been shown in previous studies that the more shared sequences CDR3 are found in animals after immunization suggesting these sequences to be antigen specific (Galson, J.D., et al., Crit. Rev. Immunol. 35 (2015) 463-478).

As can be seen from the above the combination of data generated by NGS in combination with sequence data from BCC can be used to identify alternative antigen specific variant antibodies to a reference antibody.

NGS data can be screened for sequences with similar or identical CDRH3 but higher mutation rate within other regions of the VH compared to i.e. BCC binders to find antibodies with improved properties.

VL

If a transgenic animal expressing a common light chain is used for immunization, solely analysis of the $V_H$-repertoire is sufficient. In other transgenic models or wild-type animals $V_H$/$V_L$ pairs need to be identified as belonging together and contributing both in equal manner to antigen specificity. For this, a wide range of methods can be used, ranging from single-cell sorting combined with single-cell cloning to special RNA capturing. For example, fusion PCR is suited quite well for combination with the UMI error correction method. The paired-chain information is retained through physically attachment of the alpha and beta (or heavy and light) transcripts from each single cell. The fusion of transcripts can be accomplished by (multiplexed) overlap-extension PCR.

The following examples and figures are provided to aid the understanding of the present invention, the true scope of which is set forth in the appended claims. It is understood that modifications can be made in the procedures set forth without departing from the scope of the invention.

## Description of the Figures

**Figure 1** VH (top) and VL (bottom) sequences of the reference antibody 763 and six variants of its VH domain. Framework and CDR classification follows Wolfguy nomenclature. In the VH variants, amino acid substitutions with regard to the reference are shown in light grey. CDRs are shaded grey in the numbering.

**Figure 2** VH (top) and VL (bottom) sequences of the reference antibody 763 and six variants of its VH domain. Residues forming part of the VH-VL orientation fingerprint have been marked with a gray background. Amino acid substitutions in this region are likely to induce a change VH-VL orientation as compared to the reference antibody. CDRs are shaded grey in the numbering.

**Figure 3** $EC_{50}$ (M) correlation plot; human versus murine LRP8 binding of all the NGS variants. The controls, the clones identified by screening and panning, (red and blue), and clones, identified by NGS (grey), were plotted; A: Variants for BCC 763, B: Variants for BCC 776, C: Variants for BCC 770, D: Variants for BCC 755.

**Figure 4** Absolute $EC_{50}$ [nM] values show binding of all NGS variants (white bars) to human CDCP1. Parental controls are shown in black. Successful NGS variants are marked with (*). na = not available, meaning that no $EC_{50}$ value could be derived from the binding curve due to weak binding of the variant. A: Variants for CDCP1_105; B: Variants for CDCP1_223; C: Variants for CDCP1_236; D: Variants for CDCP1_284; E: Variants for CDCP1_212; F: Variants for CDCP1_088; G: Variants for CDCP1 234.

**Figure 5** Scheme of the BCC and NGS workflow.

## Examples

### Example 1

**Immunization of rabbits**

A KLH conjugate of a human LRP8 was used for the immunization of the New Zealand White rabbit. Each rabbit was immunized with 500 µg of the immunogen, emulsified with complete Freund's adjuvant, at day 0 by intradermal application and 500 µg each at days 7, 14, 28, 42 by alternating intramuscular and subcutaneous applications. Thereafter, rabbits received monthly subcutaneous immunizations of 500 µg, and small samples of blood were taken 7 days after immunization for the determination of serum titers. A larger blood sample (10% of estimated total blood volume) was taken during the third, fourth and fifth month of immunization (at 5-7 days after immunization), and peripheral mononuclear cells were isolated, which were used as a source of antigen-specific B-cells in the B-cell cloning process.

### Example 2

**Determination of serum titers (ELISA)**

Biotinylated human LRP8 was immobilized on a 96-well streptavidin-coated plate at 0.5 µg/ml, 100 µl/well, in PBS, followed by blocking of the plate with 2 % CroteinC in PBS, 200 µl/well, Thereafter 100 µl/well serial dilutions of antisera, in duplicates, in 0.5 % CroteinC in PBS were applied. The detection was done with HRP-conjugated donkey anti-rabbit IgG antibody (Jackson Immunoresearch/Dianova, Cat. No. 711-036-152; 1/16 000), each diluted in 0.5 % CroteinC in PBS, 100 µl/well, For all steps, plates were incubated for 1 h at 37 °C. Between all steps plates were washed 3 times with 0.05 % Tween 20 in PBS. Signal was developed by addition of BM Blue POD (peroxidase)-substrate soluble (Roche Diagnostics GmbH, Mannheim, Germany), 100 µl/well; and stopped by addition of 1 M HCl, 100 µl/well. Absorbance was read out at 450 nm, against 690 nm as reference. Titer was defined as dilution of antisera resulting in half-maximal signal.

### Example 3

**Isolation of rabbit peripheral blood mononuclear cells (PBMCs)**

Blood samples were taken of immunized wild-type rabbits (NZW). EDTA containing whole blood was diluted twofold with 1x PBS (PAA, Pasching, Austria) before density centrifugation using lympholyte mammal (Cedarlane Laboratories, Burlington, Ontario, Canada) according to the specifications of the manufacturer. The PBMCs were washed twice with 1x PBS.

## Example 4

### Depletion of macrophages/monocytes

The PBMCs were seeded on sterile KLH-coated SA-6-well-plates to deplete macrophages and monocytes through unspecific adhesion and to remove cell binding to KLH. Each well was filled at maximum with 4 ml medium and up to 6 × 10E6 PBMCs from the immunized rabbit and were allowed to bind for 1 h at 37 °C and 5 % CO2. The cells in the supernatant (peripheral blood lymphocytes (PBLs)) were used for the antigen panning step.

## Example 5

### Enrichment of B-cells

Sterile streptavidin coated 6-well plates (Microcoat, Bernried, Germany) were coated either with 2 μg/ml of the biotinylated KLH protein or the biotinylated LRP8/CDCP1 protein in PBS for 3 h at room temperature. Prior to the panning step these 6-well plates were washed three times with sterile PBS. Coated plates were seeded with up to 6 × 10E6 PBLs per 4 ml medium and allowed to bind for 1 h at 37 °C and 5 % CO2. Non-adherent cells were removed by carefully washing the wells 1-2 times with 1x PBS. The remaining sticky cells were detached by trypsin for 10 min. at 37 °C and 5 % CO2. Trypsination was stopped with EL-4 B5 medium. The cells were kept on ice until the immune fluorescence staining,

EL-4 B5 medium

RPMI 1640 (Pan Biotech, Aidenbach, Germany) supplemented with 10 % FCS (Hyclone, Logan, UT, USA), 2 mM Glutamine, 1 % penicillin/streptomycin solution (PAA, Pasching, Austria), 2 mM sodium pyruvate, 10 mM HEPES (PAN Biotech, Aidenbach, Germany) and 0.05 mM beta-mercaptoethanol (Gibco, Paisley, Scotland).

## Example 6

### Immune fluorescence staining and Flow Cytometry

An anti-IgG antibody FITC conjugate (AbD Serotec, Düsseldorf, Germany) was used for single cell sorting. For surface staining, B-cells pre-treated with a depletion step and an enrichment step (Example 4 and 5) were incubated with the anti-IgG antibody FITC conjugate in PBS (phosphate buffered saline solution) and incubated for 45 min. in the dark at 4 °C. After staining the cells were washed two times with ice cold PBS. Finally, the labelled B-cells were resuspended in ice cold PBS and immediately subjected to the FACS analyses. Propidium iodide in a concentration of 5 μg/ml (BD Pharmingen, San Diego, CA, USA) was added prior to the FACS analyses to discriminate between dead and live cells.

A Becton Dickinson FACSAria equipped with a computer and the FACSDiva software (BD Biosciences, USA) were used for single cell sort.

## Example 7

### B-cell cultivation

The cultivation of the single sorted B-cells was done according to a method described by Seeber et al. (Seeber, S., et al., PLoS One, 9 (2014) e86184.). Briefly, single sorted rabbit B-cells were incubated in 96-well plates with 200 μl/well EL-4 B5 medium containing Pansorbin Cells (1:100,000) (Calbiochem (Merck), Darmstadt, Germany), 5% rabbit thymocyte supernatant (MicroCoat, Bernried, Germany) and gamma-irradiated murine EL-4 B5 thymoma cells (5 × 10E5 cells/well) for 7 days at 37 °C in the incubator. The supernatants of the B-cell cultivation were removed for screening and the remaining cells were harvested immediately and frozen at - 80 °C in 100 μl RLT buffer (Qiagen, Hilden, Germany).

## Example 8

### Enzyme-linked immunosorbent assay (ELISA)

Human antigen:

The antigen, biotinylated human LRP8, was incubated with 5 μL sample containing the anti-LRP8 antibody at a concentration of 250 ng/mL in a total volume of 25 μL in PBS, 0.5% BSA and 0.05 % Tween in a 384w microtiterplate (Maxisorb (with Streptavidin, Nunc). After 1.5 hrs. incubation at 25 °C unbound antibody was removed by washing 6 times with 90 μL PBS (dispense and aspiration). The antigen-antibody complex was detected by an anti-rabbit antibody conjugated to POD (ECL anti-rabbit IgG-POD, Cat. No. NA9340V; POD=peroxidase). 20-30 min after adding 35 μL POD-substrate 3,3',5,5'-tetramethyl benzidine (TMB; Piercenet, Cat. No. 34021) the optical density was determined at 370 nm. The $EC_{50}$ value was calculated with a four parameter logistic model using GraphPad Prism 6.0 software.

Murine antigen:

The antigen, biotinylated murine LRP8, was incubated with 5 μL sample containing the anti-LRP8 antibody at a concentration of 250 ng/mL in a total volume of 25 μL in PBS, 0.5% BSA and 0.05 % Tween in a 384w microtiterplate (Maxisorb (with Streptavidin, Nunc). After 1.5 hrs. incubation at 25 °C unbound antibody was removed by washing 6 times with 90 μL PBS (dispense and aspiration). The antigen-antibody complex was detected by an anti-rabbit antibody conjugated to POD (ECL anti-rabbit IgG-POD, Cat. No. NA9340V). 20-30 min after adding 35 μL POD-substrate 3,3',5,5'-tetramethyl benzidine (TMB, Piercenet, Cat. No. 34021) the optical density was determined at 370 nm. The $EC_{50}$ value was calculated with a four parameter logistic model using GraphPad Prism 6.0 software.

## Example 9

### PCR amplification of V-domains for SLIC cloning

Total RNA was prepared from B-cell lysates (resuspended in RLT buffer (Qiagen, Cat. No. 79216) using the NucleoSpin 8/96 RNA kit (Macherey&Nagel; Cat. No. 740709.4, 740698) according to manufacturer's protocol. RNA was eluted with 60 μl RNase free water. 6 μl of RNA was used to generate cDNA by reverse transcriptase reaction using the Superscript III First-Strand Synthesis SuperMix (Invitrogen, Cat. No. 18080-400) and an oligo dT-primer according to the manufacturer's instructions. All steps were performed on a Hamilton ML Star System. 4 μl of cDNA were used to amplify the immunoglobulin heavy and light chain variable regions (VH and VL) with the AccuPrime SuperMix (Invitrogen, Cat. No. 12344-040) in a final volume of 50 μl using the primers rbHC.up and rbHC.do for the heavy chain and rbLC.up and rbLC.do for the light chain:

| | |
|---|---|
| rbHC.up | AAGCTTGCCACCATGGAGACTGGGCTGCGCTGGCTTC (SEQ ID NO: 30) |
| rbHC.do | CCATTGGTGAGGGTGCCCGAG (SEQ ID NO: 31) |
| rbLC.up | AAGCTTGCCACCATGGACAYGAGGGCCCCCACTC (SEQ ID NO: 32) |
| rbLC.do | CAGAGTRCTGCTGAGGTTGTAGGTAC (SEQ ID NO: 33) |

All forward primers were specific for the signal peptide (of respectively VH and VL) whereas the reverse primers were specific for the constant regions (of respectively CH1 and CL). The PCR conditions for the RbVH+RbVL were as follows: hot start at 94 °C for 5 min.; 35 cycles: 20 sec. at 94 °C; 20 sec. at 70 °C; 45 sec. at 68 °C; final extension at 68 °C for 7 min.

8 μl of the 50 μl PCR solution were loaded on a 48 E-Gel 2% (Invitrogen, Cat. No. G8008-02). Positive PCR reactions were purified using the NucleoSpin Extract II kit (Macherey&Nagel; Cat. No. 740609250) according to manufacturer's protocol and eluted in 50 μl elution buffer. All purification steps were performed on a Hamilton ML Starlet System. 5 μl of purified VH and VL PCR solutions were used for DNA-sequencing.

## Example 10

### NGS VH-PCR from PBMCs and antigen-enriched B-cells

4.2 × 10E6 PBMCs and 1.2 × 10E6 antigen-enriched B-cells were resuspended in 300 μl RLT Buffer (Qiagen; Cat. No. 79216). Total RNA was prepared from B-cell lysates using RNeasy Mini or Micro kit (Qiagen; Cat. No. 74134) according to manufacturer's protocol. RNA was eluted in 50 μl and 30 μl RNase free water, respectively, for PBMCs and antigen-enriched B-cells. 6 μl of RNA was used to generate cDNA by reverse transcriptase reaction using the Superscript III First-Strand Synthesis SuperMix (Invitrogen, Cat. No. 18080-400) and an oligo dT-primer according to the manufacturer's instructions.

50-80 ng of cDNA were used to amplify the immunoglobulin heavy chain variable regions (VH) with the AccuPrime SuperMix (Invitrogen, Cat. No. 12344-040) in a final volume of 50 μl using the primers rbHCfinal_FS.up and rbHC_shortCH1_fs2.do:

| | |
|---|---|
| rbHCfinal_FS.up | ATGGAGACTGGGCTGCGCTGGCTTC (SEQ ID NO: 34) |
| rbHC_shortCH1_fs2.do | GGGAAGACTGATGGAGC (SEQ ID NO: 35) |

The forward primer is specific for the signal peptide VH whereas the reverse primers specific for the constant regions is. The PCR conditions were as follows: Hot start at 94°C for 3 min.; 22 cycles: 20 sec. at 94 °C; 20 sec. at 68 °C; 40 sec. at 68 °C; final extension at 68 °C for 5 min. Totally 6 PCR reactions were performed on each cell pool sample.

8 μl of one PCR reaction were loaded on a 12 E-Gel 2% (Invitrogen, Cat. No. G521802). All PCR reactions respectively for the 2 B-cell libraries (PBMC-library; antigen-enriched B-cell-library) were purified with one column using the NucleoSpin Extract II kit (Macherey&Nagel; Cat. No. 740609) according to manufacturer's protocol and eluted in 50 μl elution buffer. 5 μl of cleaned VH PCR solutions were used for DNA-MiSeq sequencing.

## Example 11

### Template preparation for NGS Sequencing

Paired-Ends Run 2x300 Base: Minimal DNA amount for Samples: 100 ng, good 500 ng

The NGS sequencing was run on MiSeq from Illumina. After purification on AMPure XP beads PCR templates were assessed on a DNA1000 Agilent BioAnalyzer Chip. The library preparation was performed using the TruSeq Nano DNA Sample Preparation Kit according to manufacturer's protocol.

The final libraries were quantified using qPCR technology. qPCR reactions were prepared according to the KAPA SYBR FAST qPCR protocol and run using the Roche Light Cycler 480. The samples were pooled and contrasted with PhiX.

In more detail, the libraries were analyzed by a paired-end Illumina MiSeq sequencing run with Illumina sequencing primers.

All reagents were thawed at RT just before starting experiment. The reagent cartridge was thawed in a water bath. The cartridge was inverted several times to ensure mixing of reagents and all air bubbles were removed by hitting the cartridge on the bench. 1 mL of 0.2 M NaOH was prepared by adding 200 μl 1 M NaOH to 800 μL laboratory-graded water. The prepared solution was vortexed, spun down and stored on ice. Flow cell was brought to RT, and carefully washed with laboratory-graded water, dried using kimtech precision wipes and inserted into the sequencer following the instructions. 5 μl of 4 nM DNA library pool was mixed with freshly diluted 0.2 M NaOH, vortexed briefly and spun down on a table top centrifuge. The solution was incubated for 5 min. at Room temperature and 990 μL pre-chilled HT1 was added and mixed by briefly vortexing. The resulting 20 pM denatured library in 1 mM NaOH was stored on Ice until further use. To obtain 600 μl of a 12 pM library, 360 μL of the 20 pM denatured library was diluted with 240 μl pre-chilled HT1, inverted several times to mix and then pulse centrifuged. The resulting 12 pM library was stored on ice until further use. To prepare 4 nM PhiX library, 2 μL of the 10 nM PhiX library control was added to 3 μl of 10 mM Tris-HCl, pH 8.5 with 0.1 % Tween 20. The dilution was briefly vortexed and pulse centrifuged. To denature the PhiX Control 5 μl freshly diluted 0.2 M NaOH was added to the 5 μL of the prepared 4 nM PhiX library, vortexed briefly and spun down on a table top centrifuge. The solution was incubated for 5 min. at Room temperature and 990 μL pre-chilled HT1 was added and mixed by briefly vortexing. To obtain a 12.5 pM PhiX library, 375 μL of the 20 pM denatured PhiX solution was mixed with 225 μL Pre-chilled HT1, briefly vortexed and pulse centrifuged. 520 μL 12 pM Sample library and 80 μL 12.5 pM PhiX were combined to create a library with 15% PhiX control spike-in. The combined sample library and PhiX control were stored on ice until loaded onto the MiSeq reagent cartridge.

## Example 12

### Bioinformatics Analysis of NGS Sequences for identification of clonally related VH variants

Data from Illumina MiSeq consist of two paired and usually overlapping reads per sequence. All data have been analyzed using the following workflow:

> Assembly of paired reads by FLASH (any other software tool should work as well)

- o FLASH available from http://ccb.jhu.edu/software/FLASH/
- o Using Flash v1.2.10 with DEFAULT PARAMETERS (no outies, min overlap 10bp, max overlap 65 bp)
- o Result: Overlapped sequences without Illumina adaptors

> Extraction of antibody variable domains:

o Translating DNA to all 6 ORFs
o For each ORF:

- Searching peptide sequence for FW1 by comparing to a consensus FW1 sequence and counting the difference. Continuing if that value is above a defined threshold.
- Alike searching for FW2, trying to connect to FW1 (area in between is CDR1)
- Alike searching for FW3, trying to connect to FW2 (area in between is CDR2)
- Alike searching for FW4, trying to connect to FW3 (area in between is CDR3)

o Usually, in just 1 of the 6 ORFs a variable domain with the above described procedure can be identified. If multiples are found, a score that described the distance to the consensus is calculated and best ORF is selected.
◦ For variable domains found, several values are. Most importantly, the closest germlines were detected by simply aligning the variable domain sequence to the available germline repertoire provided by IMGT and report the best hit. By this it also reports per sequence the V/D/J Germlines that are most likely to be the origin of those sequences.
◦ Result: Table with one row per sequence containing all information about the contained variable domain.
o Additional Analysis performed: Calculated #Mutations for each CDR/FR on DNA/PEP level compared to the reference sequences.

**Example 13**

**Transient transfection of NGS VH Variants with parental VL**

For recombinant expression of NGS variants, PCR-products coding for parental VL of B-cell clones were cloned as cDNA into expression vectors by the overhang cloning method (Haun, R.S., et al., BioTechniques 13 (1992) 515-518; Li, M.Z., et al., Nature Methods 4 (2007) 251-256) in an expression cassette containing the rabbit constant region to accept the VL region. The expression vectors contained an expression cassette consisting of a 5' CMV promoter including intron A, and a 3' BGH poly adenylation sequence. In addition to the expression cassette, the plasmids contained a pUC18-derived origin of replication and a beta-lactamase gene conferring ampicillin resistance for plasmid amplification in E. coli. Furthermore, the expression vector contained the rabbit kappa LC constant region to accept the VL regions.

Linearized expression plasmids coding for the kappa constant region and VL inserts were amplified by PCR using overlapping primers. Purified PCR products were incubated with T4 DNA-polymerase which generated single-strand overhangs. The reaction was stopped by dCTP addition. In the next step, plasmid and insert were combined and incubated with recA which induced site specific recombination. The recombined plasmids were transformed into E. coli. The next day the grown colonies were picked and tested for correct recombined plasmid by plasmid preparation, restriction analysis and DNA-sequencing.

Selected NGS VH variants were synthesized (Gene Art, Regensburg, Germany) and cloned as cDNA into expression vectors. The expression vectors contained an expression cassette consisting of a 5' CMV promoter including intron A, and a 3' BGH poly adenylation sequence. In addition to the expression cassette, the plasmids contained a pUC18-derived origin of replication and a beta-lactamase gene conferring ampicillin resistance for plasmid amplification in E. coli. Furthermore, the expression vector contained the rabbit IgG constant region designed to accept the VH regions.

For antibody expression, 500 ng of the isolated HC and LC plasmids were transiently co-transfected into 2 ml (96-well plate) of FreeStyle HEK293-F cells (Invitrogen, Cat. No. R790-07) by using 239-Free Transfection Reagent (Novagen) following procedure suggested by Reagent supplier. After 1-week cultivation the HEK supernatants were harvested, filtered (1.2 μm Supor-PALL) and purified with MabSelectSuRe (50 μl, GE Healthcare). Columns were equilibrated with 1 × PBS. Samples were eluted with 2.5 mM HCl, pH 2.6 and neutralized with 10 x PBS.

**Example 14**

**Staining procedure for antigen (CDCP1) specific sort**

The cells from the macrophage depletion step were used to perform the antigen specific sort. In a first round the cells were incubated with the biotinylated CDCP1 antigen at a concentration of 5 μg/ml on ice. After two washing steps the biotinylated and cell-bound CDCP1 was detected with an A647-streptavidin conjugate (Invitrogen). In parallel, the anti-IgG FITC (AbD Serotec, Düsseldorf, Germany) and the anti-IgM PE (BD Pharmingen) antibodies were added. The stained cells were washed two times. Finally, the PBMCs were resuspended in ice cold PBS and immediately subjected to the FACS analyses. The cell gate used for the single cell sorting was: rbIgM⊖/ rbIgG ⊕/ CDCP1⊕.

**Example 15**

**Screening Hu CDCP1 Binders**

Nunc Maxisorb streptavidin coated plates (MicroCoat, Cat. No. #11974998001) were coated with 25 µl/well biotinylated human CDCP1-AviHis fusion protein at a concentration of 200 ng/ml and incubated at 4 °C over night. After washing (2x90 µl/well with PBST-buffer (Phosphate Buffered Saline Tween-20)) 25 µl anti-CDCP1 antibody samples were added and incubated for one hour at RT. After washing (3x90 µl/well with PBST-buffer) 25 µl/well goat-anti-human IgG-HRP conjugate (Millipore, Cat. No. AP502P) was added in 1:1,000 dilution and incubated at RT for one hour on a shaker. After washing (4x90 µl/well with PBST-buffer) 25 µl/well TMB substrate (Roche Diagnostics GmbH, Cat. No. 11835033001) was added and incubated until OD reached 1.5 - 2.5. The reaction was stopped by the addition of 25 µl/well 1 N HCl. Measurement took place at 370/492 nm.

**Example 16**

**NGS VH-PCR from PBMCs and antigen-enriched (panning sample) B-cells**

4 * 10E6 PBMCs and 352 antigen-enriched B-cells were resuspended in 350 µl RLT Buffer (Qiagen, Cat. No. 79216). Total RNA was prepared from B-cells lysate using RNeasy Mini or Micro kit (Qiagen, Cat. No. 74134) according to manufacturer's protocol. RNA was eluted in 50 µl and 30 µl RNase free water, respectively, for PBMCs and antigen-enriched B-cells. 6 µl of RNA was used to generate cDNA by reverse transcriptase reaction using the Superscript III First-Strand Synthesis SuperMix (Invitrogen, Cat. No. 18080-400) and an oligo dT-primer according to the manufacturer's instructions.

50-80 ng of cDNA were used to amplify the immunoglobulin heavy chain variable regions (VH) with the Accu-Prime SuperMix (Invitrogen, Cat. No. 12344-040) in a final volume of 50 µl using the primers rbHCfinal_FS.up and rbHC_shortCH1_fs2.do:

| | |
|---|---|
| rbHCfinal_FS.up | ATGGAGACTGGGCTGCGCTGGCTTC (SEQ ID NO: 34) |
| rbHC_shortCH1_fs2.do | GGGAAGACTGATGGAGC (SEQ ID NO: 35) |

The forward primer is specific for the signal peptide VH whereas the reverse primers specific for the constant regions is. The PCR conditions were as follows: hot start at 94°C for 3 min; 20 and 29 cycles (respectively for PBMC and antigen-enriched samples) of 20 sec. at 94 °C; 20 sec. at 68 °C; 40 sec. at 68 °C; final extension at 68 °C for 5 min. Totally 6-8 PCR reactions were performed each cell pool sample.

CDCP1: 3 wt-rabbits (5571, 5565, 5566)

| Sample ID | Description | Animal | PCRs | Cycles |
|---|---|---|---|---|
| **G1** (5571) | PBMC (Lympholite) | **5571** | 6x50 µl PCR a 1 µl cDNA | 20 |
| **G2** (5565) | PBMC (Lympholite) | **5565** | 6x50 µl PCR a 1 µl cDNA | 20 |
| **G3** (5566) | PBMC (Lympholite) | **5566** | 6x50 µl PCR a 1 µl cDNA | 20 |
| **G4** (5565) | MΦ dep. + AG Sort | **5565** | 4x50 µl PCR a 4 µl cDNA | 29 |
| **G5** (5566) | MΦ dep. + AG Sort | **5566** | 4x50 µl PCR a 4 µl cDNA | 29 |
| **G6** (5571) | MΦ dep. + AG Sort | **5571** | 4x50 µl PCR a 4 µl cDNA | 29 |

Sequencing results: number of rabbit VH sequences and clusters

| name | total sequences | non-pairable | bad VH sequence | OK | H3 Clusters | H3 Clusters n>3 |
|---|---|---|---|---|---|---|
| **G1** | 1,021,345 | 149,110 | 44,692 | 827,543 | 28,487 | 8,506 |

(continued)

| name | total sequences | non-pairable | bad VH sequence | OK | H3 Clusters | H3 Clusters n>3 |
|---|---|---|---|---|---|---|
| **G2** | 1,040,062 | 146,080 | 47,609 | 846,373 | 21,842 | 4,544 |
| **G3** | 1,406,058 | 245,445 | 75,106 | 1,085,507 | 30,947 | 6,876 |
| **G4** | 1,019,453 | 147,601 | 41,484 | 830,368 | 4,774 | 1,349 |
| **G5** | 1,166,355 | 171,698 | 60,861 | 933,796 | 4,847 | 1,055 |
| **G6** | 1,166,807 | 173,888 | 55,718 | 937,201 | 3,753 | 806 |

## Example 17

### HEK Transient transfection of NGS VH Variants with parental VL

For recombinant expression of NGS variants, PCR-products coding for parental VL of B-cell clones were cloned as cDNA into expression vectors by the overhang cloning method (Haun, R.S., et al., BioTechniques 13 (1992) 515-518; Li, M.Z., et al., Nature Methods 4 (2007) 251-256). The expression vectors contained an expression cassette consisting of a 5' CMV promoter including intron A, and a 3' BGH poly adenylation sequence. In addition to the expression cassette, the plasmids contained a pUC18-derived origin of replication and a beta-lactamase gene conferring ampicillin resistance for plasmid amplification in E. coli. Furthermore, the expression vector contained the rabbit kappa LC constant region to accept the VL regions.

Linearized expression plasmids coding for the kappa constant region and VL inserts were amplified by PCR using overlapping primers. Purified PCR products were incubated with T4 DNA-polymerase which generated single-strand overhangs. The reaction was stopped by dCTP addition. In the next step, plasmid and insert were combined and incubated with recA which induced site specific recombination. The recombined plasmids were transformed into E. coli. The next day the grown colonies were picked and tested for correct recombined plasmid by plasmid preparation, restriction analysis and DNA-sequencing.

Selected NGS VH variants were synthesized (Gene Art, Regensburg, Germany) and cloned as cDNA into expression vectors. The expression vectors contained an expression cassette consisting of a 5' CMV promoter including intron A, and a 3' BGH poly adenylation sequence. In addition to the expression cassette, the plasmids contained a pUC18-derived origin of replication and a beta-lactamase gene conferring ampicillin resistance for plasmid amplification in E. coli. Furthermore, the expression vector contained the rabbit IgG constant region designed to accept the VH regions.

For antibody expression, 500 ng of the isolated HC and LC plasmids were transiently co-transfected into 2 ml (96-well plate) of HEK293-F cells (Invitrogen, Cat. No. R790-07) by using 239-Free Transfection Reagent (Novagen) following procedure suggested by Reagent supplier. After 1-week cultivation the HEK supernatants were harvested, filtered (1.2 μm Supor-PALL) and purified with MabSelectSuRe (50 μl, GE Healthcare). Columns were equilibrated with 1 × PBS. Samples were eluted with 2.5 mM HCl, pH 2.6 and neutralized with 10 × PBS.

## Example 18

### Human CDCP1 Binding ELISA

Nunc Maxisorb streptavidin coated plates (MicroCoat, Cat. No. #11974998001) were coated with 25 μl/well biotinylated human CDCP1-AviHis fusion protein at a concentration of 200 ng/ml and incubated at 4 °C over night. After washing (2x90 μl/well with PBST-buffer) 25 μl anti-CDCP1 antibody samples were added in a 1:2 dilution series starting at 5 μg/ml. Plates were incubated one hour at RT. After washing (3x90 μl/well with PBST-buffer) 25 μl/well of a mix of goat-anti-human IgG-HRP conjugate (Jackson, Cat. No. 109-036-098) and donkey-anti-rabbit IgG (GE Healthcare, Cat. No. NA9340V, Lot#389592,) was added in 1:9,000 dilution and incubated at RT for one hour on a shaker. After washing (4x90 μl/well with PBST-buffer) 25 μl/well TMB substrate (Roche, Cat. No. 11835033001) was added and incubated until OD reached 1.5 - 2.5. The reaction was stopped by addition of 25 μl/well 1 N HCl. Measurement took place at 370/492 nm.

## Claims

**1.** A method for selecting a variant of a reference antibody variable domain, wherein the reference antibody variable domain has at least one of i) an unpaired Cys-residue in the variable domain or the HVR, ii) a glycosylation site, or iii) a post-translationally modified Asp, Asn or Met amino acid residue, the method comprising the following steps:

- aligning antibody variable domain encoding nucleic acids produced by sequencing nucleic acids from a multitude of B-cell clones each producing an antibody specifically binding to the same target as the reference antibody with the sequence of the reference antibody being the template sequence; and
- selecting a sequence that has the highest similarity to the reference sequence wherein at least one of the at least one amino acid residue of i) an unpaired Cys-residue in the variable domain or the HVR, ii) a glycosylation site, or iii) a post-translationally modified Asp, Asn or Met amino acid residue of the reference antibody variable domain is replaced by/changed to an amino acid residue that is not post-translationally modified,

wherein the B-cells are obtained from the same immunization campaign as the reference antibody, wherein the B-cells have been enriched for antigen-specific antibody expressing B-cells.

**2.** A method for identifying a variant antibody of a reference antibody specifically binding to the same target/antigen comprising the following steps:

i) generating one or more sets of VH or/and VL sequences of a multitude of amino acid sequences or nucleic acid sequences of variant antibodies of the reference antibody specifically binding to the same target as the reference antibody, which have been determined by next generation sequencing, wherein the sequences are aligned with the amino acid sequence or nucleic acid sequence of a reference antibody as template, whereby the reference antibody specifically binds to a target/antigen, based on

α) identical length of VH or/and VL, or/and
β) identical length of all β-sheet framework regions, or/and
γ) identical length of all HVRs/CDRs, or/and
δ) identical HVR3/CDR3 sequence, mutations in frameworks and/or HVRs/CDRs with up to 3 amino acid exchanges allowed, or/and
ε) homologous HVR/CDR3 sequence with up to 2 amino acid exchanges allowed and identical HVR/CDR1 and 2, or/and
ζ) mutations in frameworks and HVR/CDRs are allowed;

ii) ranking the aligned sequences in the one or more sets of i) by

α) the number of, and/or
β) the position(s) of, and/or
γ) the change of the physico-chemical properties resulting from the, and/or
δ) the difference of VH/VL orientation resulting from the amino acid difference(s) to the reference antibody sequence;

iii) identifying one of the best 10 aligned and ranked antibodies as a variant antibody of a reference antibody, whereby the variant antibody does have at least one of i) an unpaired Cys-residue in the variable domain or the HVR, ii) a glycosylation site, or iii) a post-translationally modified Asp, Asn or Met amino acid residue less as the reference antibody,

wherein the multitude of amino acid sequences or nucleic acid sequences of variant antibodies specifically binding to the same target as the reference antibody are obtained from B-cells from the same immunization campaign as the reference antibody, wherein the B-cells have been enriched for antigen-specific antibody expressing B-cells.

**3.** The method according to claim 2, wherein step i) further comprises annotating the sequence with the same numbering scheme, which is the Wolfguy numbering scheme.

**4.** The method according to any one of claims 2 to 3, wherein differences in the sequence are annotated in the form: reference antibody amino acid residue-position-variant antibody amino acid residue and are grouped into a mutation tuple.

**5.** The method according to any one of claims 2 to 4, wherein the change of the physico-chemical properties is determined by the change in charge, hydrophobicity and/or size.

**6.** The method according to any one of claims 2 to 5, wherein the change of the physico-chemical properties is determined using a mutation risk score.

**7.** The method according to claim 6, wherein the mutation risk score is determined based on the following Table, wherein residues that are not explicitly given in this Table are weighted with the value one:

| Wolfguy Index | Weight | Wolfguy Index | Weight |
|---|---|---|---|
| 101 | 0.2 | 151 | 2 |
| 102 | 1.1 | 152 | 2.6 |
| 103 | 0 | 153 | 1.2 |
| 104 | 0.5 | 154 | 2.3 |
| 105 | 0.2 | 155 | 1.9 |
| 106 | 0.8 | 156 | 3.7 |
| 107 | 0.5 | 157 | 4 |
| 108 | 0.8 | 158 | 4 |
| 109 | 0.4 | 193 | 4 |
| 110 | 0.2 | 194 | 4 |
| 111 | 0 | 195 | 4 |
| 112 | 0.4 | 196 | 3.3 |
| 113 | 0 | 197 | 3.9 |
| 114 | 0.1 | 198 | 2.6 |
| 115 | 0.6 | 199 | 3.4 |
| 116 | 0.1 | 251 | 3.8 |
| 117 | 0.1 | 252 | 1.9 |
| 118 | 0.2 | 253 | 4 |
| 119 | 0.2 | 254 | 3.8 |
| 120 | 1.2 | 255 | 3.6 |
| 121 | 0 | 256 | 4 |
| 122 | 4 | 287 | 4 |
| 123 | 0 | 288 | 4 |
| 124 | 2 | 289 | 3.9 |
| 125 | 0.5 | 290 | 3.4 |
| 201 | 4 | 291 | 3.7 |
| 202 | 2.6 | 292 | 2.1 |
| 203 | 2 | 293 | 3.6 |
| 204 | 1.7 | 294 | 2.3 |
| 205 | 1 | 295 | 2.3 |
| 206 | 0 | 296 | 1 |
| 207 | 0 | 297 | 1.2 |
| 208 | 0.7 | 298 | 2.4 |
| 209 | 1.6 | 299 | 0.5 |
| 210 | 2 | 351 | 4 |
| 211 | 1.3 | 352 | 3.5 |
| 212 | 3.1 | 353 | 4 |
| 213 | 0.9 | 354 | 3.5 |
| 214 | 3.1 | 355 | 3.5 |
| 301 | 0.1 | 356 | 3 |
| 302 | 1.2 | 357 | 3 |
| 303 | 1.7 | 358 | 3 |
| 304 | 0.3 | 359 | 3 |
| 305 | 1.8 | 360 | 3 |
| 306 | 0 | 361 | 3 |
| 307 | 2.4 | 362 | 3 |

(continued)

| Wolfguy Index | Weight | Wolfguy Index | Weight |
|---|---|---|---|
| 308 | 0 | 363 | 3 |
| 309 | 1.2 | 364 | 3 |
| 334 | 1 | 365 | 3 |
| 335 | 1 | 366 | 3 |
| 336 | 1 | 367 | 3 |
| 337 | 1 | 382 | 3 |
| 310 | 0.8 | 383 | 3 |
| 311 | 0.9 | 384 | 3 |
| 312 | 0.8 | 385 | 3 |
| 313 | 2.5 | 386 | 3 |
| 314 | 0.1 | 387 | 3 |
| 315 | 1.5 | 388 | 3 |
| 316 | 0 | 389 | 3 |
| 317 | 1.5 | 390 | 3 |
| 318 | 0.8 | 391 | 3 |
| 319 | 0.1 | 392 | 3 |
| 320 | 1.4 | 393 | 3 |
| 321 | 0.2 | 394 | 3 |
| 322 | 0.3 | 395 | 3.5 |
| 323 | 0.2 | 396 | 3 |
| 324 | 1.8 | 397 | 3.5 |
| 325 | 0.6 | 398 | 1.5 |
| 326 | 2.2 | 399 | 3 |
| 333 | 1 | | |
| 327 | 0.6 | | |
| 328 | 3 | | |
| 329 | 2.5 | | |
| 330 | 4 | | |
| 331 | 2.9 | | |
| 332 | 2.8 | | |
| 401 | 3.5 | | |
| 402 | 2 | | |
| 403 | 0.5 | | |
| 404 | 1 | | |
| 405 | 0.3 | | |
| 406 | 0.1 | | |
| 407 | 1 | | |
| 408 | 0.1 | | |
| 409 | 1 | | |
| 410 | 0.1 | | |
| 411 | 0.1 | | |

with positions 101 to 125 corresponding to heavy chain variable domain framework 1, positions 151 to 199 corresponding to CDR-H1, positions 201 to 214 corresponding to heavy chain variable domain framework 2, positions 251 to 299 corresponding to CDR-H2, positions 301 to 332 corresponding to heavy chain variable domain framework 3, positions 351 to 399 corresponding to CDR-H3, positions 401 to 411 corresponding to heavy chain variable domain framework 4.

## Patentansprüche

1. Verfahren zum Auswählen einer Variante einer variablen Domäne eines Referenzantikörpers, wobei die variable Domäne des Referenzantikörpers mindestens eines von i) einem ungepaarten Cys-Rest in der variablen Domäne oder der HVR, ii) einer Glykosylierungsstelle oder iii) einem post-translational modifiziertem Asp-, Asn- oder Met-Aminosäurerest aufweist, wobei das Verfahren die folgenden Schritte umfasst:

   - Alignieren von für die variable Domäne des Antikörpers kodierenden Nukleinsäuren, die durch Sequenzierung von Nukleinsäuren aus einer Vielzahl von B-Zellklonen produziert werden, die jeweils einen Antikörper produzieren, der spezifisch an dasselbe Ziel bindet wie der Referenzantikörper, wobei die Sequenz des Referenzantikörpers die Matrizensequenz ist; und
   - Auswählen einer Sequenz, die die höchste Ähnlichkeit mit der Referenzsequenz hat, wobei mindestens einer des mindestens einen Aminosäurerestes von i) einem ungepaarten Cys-Rest in der variablen Domäne oder der HVR, ii) einer Glykosylierungsstelle oder iii) einem post-translational modifizierten Asp-, Asn- oder Met-Aminosäurerest der variablen Domäne des Referenzantikörpers durch einen Aminosäurerest, der nicht post-translational modifiziert ist, ersetzt/in einen solchen geändert ist,

   wobei die B-Zellen aus derselben Immunisierungskampagne wie der Referenzantikörper erhalten werden, wobei die B-Zellen hinsichtlich antigenspezifische Antikörper exprimierender B-Zellen angereichert worden sind.

2. Verfahren zum Identifizieren einer Antikörpervariante eines Referenzantikörpers, die spezifisch an dasselbe Ziel/Antigen bindet, umfassend die folgenden Schritte:

   i) Erzeugen eines Satzes oder mehrerer Sätze von VH- oder/und VL-Sequenzen einer Vielzahl von Aminosäuresequenzen oder Nukleinsäuresequenzen von Antikörpervarianten des Referenzantikörpers, die spezifisch an dasselbe Ziel binden wie der Referenzantikörper und durch Sequenzierung der nächsten Generation bestimmt wurden, wobei die Sequenzen mit der Aminosäuresequenz oder Nukleinsäuresequenz eines Referenzantikörpers als Matrize aligniert werden, wodurch der Referenzantikörper spezifisch an ein Ziel! Antigen bindet, basierend auf Folgendem

   $\alpha$) identischer Länge von VH oder/und VL oder/und
   $\beta$) identischer Länge aller $\beta$-Faltblatt-Gerüstregionen oder/und
   $\gamma$) identischer Länge aller HVR/CDR oder/und
   $\delta$) identischer HVR3/CDR3-Sequenz, Mutationen in Gerüsten und/oder HVR/CDR mit bis zu 3 Aminosäureaustauschen zulässig, oder/und
   $\varepsilon$) homologer HVR/CDR3-Sequenz mit bis zu 2 Aminosäureaustauschen zulässig und identischer HVR/CDR1 und 2 oder/und
   $\xi$) Mutationen in Gerüsten und HVR/CDR sind zulässig;

   ii) Einstufen der alignierten Sequenzen in dem einen Satz oder den mehreren Sätzen von i) nach

   $\alpha$) der Anzahl an Aminosäureunterschieden zu der Referenzantikörpersequenz und/oder
   $\beta$) der/den Position(en) von Aminosäureunterschieden zu der Referenzantikörpersequenz und/oder
   $\gamma$) der Veränderung der physikochemischen Eigenschaften, die aus dem/den Aminosäureunterschied(en) zu der Referenzantikörpersequenz resultiert, und/oder
   $\delta$) der unterschiedlichen VH/VL-Ausrichtung, die aus dem/den Aminosäureunterschied(en) zu der Referenzantikörpersequenz resultiert;

   iii) Identifizieren eines der besten 10 alignierten und eingestuften Antikörper als eine Antikörpervariante eines Referenzantikörpers, wodurch die Antikörpervariante mindestens eines von i) einem ungepaarten Cys-Rest in der variablen Domäne oder der HVR, ii) einer Glykosylierungsstelle oder iii) einem post-translational modifiziertem Asp-, Asn- oder Met-Aminosäurerest weniger als der Referenzantikörper aufweist,

   wobei die Vielzahl von Aminosäuresequenzen oder Nukleinsäuresequenzen von Antikörpervarianten, die spezifisch an dasselbe Ziel wie der Referenzantikörper binden, aus B-Zellen aus derselben Immunisierungskampagne wie der Referenzantikörper erhalten werden, wobei die B-Zellen hinsichtlich antigenspezifische Antikörper exprimierender B-Zellen angereichert worden sind.

3. Verfahren nach Anspruch 2, wobei Schritt i) ferner das Kennzeichnen der Sequenz mit demselben Nummerierungsschema, welches das Wolfguy-Nummerierungsschema ist, umfasst.

4. Verfahren nach einem der Ansprüche 2 bis 3, wobei Unterschiede in der Sequenz in folgender Form gekennzeichnet werden: Aminosäurerest des Referenzantikörpers - Position - Aminosäurerest der Antikörpervariante und in ein Mutations-Tupel gruppiert werden.

5. Verfahren nach einem der Ansprüche 2 bis 4, wobei die Veränderung der physikochemischen Eigenschaften anhand der Veränderung der Ladung, Hydrophobie und/oder Größe bestimmt wird.

6. Verfahren nach einem der Ansprüche 2 bis 5, wobei die Veränderung der physikochemischen Eigenschaften unter Verwendung eines Mutationsrisiko-Scores bestimmt wird.

7. Verfahren nach Anspruch 6, wobei der Mutationsrisiko-Score basierend auf der folgenden Tabelle bestimmt wird, wobei Reste, die in dieser Tabelle nicht ausdrücklich angegeben sind, mit dem Wert eins gewichtet werden:

| Wolfguy-Index | Wichtung | Wolfguy-Index | Wichtung |
|---|---|---|---|
| 101 | 0,2 | 151 | 2 |
| 102 | 1,1 | 152 | 2,6 |
| 103 | 0 | 153 | 1,2 |
| 104 | 0,5 | 154 | 2,3 |
| 105 | 0,2 | 155 | 1,9 |
| 106 | 0,8 | 156 | 3,7 |
| 107 | 0,5 | 157 | 4 |
| 108 | 0,8 | 158 | 4 |
| 109 | 0,4 | 193 | 4 |
| 110 | 0,2 | 194 | 4 |
| 111 | 0 | 195 | 4 |
| 112 | 0,4 | 196 | 3,3 |
| 113 | 0 | 197 | 3,9 |
| 114 | 0,1 | 198 | 2,6 |
| 115 | 0,6 | 199 | 3,4 |
| 116 | 0,1 | 251 | 3,8 |
| 117 | 0,1 | 252 | 1,9 |
| 118 | 0,2 | 253 | 4 |
| 119 | 0,2 | 254 | 3,8 |
| 120 | 1,2 | 255 | 3,6 |
| 121 | 0 | 256 | 4 |
| 122 | 4 | 287 | 4 |
| 123 | 0 | 288 | 4 |
| 124 | 2 | 289 | 3,9 |
| 125 | 0,5 | 290 | 3,4 |
| 201 | 4 | 291 | 3,7 |
| 202 | 2,6 | 292 | 2,1 |
| 203 | 2 | 293 | 3,6 |
| 204 | 1,7 | 294 | 2,3 |
| 205 | 1 | 295 | 2,3 |
| 206 | 0 | 296 | 1 |
| 207 | 0 | 297 | 1,2 |
| 208 | 0,7 | 298 | 2,4 |
| 209 | 1,6 | 299 | 0,5 |
| 210 | 2 | 351 | 4 |
| 211 | 1,3 | 352 | 3,5 |
| 212 | 3,1 | 353 | 4 |

(fortgesetzt)

| Wolfguy-Index | Wichtung | Wolfguy-Index | Wichtung |
|---|---|---|---|
| 213 | 0,9 | 354 | 3,5 |
| 214 | 3,1 | 355 | 3,5 |
| 301 | 0,1 | 356 | 3 |
| 302 | 1,2 | 357 | 3 |
| 303 | 1,7 | 358 | 3 |
| 304 | 0,3 | 359 | 3 |
| 305 | 1,8 | 360 | 3 |
| 306 | 0 | 361 | 3 |
| 307 | 2,4 | 362 | 3 |
| 308 | 0 | 363 | 3 |
| 309 | 1,2 | 364 | 3 |
| 334 | 1 | 365 | 3 |
| 335 | 1 | 366 | 3 |
| 336 | 1 | 367 | 3 |
| 337 | 1 | 382 | 3 |
| 310 | 0,8 | 383 | 3 |
| 311 | 0,9 | 384 | 3 |
| 312 | 0,8 | 385 | 3 |
| 313 | 2,5 | 386 | 3 |
| 314 | 0,1 | 387 | 3 |
| 315 | 1,5 | 388 | 3 |
| 316 | 0 | 389 | 3 |
| 317 | 1,5 | 390 | 3 |
| 318 | 0,8 | 391 | 3 |
| 319 | 0,1 | 392 | 3 |
| 320 | 1,4 | 393 | 3 |
| 321 | 0,2 | 394 | 3 |
| 322 | 0,3 | 395 | 3,5 |
| 323 | 0,2 | 396 | 3 |
| 324 | 1,8 | 397 | 3,5 |
| 325 | 0,6 | 398 | 1,5 |
| 326 | 2,2 | 399 | 3 |
| 333 | 1 | | |
| 327 | 0,6 | | |
| 328 | 3 | | |
| 329 | 2,5 | | |
| 330 | 4 | | |
| 331 | 2,9 | | |
| 332 | 2,8 | | |
| 401 | 3,5 | | |
| 402 | 2 | | |
| 403 | 0,5 | | |
| 404 | 1 | | |
| 405 | 0,3 | | |
| 406 | 0,1 | | |
| 407 | 1 | | |
| 408 | 0,1 | | |
| 409 | 1 | | |
| 410 | 0,1 | | |
| 411 | 0,1 | | |

wobei Positionen 101 bis 125 Gerüst 1 der variablen Domäne der schweren Kette entsprechen, Positionen 151 bis 199 CDR-H1 entsprechen, Positionen 201 bis 214 Gerüst 2 der variablen Domäne der schweren Kette entsprechen, Positionen 251 bis 299 CDR-H2 entsprechen, Positionen 301 bis 332 Gerüst 3 der variablen Domäne der schweren Kette entsprechen, Positionen 351 bis 399 CDR-H3 entsprechen, Positionen 401 bis 411 Gerüst 4 der variablen Domäne der schweren Kette entsprechen.

## Revendications

**1.** Procédé permettant de sélectionner un variant d'un domaine variable d'anticorps de référence, dans lequel le domaine variable d'anticorps de référence a au moins l'un parmi i) un résidu Cys non apparié dans le domaine variable ou la HVR, ii) un site de glycosylation, ou iii) un résidu d'acide aminé Asp, Asn ou Met modifié de manière post-traductionnelle, le procédé comprenant les étapes suivantes :

- l'alignement d'acides nucléiques codant pour un domaine variable d'anticorps produits par séquençage d'acides nucléiques à partir d'une multitude de clones de lymphocytes B produisant chacun un anticorps se liant spécifiquement à la même cible que l'anticorps de référence, la séquence de l'anticorps de référence étant la séquence modèle ; et
- la sélection d'une séquence qui a la similitude la plus élevée avec la séquence de référence dans laquelle au moins l'un parmi l'au moins un résidu d'acide aminé de i) un résidu Cys non apparié dans le domaine variable ou la HVR, ii) un site de glycosylation, ou iii) un résidu d'acide aminé Asp, Asn ou Met modifié de manière post-traductionnelle du domaine variable d'anticorps de référence est remplacé par/changer en un résidu d'acide aminé qui n'est pas modifié de manière post-traductionnelle,

dans lequel les lymphocytes B sont obtenus à partir de la même campagne d'immunisation que l'anticorps de référence, dans lequel les lymphocytes B ont été enrichis en lymphocytes B exprimant des anticorps spécifiques à un antigène.

**2.** Procédé permettant d'identifier un anticorps variant d'un anticorps de référence se liant spécifiquement à la même cible/au même antigène comprenant les étapes suivantes :

i) la génération d'un ou plusieurs ensembles de séquences VH et/ou VL d'une multitude de séquences d'acides aminés ou de séquences d'acide nucléique d'anticorps variants de l'anticorps de référence se liant spécifiquement à la même cible que l'anticorps de référence, qui ont été déterminés par séquençage de nouvelle génération, les séquences étant alignées avec la séquence d'acides aminés ou la séquence d'acide nucléique d'un anticorps de référence en tant que modèle, moyennant quoi l'anticorps de référence se lie spécifiquement à une cible/un antigène, sur la base de

$\alpha$) une longueur identique de VH et/ou VL, ou/et
$\beta$) une longueur identique de toutes les régions charpente en feuillet $\beta$, ou/et
$\gamma$) une longueur identique de toutes les HVR/CDR, ou/et
$\delta$) une séquence HVR3/CDR3 identique, de mutations dans les charpentes et/ou des HVR/CDR avec jusqu'à 3 échanges d'acides aminés autorisés, ou/et
$\varepsilon$) une séquence HVR/CDR3 homologue avec jusqu'à 2 échanges d'acides aminés autorisés et des HVR/CDR1 et 2 identiques, ou/et
$\zeta$) des mutations dans les charpentes et des HVR/CDR autorisées ;

ii) le classement des séquences alignées dans le ou les ensembles de i) selon

$\alpha$) le nombre de, et/ou
$\beta$) la ou les position(s) de, et/ou
$\gamma$) le changement des propriétés physico-chimiques résultant de la ou des, et/ou
$\delta$) la différence d'orientation de VH/VL résultant de la ou des différence(s) d'acides aminés par rapport à la séquence d'anticorps de référence ;

iii) l'identification d'un des 10 meilleurs anticorps alignés et classés en tant qu'anticorps variant d'un anticorps de référence, moyennant quoi l'anticorps variant n'a pas au moins l'un parmi i) un résidu Cys non apparié dans le domaine variable ou la HVR, ii) un site de glycosylation, ou iii) un résidu d'acide aminé Asp, Asn ou Met modifié de manière post-traductionnelle de moins que l'anticorps de référence,

dans lequel la multitude de séquences d'acides aminés ou de séquences d'acide nucléique d'anticorps variants se liant spécifiquement à la même cible que l'anticorps de référence sont obtenues à partir de lymphocytes B de la même campagne d'immunisation que l'anticorps de référence, dans lequel les lymphocytes B ont été enrichis en lymphocytes B exprimant des anticorps spécifiques à un antigène.

3. Procédé selon la revendication 2, dans lequel l'étape i) comprend en outre l'annotation de la séquence avec le même schéma de numérotation, qui est le schéma de numérotation de Wolfguy.

4. Procédé selon l'une quelconque des revendications 2 à 3, dans lequel les différences dans la séquence sont annotées sous la forme de : résidu d'acide aminé d'anticorps de référence-position-résidu d'acide aminé d'anticorps variant et sont regroupées en un tuple de mutations.

5. Procédé selon l'une quelconque des revendications 2 à 4, dans lequel le changement des propriétés physico-chimiques est déterminé par le changement de charge, d'hydrophobicité et/ou de taille.

6. Procédé selon l'une quelconque des revendications 2 à 5, dans lequel le changement des propriétés physico-chimiques est déterminé en utilisant un score de risque de mutation.

7. Procédé selon la revendication 6, dans lequel le score de risque de mutation est déterminé sur la base du tableau suivant, dans lequel les résidus qui ne sont pas explicitement indiqués dans ce tableau sont pondérés avec la valeur un :

| Indice de Wolfguy | Poids | Indice de Wolfguy | Poids |
|---|---|---|---|
| 101 | 0,2 | 151 | 2 |
| 102 | 1,1 | 152 | 2,6 |
| 103 | 0 | 153 | 1,2 |
| 104 | 0,5 | 154 | 2,3 |
| 105 | 0,2 | 155 | 1,9 |
| 106 | 0,8 | 156 | 3,7 |
| 107 | 0,5 | 157 | 4 |
| 108 | 0,8 | 158 | 4 |
| 109 | 0,4 | 193 | 4 |
| 110 | 0,2 | 194 | 4 |
| 111 | 0 | 195 | 4 |
| 112 | 0,4 | 196 | 3,3 |
| 113 | 0 | 197 | 3,9 |
| 114 | 0,1 | 198 | 2,6 |
| 115 | 0,6 | 199 | 3,4 |
| 116 | 0,1 | 251 | 3,8 |
| 117 | 0,1 | 252 | 1,9 |
| 118 | 0,2 | 253 | 4 |
| 119 | 0,2 | 254 | 3,8 |
| 120 | 1,2 | 255 | 3,6 |
| 121 | 0 | 256 | 4 |
| 122 | 4 | 287 | 4 |
| 123 | 0 | 288 | 4 |
| 124 | 2 | 289 | 3,9 |
| 125 | 0,5 | 290 | 3,4 |
| 201 | 4 | 291 | 3,7 |
| 202 | 2,6 | 292 | 2,1 |
| 203 | 2 | 293 | 3,6 |
| 204 | 1,7 | 294 | 2,3 |

(suite)

| Indice de Wolfguy | Poids | Indice de Wolfguy | Poids |
|---|---|---|---|
| 205 | 1 | 295 | 2,3 |
| 206 | 0 | 296 | 1 |
| 207 | 0 | 297 | 1,2 |
| 208 | 0,7 | 298 | 2,4 |
| 209 | 1,6 | 299 | 0,5 |
| 210 | 2 | 351 | 4 |
| 211 | 1,3 | 352 | 3,5 |
| 212 | 3,1 | 353 | 4 |
| 213 | 0,9 | 354 | 3,5 |
| 214 | 3,1 | 355 | 3,5 |
| 301 | 0,1 | 356 | 3 |
| 302 | 1,2 | 357 | 3 |
| 303 | 1,7 | 358 | 3 |
| 304 | 0,3 | 359 | 3 |
| 305 | 1,8 | 360 | 3 |
| 306 | 0 | 361 | 3 |
| 307 | 2,4 | 362 | 3 |
| 308 | 0 | 363 | 3 |
| 309 | 1,2 | 364 | 3 |
| 334 | 1 | 365 | 3 |
| 335 | 1 | 366 | 3 |
| 336 | 1 | 367 | 3 |
| 337 | 1 | 382 | 3 |
| 310 | 0,8 | 383 | 3 |
| 311 | 0,9 | 384 | 3 |
| 312 | 0,8 | 385 | 3 |
| 313 | 2,5 | 386 | 3 |
| 314 | 0,1 | 387 | 3 |
| 315 | 1,5 | 388 | 3 |
| 316 | 0 | 389 | 3 |
| 317 | 1,5 | 390 | 3 |
| 318 | 0,8 | 391 | 3 |
| 319 | 0,1 | 392 | 3 |
| 320 | 1,4 | 393 | 3 |
| 321 | 0,2 | 394 | 3 |
| 322 | 0,3 | 395 | 3,5 |
| 323 | 0,2 | 396 | 3 |
| 324 | 1,8 | 397 | 3,5 |
| 325 | 0,6 | 398 | 1,5 |
| 326 | 2,2 | 399 | 3 |
| 333 | 1 | | |
| 327 | 0,6 | | |
| 328 | 3 | | |
| 329 | 2,5 | | |
| 330 | 4 | | |
| 331 | 2,9 | | |
| 332 | 2,8 | | |
| 401 | 3,5 | | |
| 402 | 2 | | |
| 403 | 0,5 | | |

(suite)

| Indice de Wolfguy | Poids | Indice de Wolfguy | Poids |
|---|---|---|---|
| 404 | 1 | | |
| 405 | 0,3 | | |
| 406 | 0,1 | | |
| 407 | 1 | | |
| 408 | 0,1 | | |
| 409 | 1 | | |
| 410 | 0,1 | | |
| 411 | 0,1 | | |

les positions 101 à 125 correspondant à la charpente de domaine variable de chaîne lourde 1, les positions 151 à 199 correspondant à CDR-H1, les positions 201 à 214 correspondant à la charpente de domaine variable de chaîne lourde 2, les positions 251 à 299 correspondant à CDR-H2, les positions 301 à 332 correspondant à la charpente de domaine variable de chaîne lourde 3, les positions 351 à 399 correspondant à CDR-H3, les positions 401 à 411 correspondant à la charpente de domaine variable de chaîne lourde 4.

# Figure 1

```
                     11111111111111111111111111111111111222222222222222222222222222222
                     00000000011111111112222225555559999000000000111115555889999999999
                     12345678901234567890123451234566789123456789012341234890123456789
763 VH (Reference)   CQSVEESGGRLVTPGTPLTLTCTASGFSLSSYNMNWVRQAPGKGLEWIGYINKGGSAYYASWAKG
1101:11801:20894     CQSVEESGGRLVTPGTPLTLTCTASGFSLSSYNMNWVRQAPGKGLEWIGYINKGGSAYYASWAKG
1101:1836:14971      CQSVEESGGRLVTPGTPLTLTCTASGFSLSSYNMNWVRQAPGKGLEWIGYINKGGSAYDANWAKG
1114:12508:8829      CPPVEESGGRLVTPGTPLTLTCTAPGFSLSSSNMNWVRQAPGKGLEWIGYINKGGSAYYASWAKG
1114:28111:18680     CQLVEESGGRLVTPGTPLTLTCTASGFSLSSYKMNWVRQAPGKGLEWIGYINKGGSAYYASWAKG
2103:7194:16368      CQSVEESGGRLVTPGTPLTLTCTASGFSLSSYNMNWVRQAPGKGLAWIGYINKGGSAYYASWAKG
2106:2910:18342      CQSVEESGGRLVTPGTPLTLTCTASGFSLSSYNMNWVRQAPGKGLEWIGYINKGGSAYYASWAKG

                     55555555555555555555555555555555555666666666666666666666677777777
                     00000000011111111112222555566789999000000000111111599999900000000
                     12345678901234567890123123612116789123456789012345145678912345678
763 VL (Reference)   AAVLTQTPSPVSAAVGGTVTISCQSSPNILGNYLSWFQQKPGQPPKLLIYYTSTLASGVPSRFKG
```

# Figure 1 cont.

```
                     333333333333333333333333333333333333333444444444444
                     000000001111111111222222222233555599999000000000011
                     123456781234567890123456789012123456789123456789 01
763 VH (Reference)   RFTISRTSTTVDLKMTSPTTEDTATYFCVRSGGGGNLNLWGQGTLVTVSS
1101:11801:20894     RFTISRTSTTVDLKMTSPTPEDTATYFCVRSGGGGNLNLWGQGTLVTVSS
1101:1836:14971      RFTISRTSTTVDLKMTSPTPEDTATYFCVRSGGGGNLNLWGQGTLVTVSS
1114:12508:8829      RFTISKTSTTVDLKMTSPTTEDTATYFCVRSGGGGNLNLWGQGTLVTVSS
1114:28111:18680     RFTISRTSTTVDLKMTSPTPEDTATYVCGRSGGGGNLNLWGQGTLVTVSS
2103:7194:16368      RFTISKTSTTVDLKMTSPTTEDTATYFCVRSGGGGNLNLWGQGTLVTVSS
2106:2910:18342      RFTISKTSTTVDLKMTSPTTEDTATYFCVRSGGGGNLNLWGQGTLVTVSS

                     777777777777777777777777777777777778888888888
                     011111111222222222233333555555999990000000001
                     901256789012345678901234123456567891234567890
763 VL (Reference)   SGSGTQFTLTISDVQCDDAATYYCLGVYRSDSDNVFGGGTEVVVK
```

# Figure 2

```
                              11111111111111111111111111111111111222222222222222222222222222222
                              00000000011111111112222225555559999000000000111115555889999999999
                              12345678901234567890123451234566789123456789012341234890123456789
BBB.763 VH (Reference)        CQSVEESGGRLVTPGTPLTLTCTASGFSLSSYNMNWVRQAPGKGLEWIGYINKGGSAYYASWAKG
1101:11801:20894 1:N:0:1      CQSVEESGGRLVTPGTPLTLTCTASGFSLSSYNMNWVRQAPGKGLEWIGYINKGGSAYYASWAKG
1101:1836:14971 1:N:0:1       CQSVEESGGRLVTPGTPLTLTCTASGFSLSSYNMNWVRQAPGKGLEWIGYINKGGSAYDANWAKG
1114:12508:8829 1:N:0:1       CPPVEESGGRLVTPGTPLTLTCTAPGFSLSSSNMNWVRQAPGKGLEWIGYINKGGSAYYASWAKG
1114:28111:18680 1:N:0:1      CQLVEESGGRLVTPGTPLTLTCTASGFSLSSYKMNWVRQAPGKGLEWIGYINKGGSAYYASWAKG
2103:7194:16368 1:N:0:1       CQSVEESGGRLVTPGTPLTLTCTASGFSLSSYNMNWVRQAPGKGLAWIGYINKGGSAYYASWAKG
2106:2910:18342 1:N:0:1       CQSVEESGGRLVTPGTPLTLTCTASGFSLSSYNMNWVRQAPGKGLEWIGYINKGGSAYYASWAKG

                              55555555555555555555555555555555555666666666666666666666677777777
                              00000000011111111112222555566789999000000000111111599999900000000
                              12345678901234567890123123612116789123456789012345145678912345678
BBB.763 VL (Reference)        AAVLTQTPSPVSAAVGGTVTISCQSSPNILGNYLSWFQQKPGQPPKLLIYYTSTLASGVPSRFKG
```

# Figure 2 cont.

```
                                 333333333333333333333333333333333333333344444444444
                                 000000001111111111222222222233555559999900000000011
                                 123456781234567890123456789012123455678912345678901
BBB.763 VH (Reference)           RFTISRTSTTVDLKMTSPTTEDTATYFCVRSGGGXGNLNLWGQGTLVTVSS
1101:11801:20894 1:N:0:1         RFTISRTSTTVDLKMTSPTPEDTATYFCVRSGGGXGNLNLWGQGTLVTVSS
1101:1836:14971 1:N:0:1          RFTISRTSTTVDLKMTSPTPEDTATYFCVRSGGGXGNLNLWGQGTLVTVSS
1114:12508:8829 1:N:0:1          RFTISKTSTTVDLKMTSPTTEDTATYFCVRSGGGXGNLNLWGQGTLVTVSS
1114:28111:18680 1:N:0:1         RFTISRTSTTVDLKMTSPTPEDTATYVCGRSGGGXGNLNLWGQGTLVTVSS
2103:7194:16368 1:N:0:1          RFTISKTSTTVDLKMTSPTTEDTATYFCVRSGGGXGNLNLWGQGTLVTVSS
2106:2910:18342 1:N:0:1          RFTISKTSTTVDLKMTSPTTEDTATYFCVRSGGGXGNLNLWGQGTLVTVSS

                                 777777777777777777777777777777777778888888888
                                 011111111222222222233333555555999990000000001
                                 901256789012345678901234123456567891234567890
BBB.763 VL (Reference)            SGSGTQFTLTISDVQCDDAATYYCLGVYRSDSDNVFGGGTEVVVK
```

Figure 3A

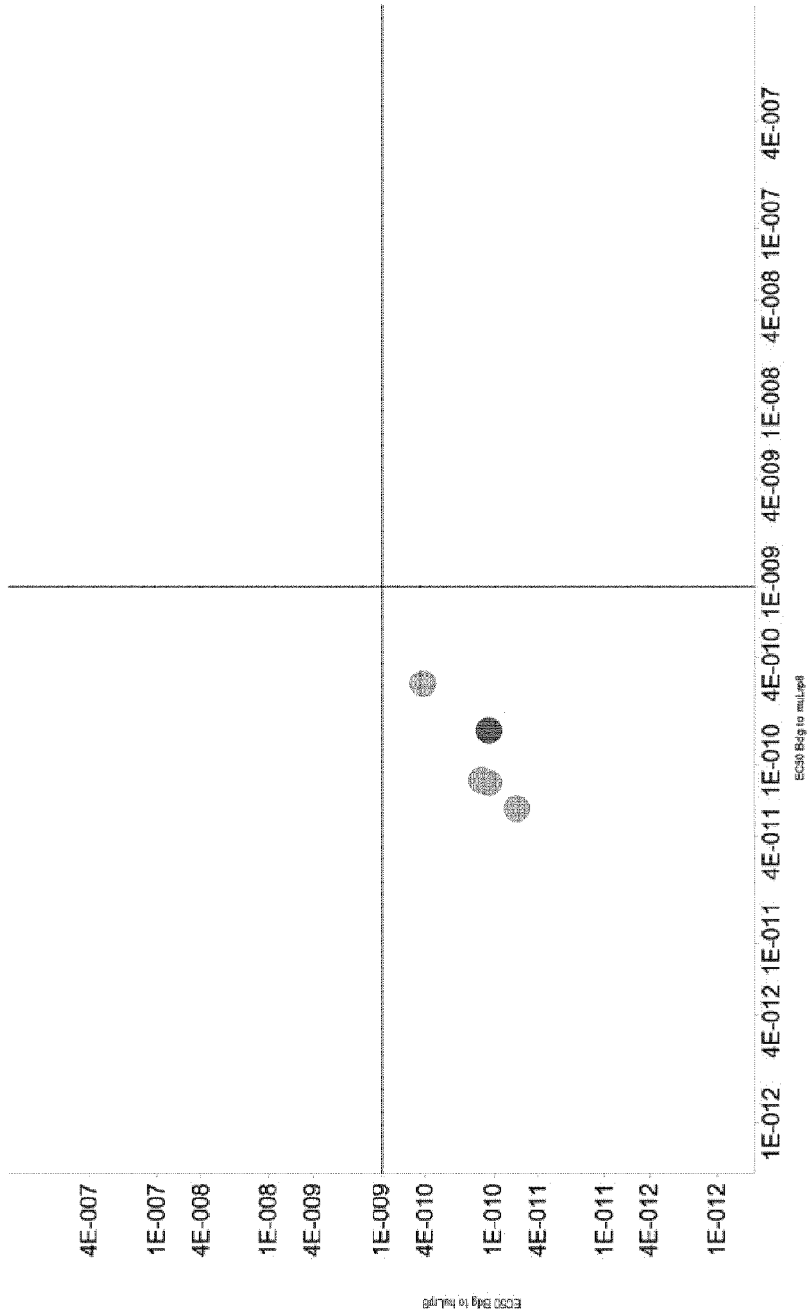
1E-012 4E-012 1E-011 4E-011 1E-010 4E-010 1E-009 4E-009 1E-008 4E-008 1E-007 4E-007
EC50 Bdg to muLrp8
4E-007 1E-007 4E-008 1E-008 4E-009 1E-009 4E-010 1E-010 4E-011 1E-011 4E-012 1E-012
EC50 Bdg to huLrp8

Figure 3B

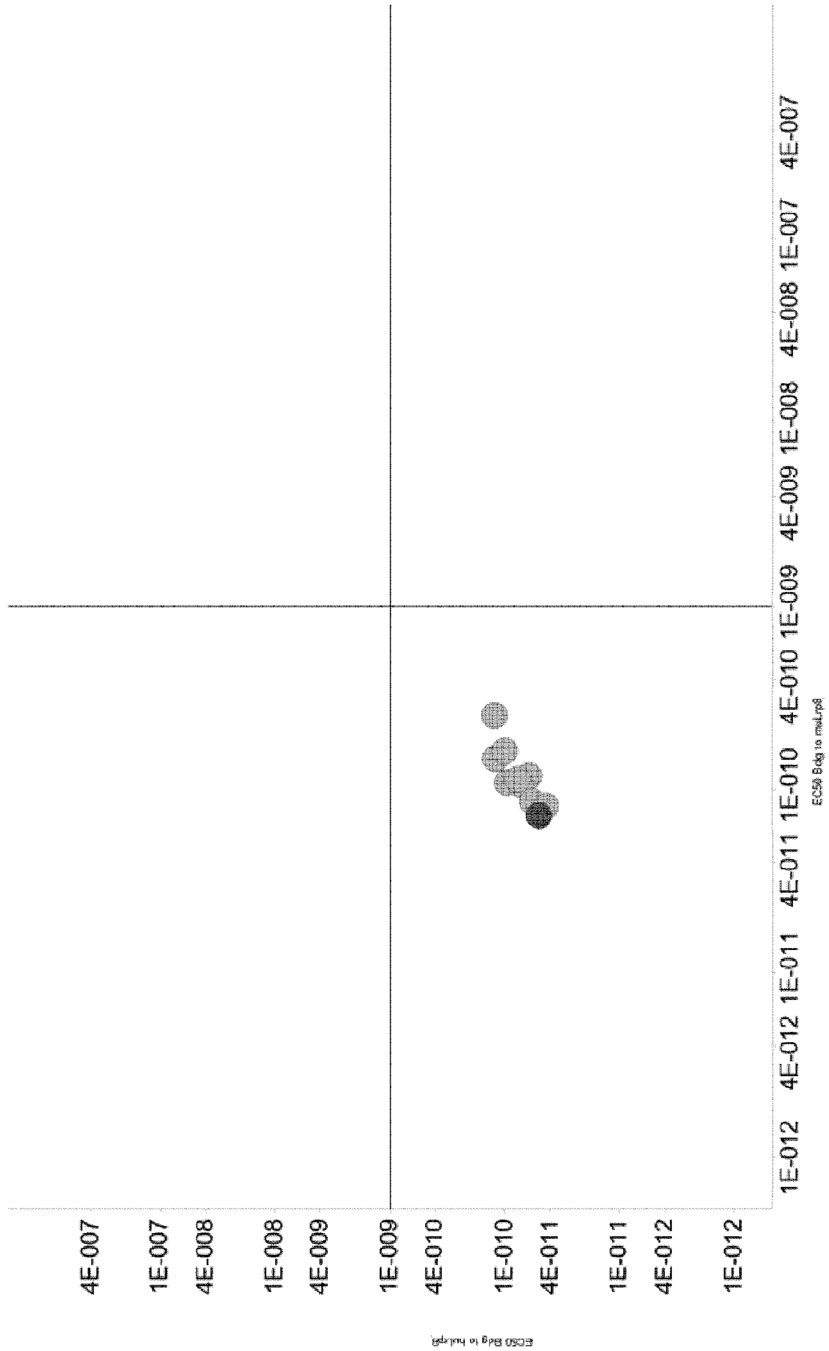
4E-007
1E-007
4E-008
1E-008
4E-009
1E-009
4E-010
1E-010
4E-011
1E-011
4E-012
1E-012
EC50 Bdg to huLrp8
1E-012 4E-012 1E-011 4E-011 1E-010 4E-010 1E-009 4E-009 1E-008 4E-008 1E-007 4E-007
EC50 Bdg to muLrp8

Figure 3C

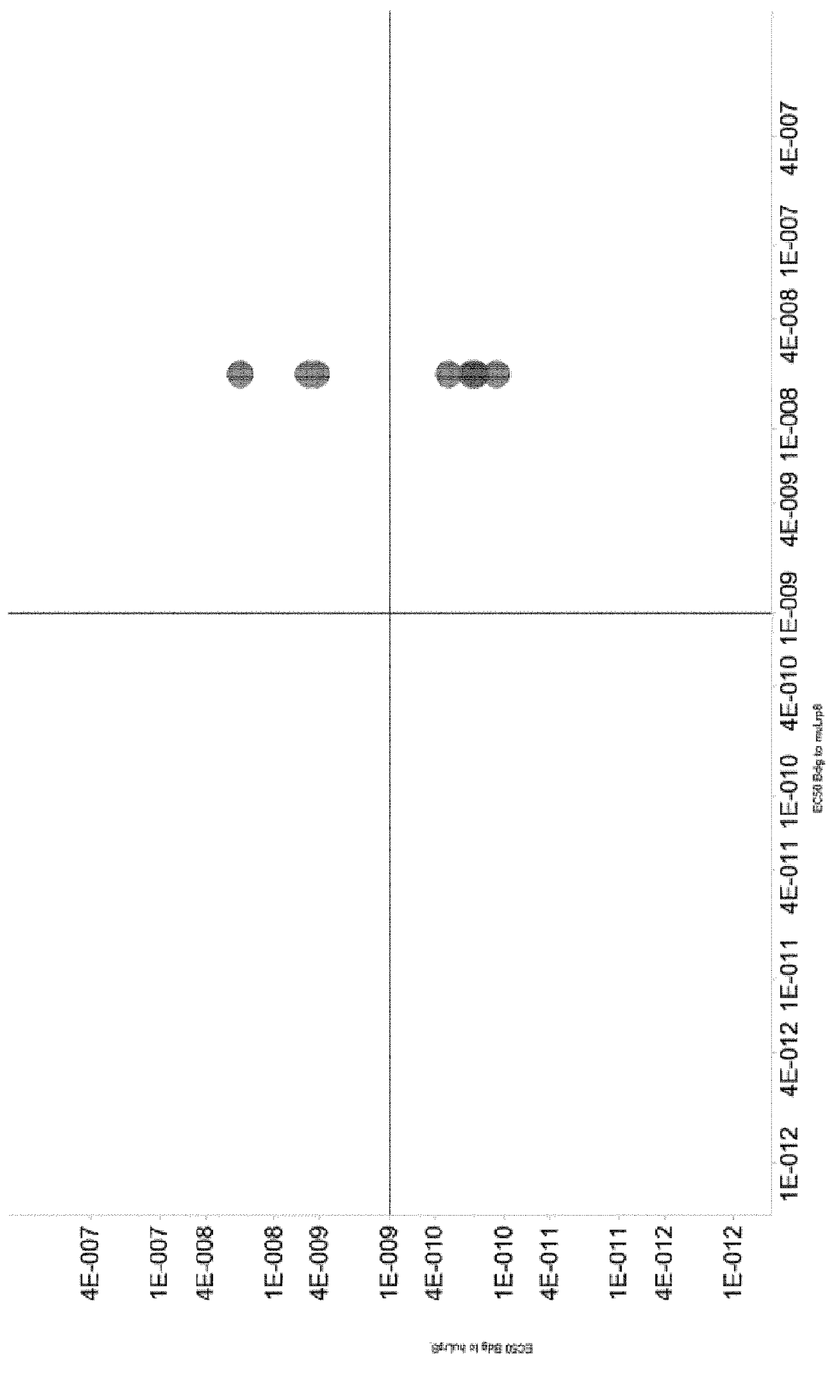
1E-012
4E-012
1E-011
4E-011
1E-010
4E-010
1E-009
4E-009
1E-008
4E-008
1E-007
4E-007
EC50 Bdg to msLrp8
EC50 Bdg to huLrp8

Figure 3D

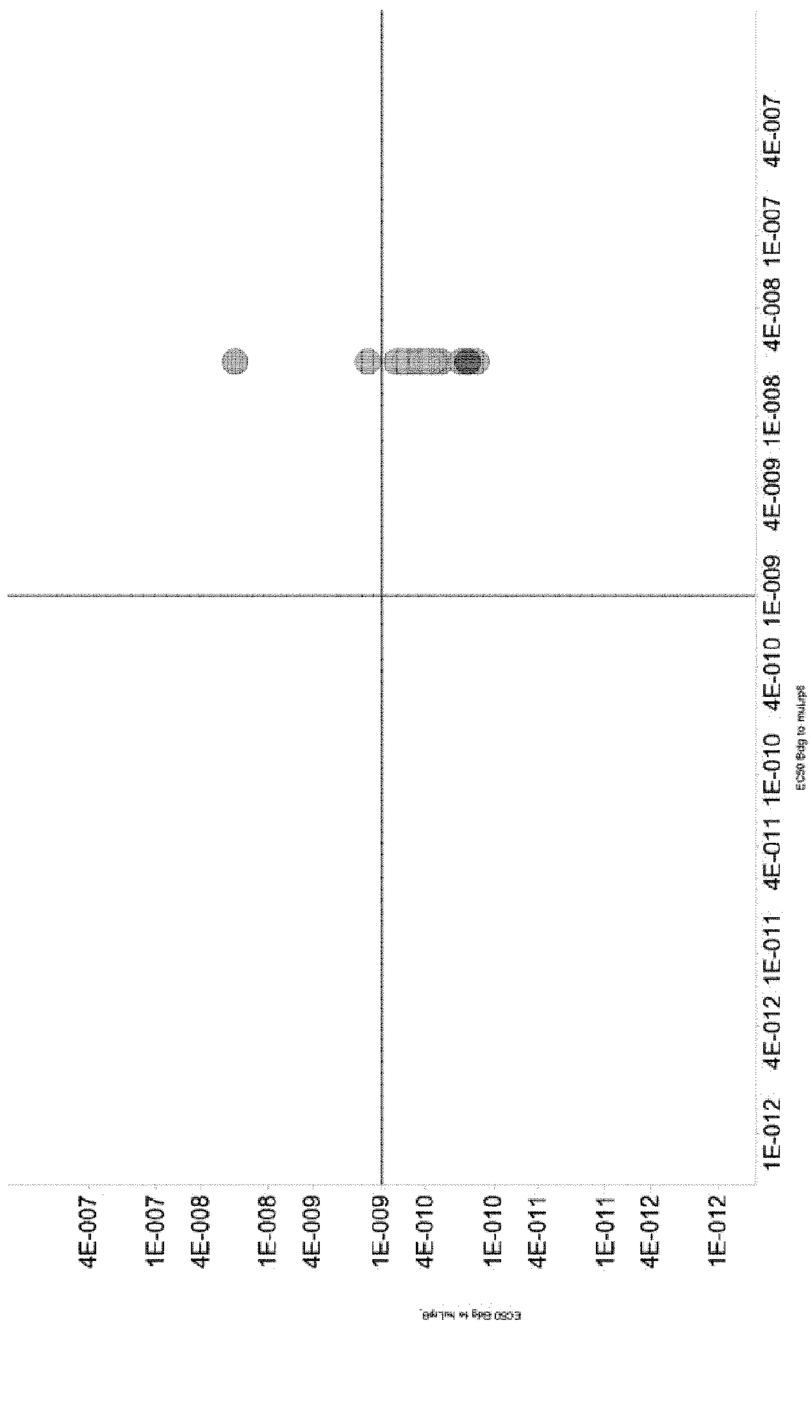
4E-007
1E-007
4E-008
1E-008
4E-009
1E-009
4E-010
1E-010
4E-011
1E-011
4E-012
1E-012
1E-012 4E-012 1E-011 4E-011 1E-010 4E-010 1E-009 4E-009 1E-008 4E-008 1E-007 4E-007
EC50 Bdg to muLrp6

Figure 4A

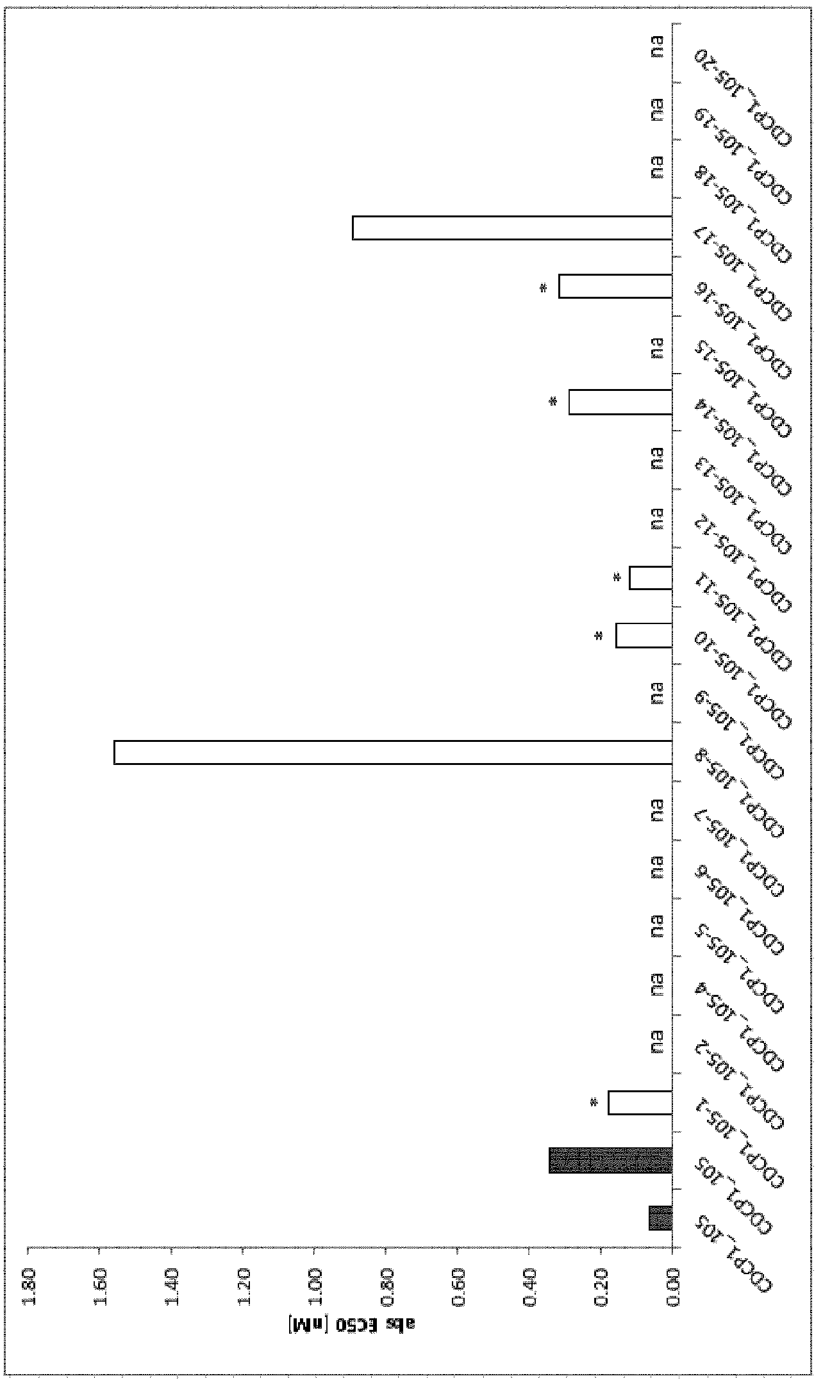
abs EC50 [nM]
0.00
0.20
0.40
0.60
0.80
1.00
1.20
1.40
1.60
1.80
CDCP1_105
CDCP1_105
CDCP1_105-1
CDCP1_105-2
CDCP1_105-4
CDCP1_105-5
CDCP1_105-6
CDCP1_105-7
CDCP1_105-8
CDCP1_105-9
CDCP1_105-10
CDCP1_105-11
CDCP1_105-12
CDCP1_105-13
CDCP1_105-14
CDCP1_105-15
CDCP1_105-16
CDCP1_105-17
CDCP1_105-18
CDCP1_105-19
CDCP1_105-20
*
na
na
na
na
na
na
*
*
na
na
*
na
*
na
na
na

Figure 4B

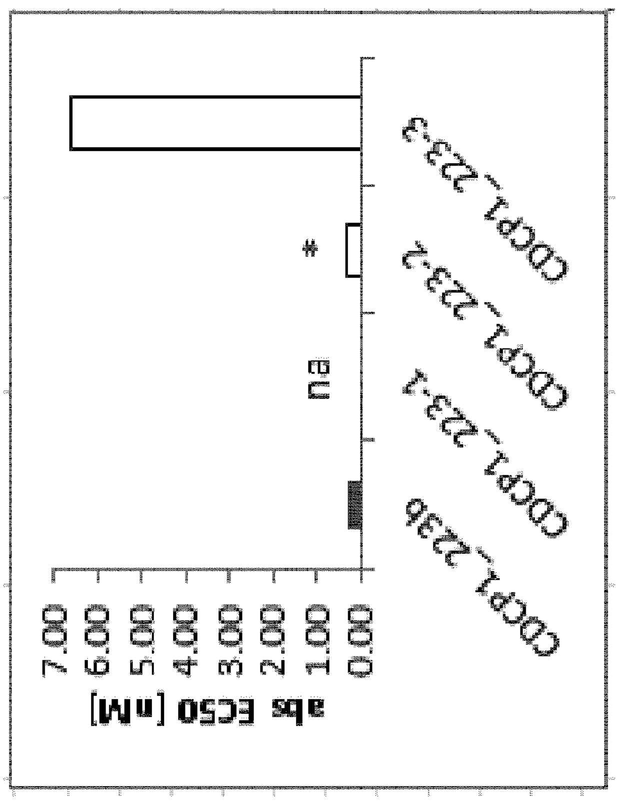
abs EC50 [nM]
7.00
6.00
5.00
4.00
3.00
2.00
1.00
0.00
na
*
CDCP1_223b
CDCP1_223-1
CDCP1_223-2
CDCP1_223-3

Figure 4C

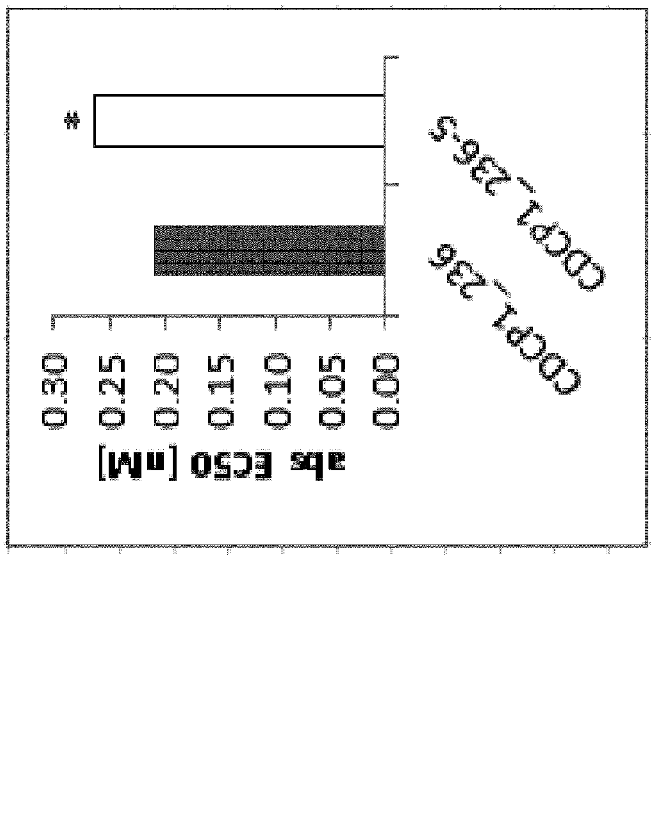

# Figure 4D

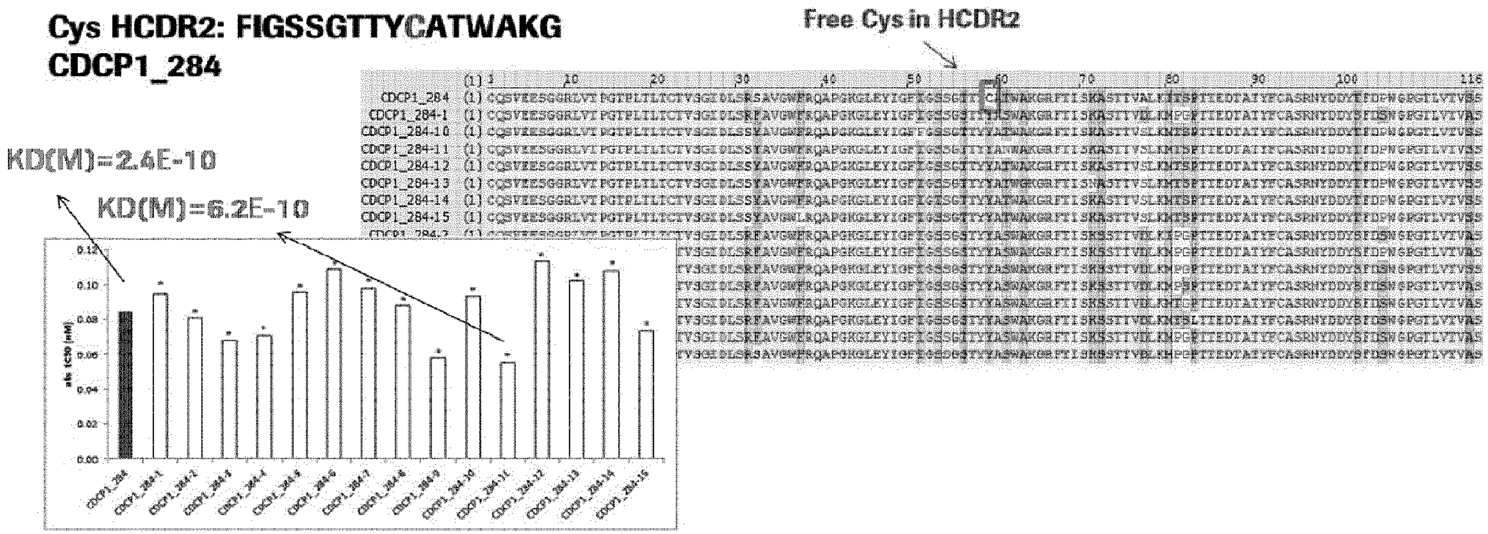
Cys HCDR2: FIGSSGTTYCATWAKG
CDCP1_284
Free Cys in HCDR2
KD(M)=2.4E-10
KD(M)=6.2E-10
0.12
0.10
0.08
0.06
0.04
0.02
0.00

# Figure 4E

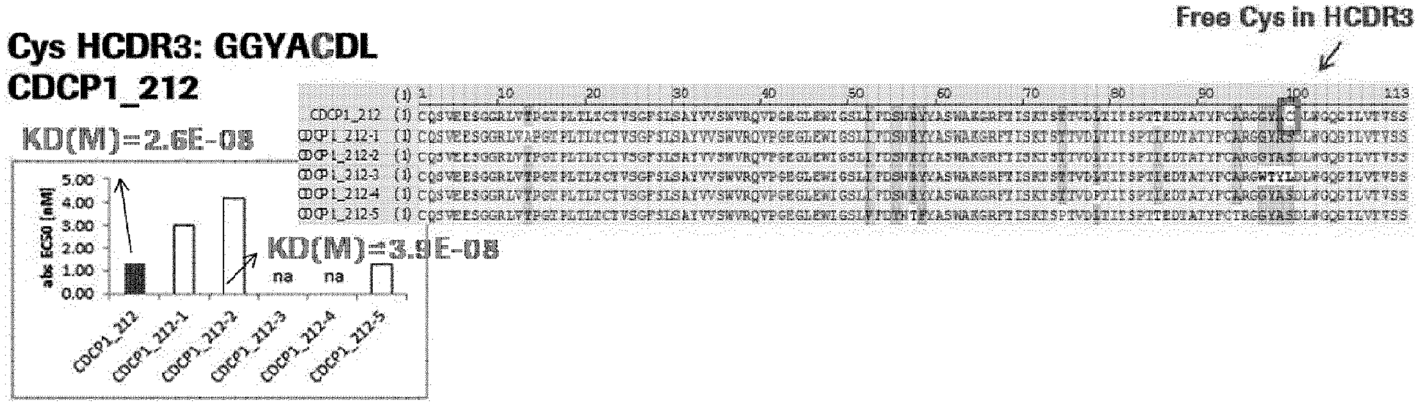
Free Cys in HCDR3
Cys HCDR3: GGYACDL
CDCP1_212
KD(M)=2.6E-08
KD(M)=3.9E-08
abs EC50 [nM]
na
CDCP1_212
CDCP1_212-1
CDCP1_212-2
CDCP1_212-3
CDCP1_212-4
CDCP1_212-5

Figure 4F

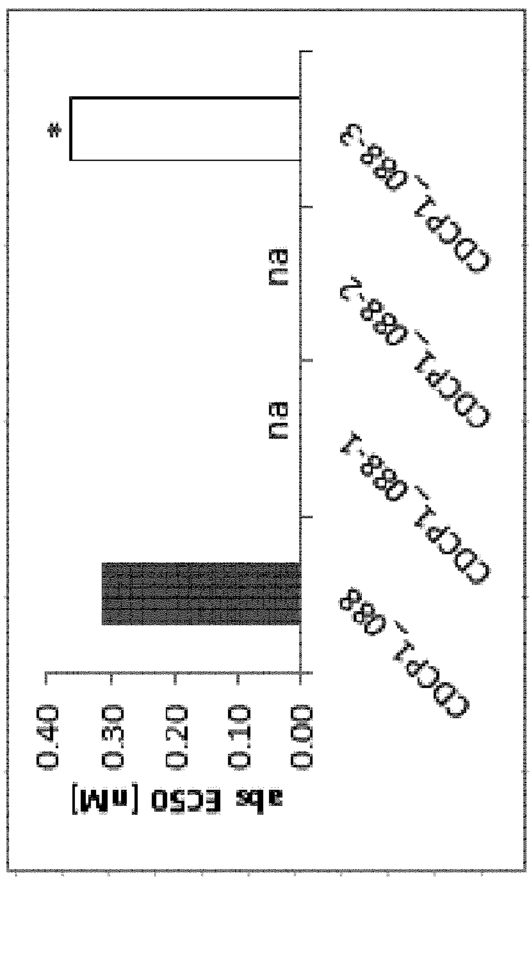
abs EC50 [nM]
0.40
0.30
0.20
0.10
0.00
na
na
*
CDCP1_088
CDCP1_088-1
CDCP1_088-2
CDCP1_088-3

Figure 4G

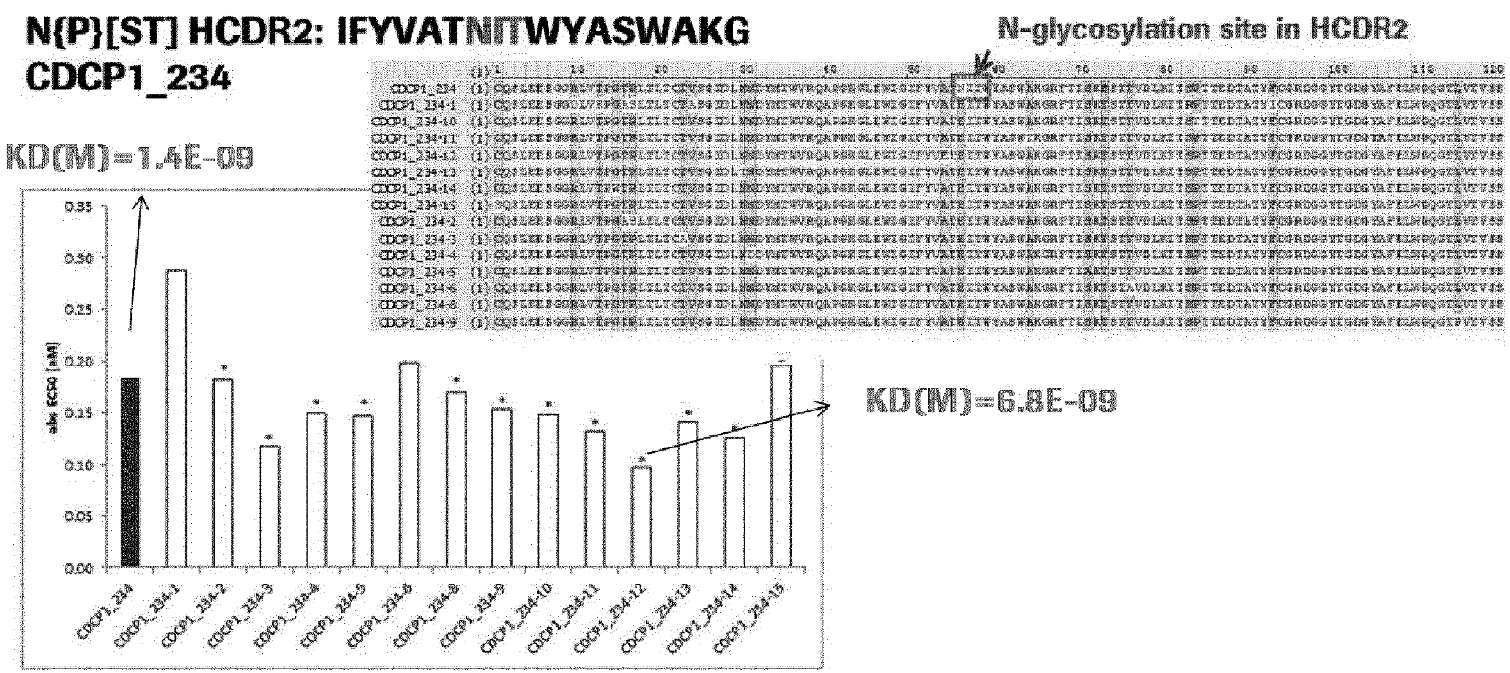
N{P}[ST] HCDR2: IFYVATNITWYASWAKG
CDCP1_234
N-glycosylation site in HCDR2
KD(M)=1.4E-09
KD(M)=6.8E-09
abs EC50 [nM]
0.35
0.30
0.25
0.20
0.15
0.10
0.05
0.00
CDCP1_234
CDCP1_234-1
CDCP1_234-2
CDCP1_234-3
CDCP1_234-4
CDCP1_234-5
CDCP1_234-6
CDCP1_234-8
CDCP1_234-9
CDCP1_234-10
CDCP1_234-11
CDCP1_234-12
CDCP1_234-13
CDCP1_234-14
CDCP1_234-15

Figure 5

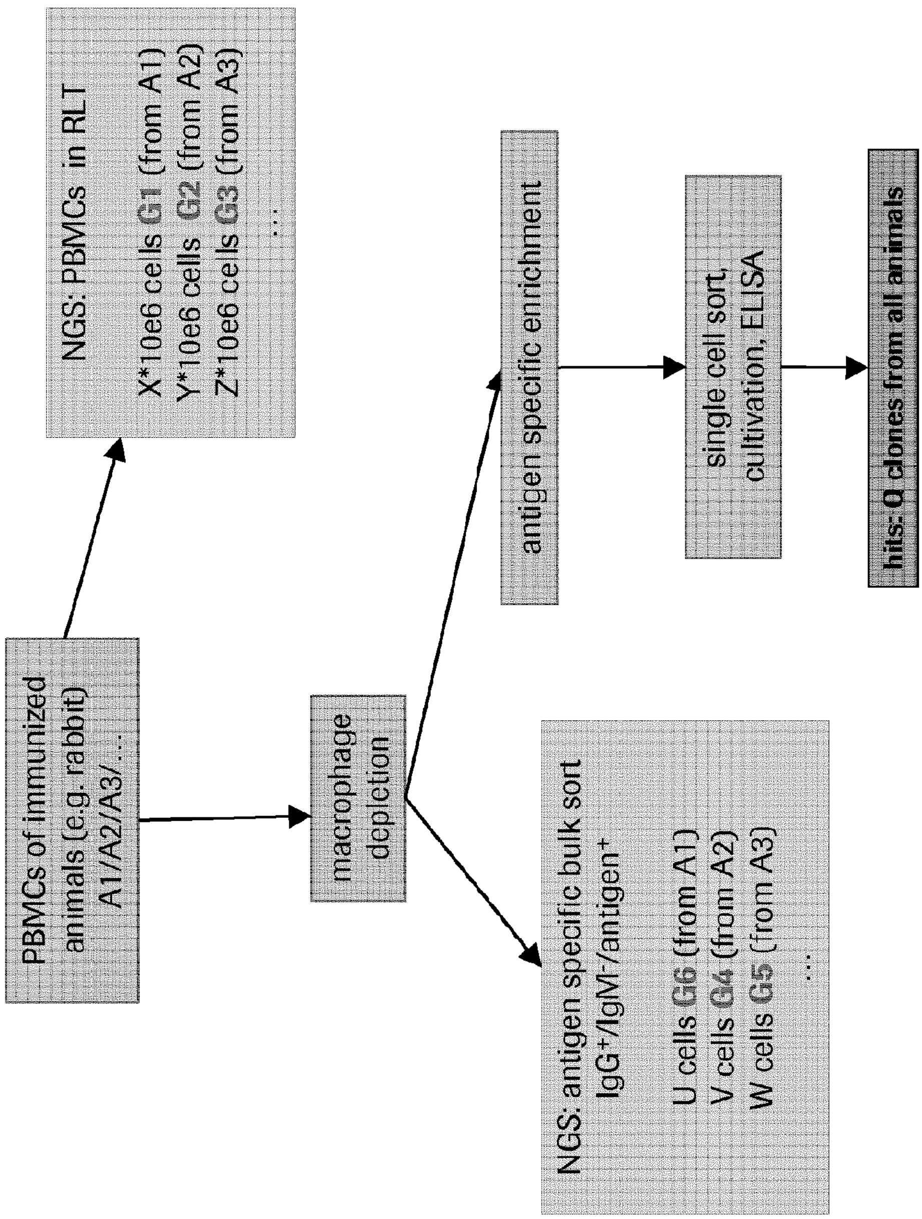
PBMCs of immunized animals (e.g. rabbit) A1/A2/A3/...
NGS: PBMCs in RLT
X*10e6 cells G1 (from A1)
Y*10e6 cells G2 (from A2)
Z*10e6 cells G3 (from A3)
...
macrophage depletion
antigen specific enrichment
single cell sort, cultivation, ELISA
hits: 0 clones from all animals
NGS: antigen specific bulk sort
IgG+/IgM-/antigen+
U cells G6 (from A1)
V cells G4 (from A2)
W cells G5 (from A3)
...

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015070191 A
- WO 2015155035 A
- WO 2015164757 A
- WO 2016023962 A
- WO 2016118883 A
- US 20060057638 A
- US 20060127950 A


### Non-patent literature cited in the description


- **WU et al.** *Science,* 2011, vol. 333, 1593-1602
- **ZHU et al.** *Proc. Natl. Acad. Sci. USA,* 2013, vol. 110, E4088-E4097
- **FRIDY et al.** *Nat. Meth.,* 2014, vol. 11, 1253-1260
- **GLANVILLE et al.** *Curr. Opin. Struct. Biol.,* 2015, vol. 33, 146-160
- **OBREZANOVA et al.** *Mabs,* 2015, vol. 7, 352-363
- **JARASCH et al.** *J. Pharm. Sci.,* 2015, vol. 104, 1885-1898
- **KABAT, E.A. et al.** Sequences of Proteins of Immunological Interest. National Institutes of Health, Bethesda, 1991
- **KABAT et al.** Sequences of Proteins of Immunological Interest. National Institutes of Health, Bethesda, 1991
- **KABAT et al.** Sequences of Proteins of Immunological Interest. National Institutes of Health, 1991, 647-660
- **SAMBROOK, J. et al.** Molecular Cloning: A laboratory manual. Cold Spring Harbor Laboratory Press, 1999
- **HAMES, B.D. ; HIGGINS, S.G.** Nucleic acid hybridization - a practical approach. IRL Press, 1985
- Current Protocols in Molecular Biology. 1997, vol. 1-3
- DNA Cloning: A Practical Approach. Oxford University Press, 1985, vol. 1-2
- Animal Cell Culture - a practical approach. IRL Press Limited, 1986
- **WATSON, J.D. et al.** Recombinant DNA. CHSL Press, 1992
- From Genes to Clones. VCH Publishers, 1987
- Cell Biology. Academic Press, 1998
- **FRESHNEY, R.I.** Culture of Animal Cells: A Manual of Basic Technique. Alan R. Liss, 1987
- **METZKER, M.L.** *Nat. Rev. Genet.,* 2010, vol. 11, 31-46
- **MARDIS, E.R.** *Annu. Rev. Genom. Hum. Genet.,* 2008, vol. 9, 387-402
- **CHOTHIA, C. ; LESK, A.M.** *J. Mol. Biol.,* 1987, vol. 196, 901-917
- **KABAT, E.A. et al.** Sequences of Proteins of Immunological Interest. National Institutes of Health, 1991
- **MACCALLUM et al.** *J. Mol. Biol.,* 1996, vol. 262, 732-745
- **FLATMAN, S. et al.** *J. Chromatogr. B,* 2007, vol. 848, 79-87
- **KINDT, T.J. et al.** Kuby Immunology. W.H. Freeman and Co, 2007, 91
- **PORTOLANO, S. et al.** *J. Immunol.,* 1993, vol. 150, 880-887
- **CLACKSON, T. et al.** *Nature,* 1991, vol. 352, 624-628
- **RAJU, T.S.** *Bioprocess Int.,* 2003, vol. 1, 44-53
- **BRUEGGEMANN, M. et al.** *J. Exp. Med.,* 1987, vol. 166, 1351-1361
- **LOVE, T.W. et al.** *Methods Enzymol.,* 1989, vol. 178, 515-527
- **ROUTIER, F.H.** *Glycoconjugate J.,* 1997, vol. 14, 201-207
- **JEFFERIS, R.** *Biotechnol. Prog.,* 2005, vol. 21, 11-16
- **TANIGUCHI, T. et al.** *Biochem.,* 1985, vol. 24, 5551-5557
- **MIZUOCHI, T. et al.** *Arch. Biochem. Biophys.,* 1987, vol. 257, 387-394
- **PAREKH, R.B. et al.** *Nature,* 1985, vol. 316, 452-457
- **SABA, J.A. et al.** *Anal. Biochem.,* 2002, vol. 305, 16-31
- Essentials of Glycobiology. CSHL Press, 1999
- **LI H. ; DURBIN R.** Fast and accurate short read alignment with Burrows-Wheeler Transform. *Bioinformatics,* 2009, vol. 25, 1754-60
- **BUJOTZEK, A. et al.** *Prot. Struct. Funct. Bioinform.,* 2015, vol. 83, 681-695
- **HENIKOFF, S. ; HENIKOFF, J.G.** *Proc. Nat. Acad. Sci.,* 1992, vol. 89, 10915-10919

- **DUNBAR et al.** *Prot. Eng. Des. Sel.,* 2013, vol. 26, 611-620
- **LI, W. ; GODZIK, A.** *Bioinformatics,* 2006, vol. 22, 1658-1659
- **LUPYAN, D. et al.** *Bioinf.,* 2005, vol. 21, 3255-3263
- **BUJOTZEK, A. et al.** *Proteins, Struct. Funct. Bioinf.,* 2015, vol. 83, 681-695
- **DUNBAR, J. et al.** *Protein Eng. Des. Sel.,* 2013, vol. 26, 611-620
- **BUJOTZEK, A. et al.** *Prot. Struct. Funct. Bioinf.,* 2015, vol. 83, 681-695
- **SEEBER, S. et al.** *PLoS One,* 2014, vol. 9, e86184
- **GALSON, J.D. et al.** *Crit. Rev. Immunol.,* 2015, vol. 35, 463-478
- **HAUN, R.S. et al.** *BioTechniques,* 1992, vol. 13, 515-518
- **LI, M.Z. et al.** *Nature Methods,* 2007, vol. 4, 251-256